# EUROPEAN PATENT SPECIFICATION

(11) **EP 1 100 830 B1**
(45) Date of publication and mention of the grant of the patent: **01.10.2003**
(21) Application number: 99944302.1
(22) Date of filing: 28.07.1999
(51) Int. Cl.: C07K 19/00, C12N 15/62, C12N 15/85, C12N 5/10, A61K 38/17, G01N 33/53, A61K 31/70, C07K 16/28

(54) **HETEROMINIBODIES**
HETEROMINIKÖRPER
HETEROMINICORPS

(30) Priority: 28.07.1998 EP 98114082
(43) Date of publication of application: 23.05.2001
(73) Proprietor: Micromet AG, 81477 München (DE)
(72) Inventor: KUFER, Peter, D-85368 Moosburg (DE); DREIER, Torsten, D-81369 München (DE); BAEUERLE, Patrick, A., D-82131 Gauting (DE); BORSCHERT, Katrin, D-97074 Würzberg (DE); ZETTL, Florian, D-87435 Kempten (DE)
(74) Representative: VOSSIUS & PARTNER
(86) International application number: EP9905416
(87) International publication number: WO00006605

(56) References cited:
- EP-A- 0 404 097
- EP-A- 0 517 024
- WO-A-95/09917
- WO-A-97/01580
- K. MÜLLER ET AL.: "The first constant domain (CH1 and CL) of an antibody used as heterodimerization domain for bispecific miniantibodies." FEBS LETTERS, vol. 422, no. 2, 30 January 1998 (1998-01-30), pages 259-264, XP002135067 Amsterdam, The Netherlands
- M. RHEINNECKER ET AL.: "Multivalent antibody fragments with high functional affinity for a tumor-associated carbohydrate antigen." THE JOURNAL OF IMMUNOLOGY, vol. 157, no. 7, 1 October 1996 (1996-10-01), pages 2989-2997, XP002135068 Baltimore, MD, USA
- F. DUCANCEL ET AL.: "Recombinant colorimetric antibodies: construction and characterization of a bifunctional F(ab)2/alkaline phosphatase conjugate produced in Escherichia coli." BIO/TECHNOLOGY, vol. 11, no. 5, May 1993 (1993-05), pages 601-605, XP002135069 USA
- I. KURUCZ ET AL.: "Retargeting of CTL by an efficiently refolded bispecific single-chain Fv dimer produced in bacteria." THE JOURNAL OF IMMUNOLOGY, vol. 154, no. 9, 1 May 1995 (1995-05-01), pages 4576-4582, XP002135070 Baltimore, MD, USA
- A. TRAUNECKER ET AL.: "Bispecific single chain molecules (Janusins) target cytotoxic lymphocytes on HIV infected cells." THE EMBO JOURNAL, vol. 10, no. 12, December 1991 (1991-12), pages 3655-3659, XP000232579 Oxford, GB
- P. KUFER ET AL.: "Construction and biological activity of a recombinant bispecific single-chain antibody designed for therapy of minimal residual colorectal cancer." CANCER IMMUNOLOGY, IMMUNOTHERAPY, vol. 45, no. 3-4, November 1997 (1997-11), pages 193-197, XP002076121 Heidelberg, Germany
- B. GERSTMAYER ET AL.: "Costimulation of T cell proliferation by a chimeric B7-2 antibody fusion protein specifically targeted to cells expressing the erbB2 proto-oncogene." THE JOURNAL OF IMMUNOLOGY, vol. 158, no. 10, 15 May 1997 (1997-05-15), pages 4584-4590, XP002116142 Baltimore, MD, USA

## Description

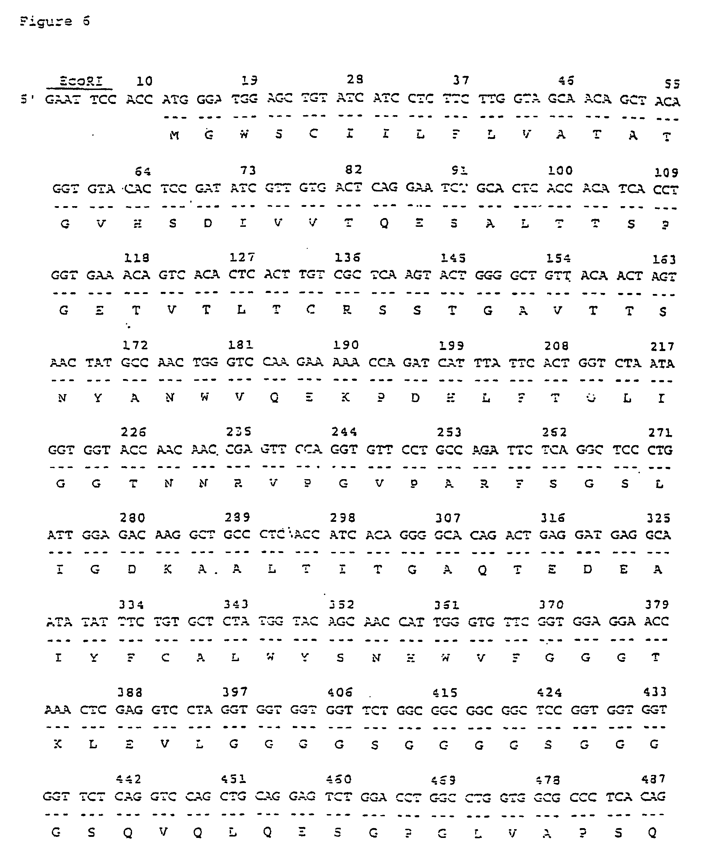

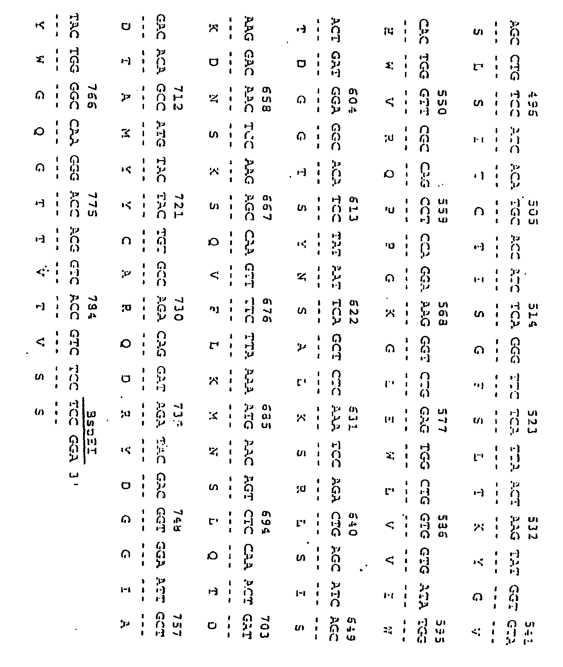

The present invention relates to a multifunctional compound, produceable in a mammalian host cell as a secretable and fully functional heterodimer of two polypeptide chains, wherein one of said polypeptide chains comprises, as the only constant region domain of an immunoglobulin heavy chain, the C_{H}1-domain and the other polypeptide chain comprises the constant C_{L}-domain of an immunoglobulin light chain, wherein said multifunctional compound further comprises, fused to said constant region domain(s) at least two and up to four (poly)peptides having different receptor or ligand functions, wherein further at least two of said different (poly)peptides lack an intrinsic affinity for one another and wherein said polypeptide chains are linked via said constant region domains. Preferably, said domains, having receptor or ligand function, are in the format of a scFv-fragment and/or are immuno-modulating effector molecules. Most preferably, said scFV-fragment comprises the V_{H} and the V_{L} regions of the murine anti 17-1A antibody M79, the V_{H} and the V_{L} regions of the anti-Lewis Y antibody, as shown in Fig. 6, or the V_{H} and the V_{L} regions of the anti-CD3 antibody TR66 and/or said immuno-modulating effector molecule comprises cytokines or chemokines. Furthermore, the present invention relates to polynucleotides encoding said polypeptide chains as well as vectors comprising said polynucleotides and host cells transformed therewith as well as the use of the above embodiments for the production of said multifunctional compounds. In addition, pharmaceutical and diagnostic compositions are provided, comprising any of the afore-described multifunctional compounds, polynucleotides or vectors. Described is also the use of the afore-mentioned multifunctional compound for preventing and/or treating malignant cell growth, related to malignancies of hemapoietic cells or to solid tumors.

In the mid 1980s, the concept of bispecific antibodies has been developed. By virtue of bispecific antibodies, different antigens, receptors or ligands can be crosslinked, which do not physiologically interact with each other, thus providing novel means of interfering with disease processes, recruiting cytotoxic effector cells to kill target cells, e.g. tumor cells, or virus-infected cells or facilitating the elimination of pathogens from the body.
Small bispecific antibody constructs are commonly thought to have great diagnostic and therapeutic potential. In contrast to bispecific versions of whole immunoglobulin molecules (Merchant, Nature Biotechnology 16 (1998), 677-681) expected to share the in vivo properties and especially the long serum half life of their natural monospecific counterparts, small bispecific antibody constructs due to their reduced molecular weight are preferable for applications requiring improved biodistributional properties. In addition, small bispecific antibodies have. been presumed to be producible in significant better yields than bispecific versions based on whole immunoglobulins. Accordingly, several recombinant routes have been developed for the production of such bispecific antibody fragments in order to overcome the low yields of conventional methods (Carter, J. Hematother. 4 (1995) 463-470).

Prior art bispecific antibody fragments usually could not be glycosylated due to their lack of glycosylation sites. Accordingly, production methods have been focused on E.coli as expression host, although functional expression of antibody derivatives in E.coli can be critical, depending on the successful translocation of the corresponding polypeptide chains into the periplasmatic space and on the structural complexity of the recombinant protein. Thus, bispecific single-chain antibodies consisting of four Ig-variable regions on a single polypeptide chain proved to be not expressable as functional proteins in the periplasma of E.coli. In contrast, bispecific single-chain antibodies can be expressed as fully functional recombinant proteins within the secretory pathway of mammalian cells thus allowing the purification from the culture supernatant (Mack, Proc. Natl. Acad. Sci. USA 92 (1995), 7021-7025).

In general, strategies for the expression of bispecific antibodies can be divided into two-host and single-host systems (Carter, J. Hematother. 4 (1995), 463-470). In two-host systems, the two different specificities are separately expressed and purified and subsequently combined in vitro to form bispecific heterodimers. In single-host systems, the bispecific antibody is either expressed in the single chain format or two different polypeptide chains form heterodimers during expression in the same host cell. In principle, single-host systems are more preferable than two-host systems, since the additional in vitro steps required in the two-host systems tend to increase production costs, reduce the yield and limit the attainable purity of the resulting bispecific antibodies. Of course, functional expression of bispecific antibody derivatives in a suitable single-host system is also preferable to conventional methods relying on non-functional expression followed by complete denaturation of the recombinant protein, and subsequent refolding. Accordingly, efficient methods for the functional expression of bispecific antibodies in single host systems are highly preferable compared to alternative methods.

In addition to the functional expression of bispecific single-chain antibodies in mammalian cells, the only single host system that predominantly produces bispecific antibody fragments and proved to be feasible for production upscaling is the expression of diabodies in the periplasma of E.coli, which is based on the preferential dimerization of two different polypeptide chains (Hollinger, Proc. Natl. Acad. Sci. USA 90 (1993), 6444-6448). Recently published bispecific miniantibodies also expressed in the periplasm of E.coli (Müller, FEBS Letters 422 (1998), 259-264) still have to prove their feasibility for production upscaling. As regards the functional expression of small bifunctional antibody constructs comprising at least one non-immunoglobulin part, only mammalian host cells fully meet expression requirements. This is because non-immunoglobulin portions such as the extracellular domains of cellular receptors are often glycosylated and frequently exceed Ig-antigen binding sites in structural complexity. In contrast to mammalian systems, E.coli, yeast or baculovirus systems do not or only partially meet these requirements.

For example, many of the N-linked carbohydrates of vertebrate glycoproteins are not found in E.coli (e.g. sialic acid). Those carbohydrates have important functions in cell-cell recognition, adhesion and protein function. Additionally, O-glycosylation which also occurs in E.coli is fundamentally different to mammalian O-glycosylation processes since, inter alia, different carbohydrates are added.

As has been demonstrated by others (Gerstmayer, J. Immunol. 158 (1997), 4584-4590), an appropriate format for the expression of such bifunctional antibody constructs comprising non-immunoglobulin parts in higher host cells is the single chain format. Whereas the single chain format bears a number of significant advantages, it is generally believed that many non-immunoglobulin parts comprised therein require the native N-terminus within bifunctional single-chain molecules in order to maintain their function. As a consequence, the Ig-antigen binding site within such a single chain has to be placed at the C-terminus. However, in such constructs the antigen binding activity at the C-terminal position is often lost (see Example 8). This holds true even when the advantageous mammalian expression system is used. Therefore it has to be concluded, that the single-chain approach does not provide a generally applicable format for the functional expression of bifunctional antibody constructs.

Accordingly, the technical problem underlying the present invention was to develop a molecular format for the functional expression of bi- and multifunctional antibody constructs that is generally applicable for combinations of any given scFv-antibody fragment optionally in combination with different non-immunoglobulin portions. The solution to this technical problem is achieved by providing the embodiments characterized in the claims.

Accordingly, the present invention relates to a multifunctional compound, produceable in a mammalian host cell as a secretable and fully functional heterodimer of two polypeptide chains, wherein one of said polypeptide chains comprises, as the only constant region domain of an immunoglobulin heavy chain the C_{H}1-domain and the other polypeptide chain comprises the constant C_{L}-domain of an immunoglobulin light chain, wherein said polypeptide chains further comprise, fused to said constant region domains at least two and use to four (poly)peptides having different receptor or ligand functions, wherein further at least two of said different (poly)peptides lack an intrinsic affinity for one another and wherein said polypeptide chains are linked via said constant domains.

The term "multifunctional compound" as used herein denotes a compound comprising two polypeptide chains, wherein said compound comprises at least two functional domains conferring different functions. Such multifunctional compounds include, e.g., bi-, tri-, or tetraspecific heterominibodies. The term "heterominibody" means a heterodimer of two different polypeptide chains wherein the domains that mediate heterodimerization consist solely of the immunoglobulin constant region domains C_{H}1 and C_{L}.

The term "domains, having receptor or ligand function" in accordance with the present invention denotes functional domains comprising a three-dimensional structure capable of specifically binding to or interacting with a molecule. Such a molecules can be, but are not limited to, peptides or polypeptides and their post-translational modifications. These post-translational modifications comprise, but are not limited to glycosylations (N- and/or O-glycosylations), tyrosine sulfation, phosphorylation and/or proline hydroxylation.

The term "different (poly)peptides do not have an intrinsic affinity for one another" means, in accordance with the present invention, that the different (poly)peptides do not naturally tend to associate under physiological conditions such as, for example, V_{H} and V_{L} chains do.

The term "fully functional" means, in accordance with the present invention, that the compounds of the invention secreted by mammalian host cells into the culture supernatant in contrast to e.g. proteins expressed as inclusion bodies in E.coli do not require any protein refolding after purification; all subunits of the compounds of the invention are correctly folded and thus express their specific functions simply by being expressed in and secreted by mammalian host cells.

Thus, the present invention provides a multifunctional compound comprising two different polypeptide chains wherein efficient heterodimerization of said polypeptide chains during the expression and the secretion process in mammalian host cells is mediated by the interaction of the above specified constant region domains of immunoglobulin light and heavy chains.

The heterodimerization of constant immunoglobulin domains allows the interaction of at least two additional different (poly)peptide chains fused thereto without any intrinsic affinity to each other in a single mammalian expression system further allowing relevant post-translational modification and leading to a secretable compound of higher structural complexity.

The domains of the multifunctional compound of the present invention, having receptor or ligand function can be either linked to the C- and the N-terminus of one or both constant immunoglobulin domains. Therefore, the present invention provides multifunctional compounds, which can comprise bi-, tri- or tetrafunctional molecules, wherein each of said receptor or ligand functions can be linked to either the C- or the N-terminus of said constant immunoglobulin domains.
The linkage of said functional domains to the constant immunoglobulin domains can be provided by, e.g. genetic engineering, as described in the examples. Methods for preparing fused and operatively linked polypeptide chains and expressing them in mammalian cells are well-known in the art (e.g. Sambrook et al., Molecular Cloning: A Laboratory Manual, Cold Spring Harbor Laboratory Press, Cold Spring Harbor, New York, 1989).

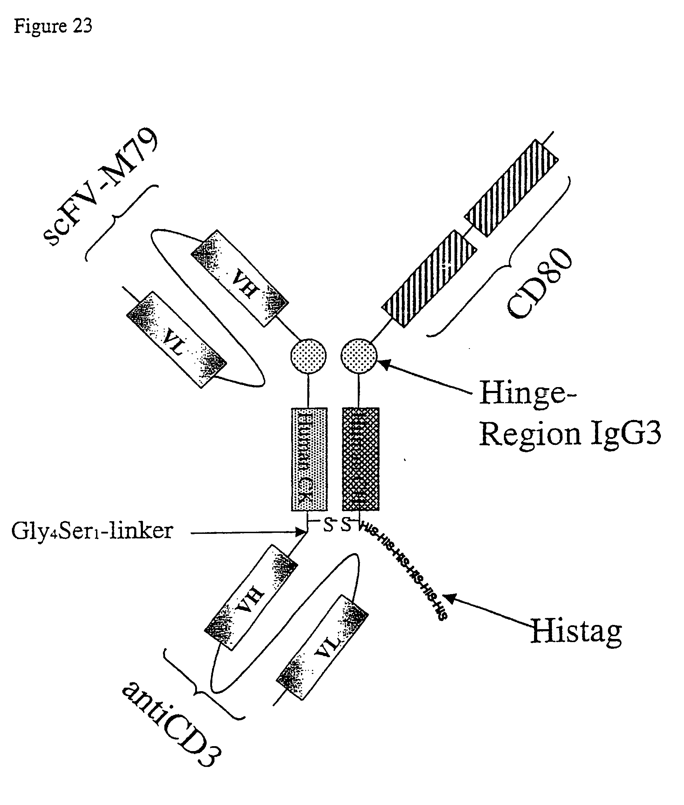

As has been detailed above, the solution to the various problems dealt with in the prior art was found to be a molecular format consisting of two different polypeptide chains, wherein one polypeptide chain contains the C_{H}1-domain of an immunoglobulin heavy chain and the other polypeptide chain contains the constant domain of an immunoglobulin light chain, thus mediating efficient heterodimerization of said polypeptide chains during the expression and secretion process in mammalian host cells. This molecular format advantageously provides two N-terminal positions in contrast to the prior art single chain format and proved to be efficiently secreted by mammalian host cells into the culture supernatant, where it is found as fully functional and adequately glycosylated heterodimeric protein that can be easily purified.
Furthermore, the molecular format of the invention additionally provides two C-terminal positions that can be occupied by further protein domains thus resulting in a multifunctional compound carrying more than two different functional entities. In the case that two different (poly)peptides which do not tend to associate under the above recited conditions are fused to the N-termini of the constant region domains, then to each C-terminus of said constant region domains one V_{H} and V_{L} region, respectively, may be found. Identifying appropriate heterodimerization domains that meet the above mentioned criteria was no trivial task since strategies to obtain preferential heterodimerization in single host expression systems preferably of two different receptors or ligands without any intrinsic affinity for one another by using heterodimerization domains previously failed or had still to be developed. Heterodimerization domains based on leucine zippers, for example, proved to facilitate the heterodimerization in single host expression systems of polypeptid chains that, although very weakly (Chang, Proc. Natl. Acad. Sci. USA 91 (1994), 11408-11412; Kalandadze, J. Biol. Chem. 271 (1996), 20156-20162) intrinsically tend to heterodimerize with each other like the α and β chains of T-cell receptors or MHC-class II-molecules. However, in cases of two different proteins without any intrinsic affinity to each other or with some intrinsic affinity to one another but without any heterodimerization preference, as for example the two different antigen binding sites within heterodimeric bispecific antibodies, jun-and fos-based domains quantitatively produced homodimers instead of heterodimers in single host expression systems (de Kruif, J. Biol. Chem. 271 (1996), 7630-7634). Such jun-and-fos homodimers could be dissociated in vitro, mixed and subjected to conditions facilitating reassociation, which turned out to mainly result in jun-fos-heterodimers (Kostelny, J. Immunol. 148 (1992), 1547-1553). A naturally occurring example of protein heterodimerization is found in immunoglobulins, where heavy and light chains associate to form the antigen binding sites. In the antibody molecule, heterodimerization of the variable region domains V_{L} and V_{H}, which have an intrinsic affinity to each other, is facilitated by the constant region domains C_{L} and C_{H}1. Thus, it was not surprising that C_{L} and C_{H}1 could also support the dimerization of scFv-fragments (Müller, FEBS Letters 422 (1998), 259-264), because scFv-fragments are also known to form dimers with each other even without the support of any special dimerization domains (Korff, Eur. J. Biochem. 221 (1994), 151-157; Griffiths, EMBO J. 12 (1993), 725-734). Autochthonous dimerization of scFv-fragments is most likely due to the disruption of hydrophobic patches at the antibody variable/constant domain interface, leading to the solvent exposure of residues that are normally buried in intact immunoglobulins or Fab-fragments (Nieba, Protein Eng. 10 (1997), 435-444). Thus, C_{L} and C_{H}1, according to the prior art, similar to jun- and fos-domains, were known to support the autochthonous dimerization process of polypeptides with an intrinsic affinity for one another, thereby favoring the formation of heterodimers over homodimers. The approach of the present invention, however, allows heterodimerization of two different (poly)peptide chains without any intrinsic affinity to each other in a single host expression system wherein said (poly)peptides are fused to said constant region domains.
Thus, it was surprisingly found that C_{L} and C_{H}1 (solely by themselves) can provide sufficient dimerization forces capable of joining different receptors or ligands (e.g. CD80 and the M79scFv-fragment, see Example 1) which normally do not at all associate autochthonously or may, to some extent, even resist heterodimerization for steric and thermodynamic reasons. Therefore, the approach of the present invention allows heterodimerization of two different (poly)peptide chains without any intrinsic affinity to each other in a single host expression system wherein said (poly)peptides are fused to said constant region domains. This approach most importantly meets the functional expression and secretion requirements of mammalian host cells, thus enabling heterodimerization-based multifunctional (like bi-, tri-,or tetra-functional) compounds that may be glycosylated and of higher structural complexity. Identifying oligomerization domains that generally meet the expression and secretion requirements of mammalian host cells was by no means obvious, since feasibility of such domains in bacterial expression systems turned out to be not predictable for their feasibility in mammalian host cells. Results shown in example 9 illustrate this general inconsistency.
Surprisingly it was found in the present invention, as demonstrated in the examples, that the molecular format of the invention designated "heterominibody" proved to be capable of carrying many different and varying numbers (up to 4) polypeptides having receptor or ligand function fused to the N- and/or C-terminus of C_{L} or C_{H}1, without losing produceability as secretable and fully functional molecule in mammalian host cells.
Bifunctional antibody derivatives described in examples 1-4 (the corresponding embodiments of which are also described further below) and produced according to the molecular format of the invention consist of a scFv-antibody fragment directed against a tumor-associated antigen, e.g. 17-1A or LewisY, and the extracellular part of cellular receptors (e.g. CD80, CD86, CD58, CD54) mainly expressed on antigen presenting cells e.g. dendritic cells and known for their T-cell costimulatory and/or adhesion function. One of these bifunctional antibody derivatives, heterominibody M79scFvCK/CD80CH1 was extensively tested for its functional activity. The recombinant molecule proved to bind to its native target antigen 17-1A on intact cells and was surprisingly found to subsequently provide not only one necessary costimulatory signal to naive T-lymphocytes by virtue of its CD80(B7-1) arm but mediates sufficient costimulation in order to prime naive CD4+-and CD8+-T-cells, that simultaneously receive the first activation signal via an anti-17-1A x anti-CD3 bispecific single chain antibody (Mack, Proc. Natl. Acad. Sci. USA 92 (1995), 7021-7025) further designated M79scFv-antiCD3scFv. The priming events could be clearly demonstrated by switching of the T-cell surface phenotype from that of naive (CD45RA+R0-) to that of primed T-lymphocytes (CD45RA-RO+) and could be confirmed by determination of characteristic cytokines in the T-cell supernatant. Exclusive secretion of INF-γ but not IL-5 and IL-4 by in vitro primed CD4+-T-lymphocytes furthermore interestingly demonstrated, that the heterominibody M79scFvCK/CD80CH1 selectively mediates the priming and differentiation of CD4+T-cells that express the TH1-phenotype, which is envisaged to advantageously augment the cellular immune response against tumor cells in vivo. Other bifunctional tumorspecific B7(CD80 or CD86) constructs described in the literature, have so far never been shown to provide sufficient costimulation for the priming of naive T-cells (Gerstmayer, J. Immunol. 158 (1997), 4584-4590; Challita-Eid, J. Immunol. 160 (1998), 3419-3426). It is envisaged that such costimulatory heterominibody constructs can be applied in vivo alone or in combination with a bispecific antibody which provides the primary T-cell activation signal independent of the clonotypic T-cell receptor. It is further envisaged that such a combination can be attained by structurally combining features of both molecules within one multifunctional compound according to the molecular format of the invention as described in example 7 and shown in figure 23.

Additionally, the present invention relates to a multifunctional compound consisting of two polypeptide chains, wherein one of said polypeptide chains comprises, as the only constant region domain of an immunoglobulin heavy chain the C_{H}1-domain and the other polypeptide chain comprises the constant C_{L}-domain of an immunoglobulin light chain, wherein said polypeptide chains further comprise, fused to said constant region domains at least two (poly)peptides having different receptor or ligand functions, wherein further at least two of said different (poly)peptides lack an intrinsic affinity for one another and wherein said polypeptide chains are linked via said constant domains. Advantageously, said multifunctional compound is produceable in a mammalian host cell as a secretable and fully functional heterodimer.

In a preferred embodiment, the multifunctional compound of the present invention comprises at least three functional domains, having receptor or ligand function, providing a tri-specific heterominibody.

In a more preferred embodiment, the multifunctional compound of the present invention comprises four domains, having receptor or ligand function, providing for a multifunctional compound/heterominibody with effector domains present in all four positions. Such a multifunctional compound may be bi-, tri- or tetra-specific.

The present invention also relates, in a further preferred embodiment, to the multifunctional compound of the present invention, wherein at least two domains, having receptor or ligand function, are N-terminally linked to said constant C_{H}1 or C_{L} domains. Additionally, the present invention relates in yet another preferred embodiment to the multifunctional compound of the invention, wherein at least two domains having receptor or ligand function, are C-terminally linked to said C_{H}1 or C_{L} domains. In this case C_{H}1 and/or C_{L} that do not carry receptor or ligand domains at their N-terminus must carry an N-terminal leader peptide preferably derived from immunoglobulin heavy or light chains in order to facilitated secretion by mammalian host cells.

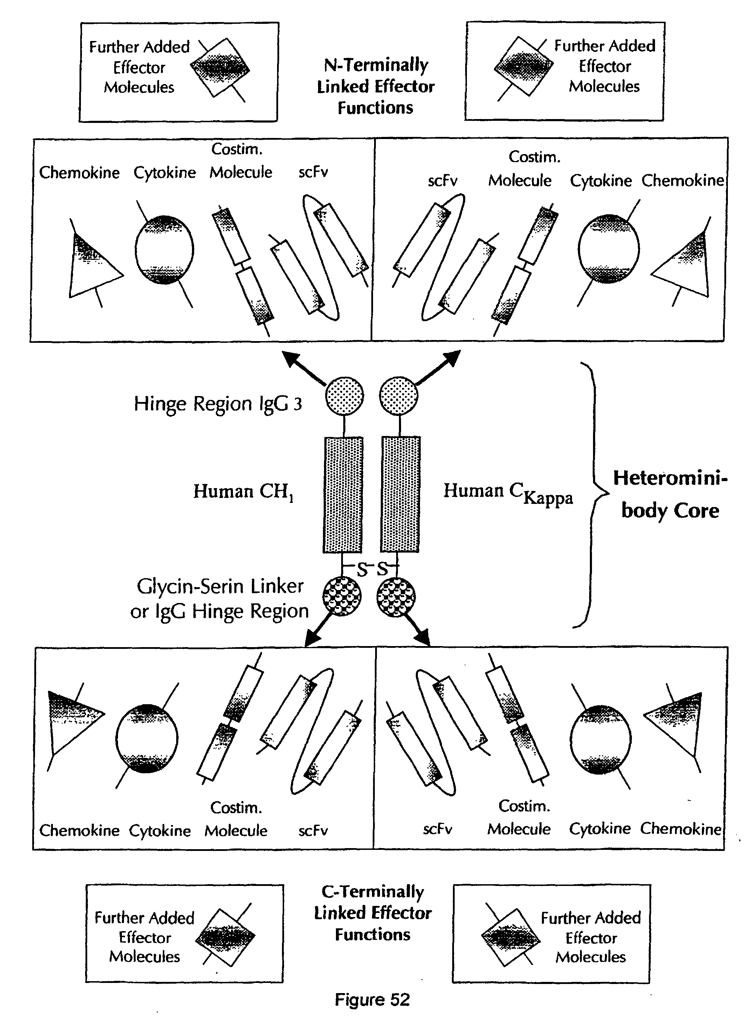

All of the above embodiments envisage multifunctional compounds/heterominibodies wherein two or more functional domains/effector domains are fused N- or C-terminally to the same constant (C) domain. For example, if each N- and/or C-terminal position of said C domains are occupied by an effector/functional domain then one or more additional effector/functional domain(s) may be fused to each of said effector domains in said position. An example of this type of construct is provided in Figure 52.

In case that polypeptides with receptor or ligand functions consist of the extracellular parts of integral transmembrane proteins type I or type II it is preferred that extracellular parts of type I-proteins are fused to the N-terminus of C_{L} or C_{H}1 whereas extracellular parts of type II-proteins are preferably fused to the C-terminus of C_{L} or C_{H}1.

Polypeptides with receptor or ligand function are preferably fused to the N- or C-terminus of C_{L} or C_{H}1 via Ig-Hinge regions or flexible peptide linkers e.g. via a Glycin-Serin-linker in order to ensure functionality of said polypeptides. Linkers of different types or lengths may be identified without undue burden to obtain full functional activity of specific polypeptides.

In yet another preferred embodiment, the invention relates to the multifunctional compound of the invention, wherein at least one of said domains, having receptor or ligand function, is in the format of a scFV-fragment or a functional part thereof.

The multifunctional compound of the invention, wherein at least one of said domains, having receptor or ligand function, is a T-cell co-stimulatory ligand, an antigen binding region specific for a tumor associated antigen, or a proteinaceous compound providing the primary activation signal for T-cells, is still another preferred embodiment of the invention.
Adequate activation resulting in priming of naive T-cells is critical to primary immunoresponses and depends on two signals derived from professional APCs (antigen-presenting cells) like dendritic cells. The first signal is antigen-specific and normally mediated by stimulation of the clonotypic T-cell antigen receptor that is induced by processed antigen presented in the context of MHC class-I or MHC class-II molecules. However, this primary stimulus is insufficient to induce priming responses of naive T-cells, and the second signal is required which is provided by an interaction of specific T-cell surface molecules binding to co-stimulatory ligand molecules on antigen presenting cells, further supporting the proliferation of primed T-cells. The term "T-cell co-stimulatory ligand" therefore denotes in the light of the present invention molecules, which are able to support priming of naive T-cells in combination with the primary stimulus and include, but are not limited to, members of the B7 family of proteins, including B7-1 (CD80) and B7-2 (CD86).

An antigen binding region specific for a tumor associated antigen denote antibody fragments directed against tumor associated antigen known in the art, e.g. 17-1A or Lewis Y, Muc-1, erbB2 or s-Tn.

In the light of the present invention, "proteinaceous compounds" providing the primary activation signal for T-cells" can comprise, but are not limited to, anti-CD3-svFv fragments, anti-T-cell receptor svFv fragments or superantigens. Superantigens directly bind to certain subfamilies of T-cell receptor variable regions in an MHC-independent manner thus mediating the primary T-cell activation signal.

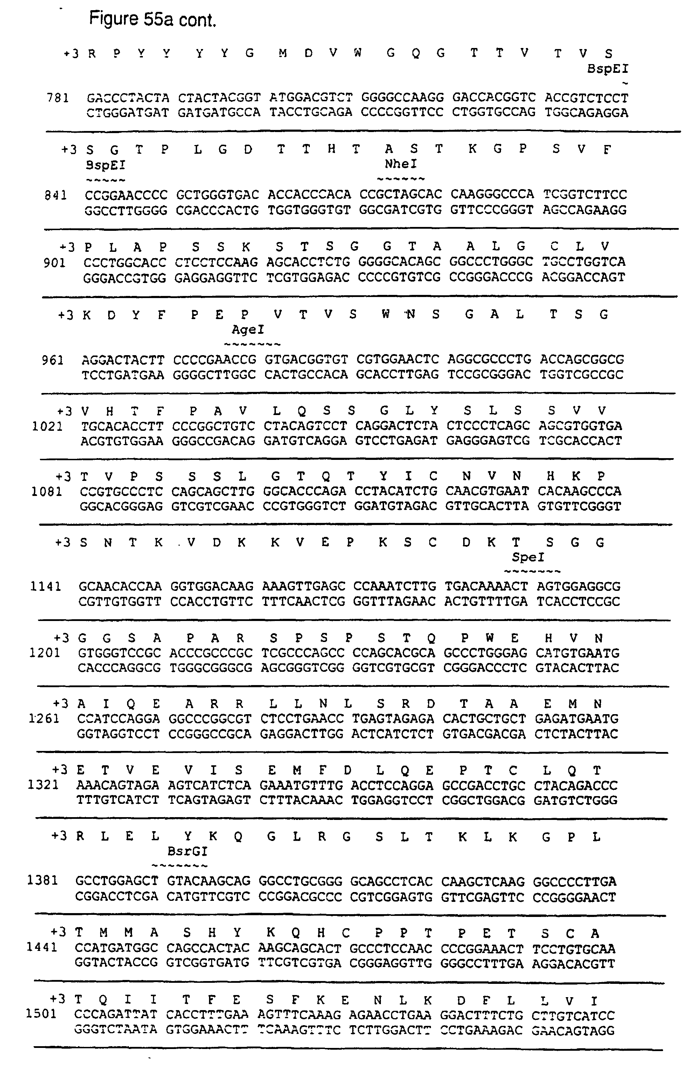

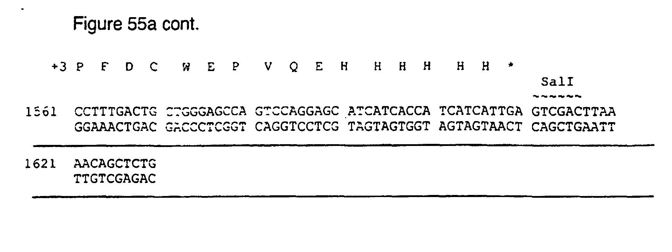

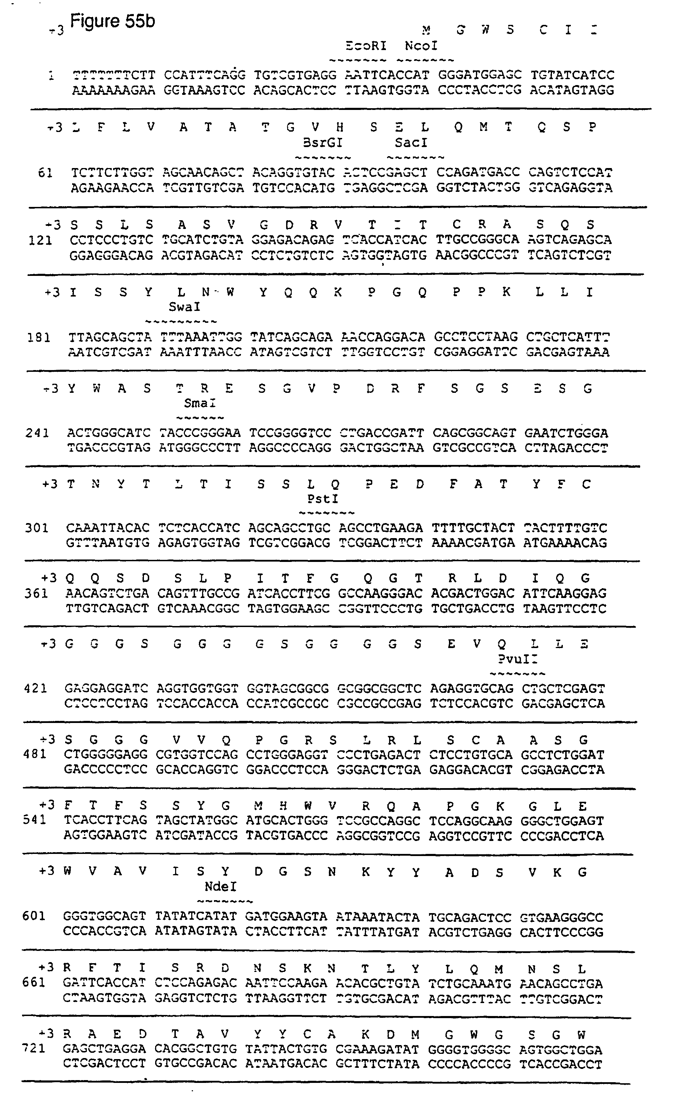

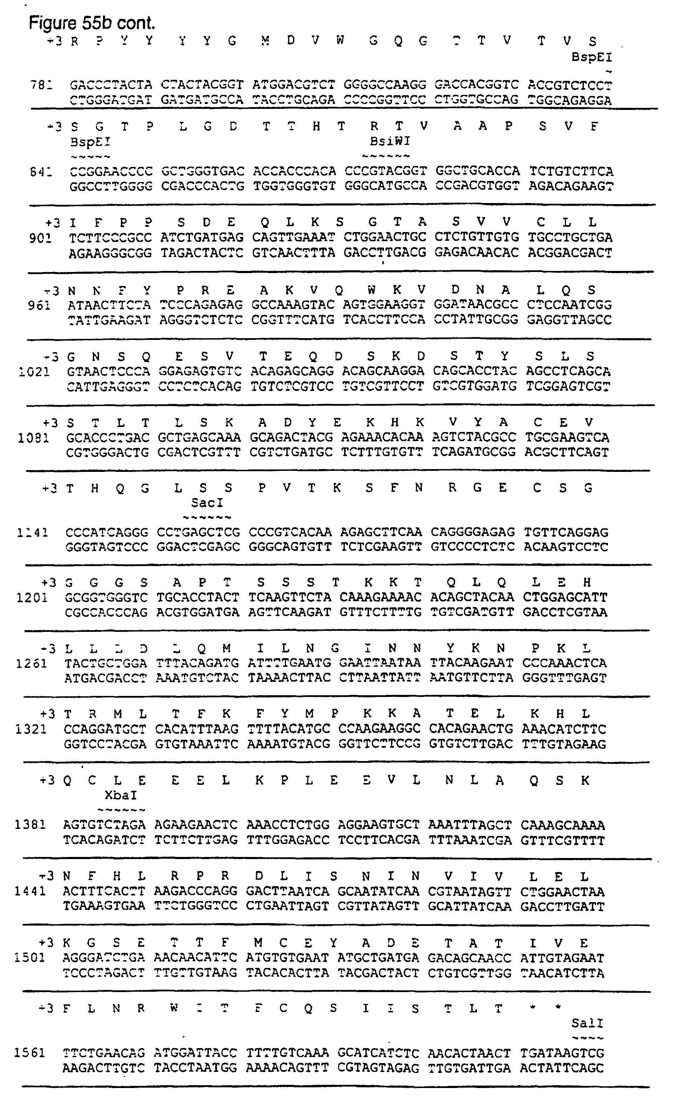

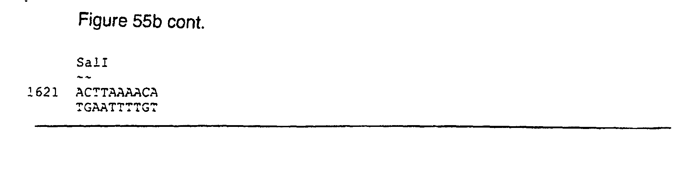

Moreover, in yet another preferred embodiment, the invention relates to the multifunctional compound of the invention, wherein said scFv fragment or said functional part thereof comprises the V_{H} and the V_{L} regions of the murine anti 17-1A antibody M79 (Göttlinger, Int. J. Cancer 38 (1986), 47), the V_{H} and the V_{L} regions of the anti-Lewis Y antibody as shown in Figure 6, the V_{H} the V_{L} regions of the anti-CD3 antibody TR66 (Traunecker, EMBO J. 10 (1991) 3655) and/or the V_{H} and V_{L} regions of the human anti-human EpCAM antibody as shown in Figure 55 (based on the corresponding human anti-human EpCAM antibody "HD70" as described in WO 98/46645).

In a more preferred embodiment, the invention relates to the multifunctional compound of the invention, wherein the T-cell co-stimulatory ligand is a cell surface molecule or a fragment thereof expressed on antigen-presenting cells (APC).

In an even more preferred embodiment, the multifunctional compound of the invention comprises an antigen-presenting cell, which is a dendritic cell.

Furthermore, in a most preferred embodiment, the present invention relates to the multifunctional compound of the invention, wherein the cell surface molecule on an APC is a T-cell co-stimulatory factor like B7-1 (CD80) or B7-2 (CD86), or adhesion proteins like LFA-3 (CD58), ICAM-1 (CD54). ICAM-2 or ICAM-3 or like the CD137-ligand.

The multifunctional compound of the invention, wherein at least one of said domains, having receptor or ligand function, is an immuno-modulating effector molecule or a fragment thereof, is still another preferred embodiment of the invention. An immuno-modulating effector molecule positively and/or negativly influences the humoral and/or cellular immune system, particulary its cellular and/or non-cellular components, its functions, and/or its interactions with other physiological systems.

In an even more preferred embodiment, said immuno-modulating effector molecule is selected from the group consisting of cytokines, chemokines, macrophage migration inhibitory factor (MIF; as described, inter alia, in Bemhagen (1998), Mol Med 76(3-4); 151-61 or Metz (1997), Adv Immunol 66,197-223) , T-cell receptors and soluble MHC molecules. Such immuno-modulating effector molecules are well known in the art and are described, inter alia, in Paul, "Fundamental immunology", Raven Press, New York (1989). In particular, known cytokines and chemokines are described in Meager, "The Molecular Biology of Cytokines" (1998), John Wiley & Sons, Ltd., Chichester, West Sussex, England; (Bacon (1998). Cytokine Growth Factor Rev 9(2):167-73; Oppenheim (1997). Clin Cancer Res 12, 2682-6; Taub, (1994) Ther Immunol 1(4), 229-46 or Michiel, (1992). Semin Cancer Biol 3(1),3-15).

Particularly preferred are cytokines which are selected from the group consisting of interleukin(s), interferon(s), TNF(s) and VEGF (Veikkola Semin Cancer Biol 9(3), 211-20), wherein said interleukin(s) comprise, but are not limited to IL-1α, IL-2, IL-3, IL-4, IL-5, IL-6, IL-7, IL-8, IL-9, IL-10, IL-11, IL-12, IL-13, IL-14, IL-15, IL-16, IL-17 and IL-18, wherein interferon(s) comprise IFN-γ as well as IFN-β and IFN-α and wherein TNF(s) comprise members of the lymphotoxin superfamily like TNF-α and TNF-β (Gruss (1996) Int J Clin Lab Res 26(3),143-59). Other suitable cytokines are well known in the art and comprise, inter alia, GM-CSF, G-CSF, M-CSF. In a particular preferred embodiment, said immuno-modulating effector molecule is a chemokine and is selected from the group consisting of IL-8, Eotaxin, GROα, GROβ, GROγ, IP-10, MCP-1, MCP-2, MCP-3, MCP-4, MIG, MIP-1α, MIP-1β, NAP-2, RANTES, 1309, Lymphotactin, SDF-1 and C5a.

Within the scope of the present invention are furthermore multifunctional compounds, wherein said domains comprise, inter alia, different immuno-modulating effector molecules, optionally, in combination with domains which are in the format of (a) scFv fragment(s). Particularly preferred are multifunctional compounds which may comprise an heterominibody with C-terminally linked cytokines, like IL-2 and GM-CSF, and N-terminally linked scFvs recognizing, for example, EpCAM as illustrated in the appended examples. These molecules are not limited to the activation of effector cells which are specifically activated by a single cytokine. By addition of two functional domains, having ligand function and comprising two different cytokines, this multifunctional component may be useful in the activation of a wider spectrum of effector cells at a tumor environment. These effector cells can thereby be stimulated to evoke an immune response against (surrounding) tumor cells and/or cells affected by malignant cell growth. These constructs retain their proper biological activities as illustrated herein in appended example 11 and may find application in the therapy and/or prevention of malignant cell growth.

In yet another preferred embodiment, the present invention relates to the multifunctional compound of the invention, wherein at least one of said domains, having receptor or ligand function, is FAS ligand (CD95L) or a fragment thereof. FAS ligand (CD95L) is well known in the art and is described, inter alia, in Janeway and Travers, "Immunologie", Spektrum Verlag Heidelberg, Berlin, Oxford (1997).

Furthermore, in yet another preferred embodiment the present invention relates to the multifunctional compound of the invention, wherein said domain(s) having receptor or ligand function, is a growth factor or a fragment thereof. Said growth factor may furthermore be a modified growth factor with binding activity to its relevant receptor but may work as an antagonist. Since many tumors or other cells (which may be (a) target(s) for the here described multifunctional compounds), express receptors for growth factors on their surface these kind of molecule may be useful in targeting. Growth factors comprise, but are not limited to, EGF (epidermal growth factor) ligands of the ErbB-receptor family , MSH, interleukin 1 (IL-1). Furthermore, growth factors and modifications therof are konown in the art and described, inter alia, in Gullick (*1996*) Cancer Surv.*27*, *339-49;* Siegall (1996)Recent Results Cancer Res 140,51-60; Carrithers 1996 Chem Biol 3(7), 537-42 or Bresnihan (1998) Rheum Dis Clin North Am 24(3);615-28.

Additionally, in a most preferred embodiment, the present invention relates to the multifunctional compound of the invention, wherein said domain(s), having receptor or ligand function, is an angiogenesis inhibitor (as defined in, inter alia, Malonne (1999) Clin Exp Metastasis 17(1), 1-14; Veikkola (1999) Semin Cancer Biol 9(3), 211-20; Desa (1999), J Immunother 22(3), 186-211; Strohmeyer (1999) Anticancer Res 19(2C), 1557-61; Folkman (1995) Nat Med 1(1),27-31) or a fragment thereof.

Preferably, the multifunctional compound of the invention comprises functional domains, having receptor or ligand function, which are of mammalian origin. Most preferably, said functional domains are of human origin.

The multifunctional compound of the invention, wherein said constant domain of an immunoglobulin light chain is of the κ type, is another subject matter of the present invention. Optionally, the constant domain of an immunoglobulin light chain can be of the λ type.

In yet another embodiment, the present invention relates to the multifunctional compound of the invention, wherein said constant immunoglobulin domains and the above-described functional receptor-ligand domains are connected by a polypeptide linker. This polypeptide linker, disposed between the immunoglobulin domains and the functional receptor-ligand domains preferably comprises plural, hydrophilic, peptide-bonded amino acids that are covalently linked to these domains.

In a more preferred embodiment, said polypeptide linker comprises an Ig-hinge region or a plurality of glycine, alanine and/or serine.

In a particularly preferred embodiment, said Ig-hinge region is an IgG hinge region.

In a most preferred embodiment, the IgG hinge region is the upper hinge region of human IgG₃.

Preferably, the multifunctional compound of the invention comprises a multifunctional compound, wherein said functional domains, having receptor or ligand function, comprise GM-CSF, IL-2 and/or (an) scFv fragment(s) comprising the V_{H} and the V_{L} regions of the human anti-human EpCAM antibody, as shown in Figure 55. Preferably, said GM-CSF and said IL-2 are C-terminally linked to said C_{H} or C_{L} domains and said scFv fragment(s) comprising the V_{H} and the V_{L} regions of the human anti-human EpCAM antibody is (are) N-terminally linked to said constant C_{H}1 or C_{L} domains.

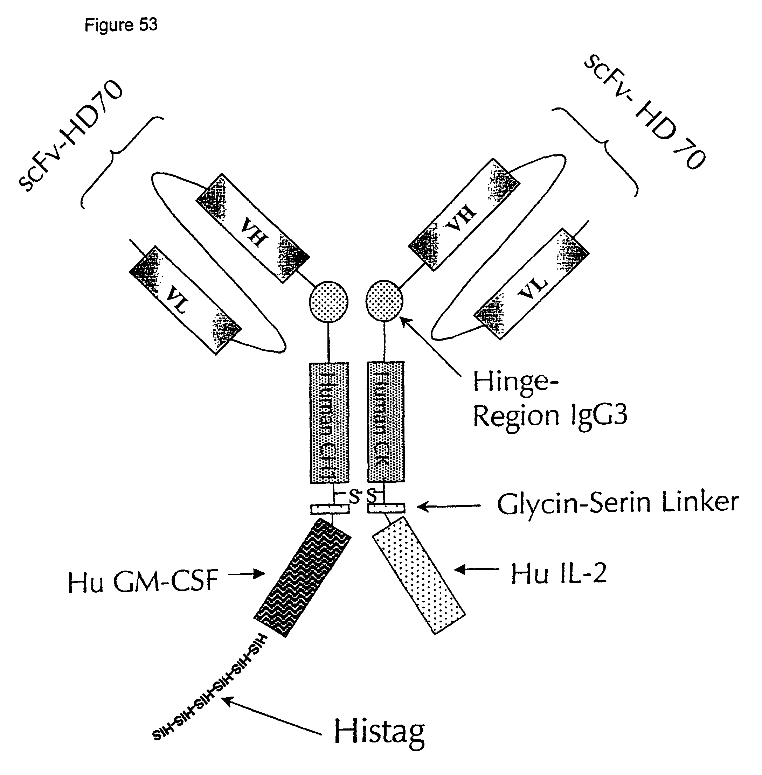

Most preferably said GM-SCF and said IL-2 are of human origin. Such a multifunctional compound is particularly suitable for therapeutic and diagnostic purposes. This multifunctional compound, comprising GM-CSF, IL-2 and said scFv fragments comprising the V_{H} and the V_{L} region of the human anti-human EpCAM antibody is shown in Figure 53. Considering that in this multifunctional compound all domains, having receptor or ligand function(s), as well as the heterodimerization domains are of human origin, it may be a specific and special value for the treatment or the prevention of malignant cell growth. Such a multifunctional compound is potentially less immunogenic that a similar molecule containing domains from other species. Human IL-2 and human GS-CSF are in this context particularly useful since these cytokines are capable of stimulating a wide and mostly different range of effector cells. A construct as depicted in Fig. 53 is particularly useful in the treatment of malignant cell growth, especially for the treatment of solid tumors like carcinomas.

In another preferred embodiment, the present invention relates to the multifunctional compound of the invention, wherein said C_{H}1 domain further comprises a histidine tag, GST, a Staphylococcus protein A tag, Lex A, a FLAG tag or a MYC-tag. These additional sequences, capable of selective binding to a solid support or to be used for purification purposes, can be either full-length polypeptide sequences or fragments thereof. Due to the fact that these tags are fused to the C_{H}1 domain, the complete multifunctional compound can conventionally be isolated.

Additionally, the present invention relates to a multifunctional compound, wherein said functional domains, having receptor or ligand function is or is derived form a non-immunoglobulin domain. The term "derived form" means in this context that the amino acid molecule of said non-immunoglobulin domain may comprise substitution(s), deletions(s), addition(s), inversion(s), duplication(s), recombinations, etc.

Apart from that, the present invention also relates to a polynucleotide encoding one and/or two polypeptide chains of the multifunctional compound as defined herein above.
Said polynucleotide may be fused to suitable expression control sequences known in the art to ensure proper transcription and translation of the polypeptide chains. Said polynucleotide may be, e.g., DNA, cDNA, RNA or synthetically produced DNA or RNA or a recombinantly produced chimeric nucleic acid molecule comprising any of those polynucleotides either alone or in combination. Preferably said polynucleotide is part of a vector. Such vectors may comprise further genes such as marker genes which allow for the selection of said vector in a suitable host cell and under suitable conditions. Preferably, the polynucleotide of the invention is operatively linked to expression control sequences allowing expression in eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory seuqences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in mammalian host cells comprise the CMV-, SV40, RSV-promoter (Rous sarcome virus), human elongation factor 1α-promoter, CMV-enhancer or SV40-enhancer. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDMB, pRc/CMV, pcDNA1, pcDNA3 (In-vitrogene), pSPORT1 (GIBCO BRL), pEF-DHFR (Mack, PNAS 92 (1995), 7021-7025). Preferably, the expression control sequences will be eukaryotic promoter systems in vectors capable of transforming or transfecting mammalian host cells. Once the vector has been incorporated into the appropriate host, the host is maintained under conditions suitable for high level expression of the nucleotide sequences, and, as desired, the collection and purification of the polypeptide of the invention may follow.

A vector comprising at least one of the above-mentioned polynucleotide is another subject matter of the present invention.
The vector of the present invention may be, e.g., a plasmid, cosmid, virus, bacteriophage or another vector used conventionally in genetic engineering, and may comprise further genes such as marker genes which allow for the selection of said vector in a suitable host cell and under suitable conditions.

In another embodiment, the present invention relates to a mammalian host cell comprising at least one vector of the present invention.

In a preferred embodiment, the mammalian host cell of the invention is a CHO cell or a myeloma cell.
Furthermore, the present invention relates to a method of producing the multifunctional compound of the invention, this method comprising culturing the host cell of the present invention under conditions that allow the synthesis of said multifunctional compound, and recovering said multifunctional compound from the culture.

Thus, the present invention allows the recombinant production of multifunctional compounds comprising sites and domains of T-cell co-stimulating ligands, antigen binding regions specific for a tumor associated antigen or proteinaceous compounds providing first activation signals for T-cells. As is evident from the foregoing, the invention provides a large family of multifunctional comprising receptor-ligand functions for any use in therapeutic and diagnostic approaches.
It will be apparent to those skilled in the art that the multifunctional compounds of the invention can be further coupled to other moieties for, e.g. drug targeting or diagnostic imaging applications. Such coupling may be conducted chemically after expression of the multifunctional compound or of the expression of the polypeptide chains of the invention or the coupling product may be engineered into the polynucleotides of the invention as discussed herein above. The polynucleotides are then expressed in a suitable host system and the expressed multifunctional compounds are collected and purified, if necessary.

In another embodiment, the present invention relates to a composition comprising the multifunctional compound of the present invention, the polynucleotide of the invention, and/or the vector of the invention and, optionally, a proteinaceous compound capable of providing the primary activation signal for T-cells. Most preferably, said composition is a pharmaceutical composition further comprising, optionally, a pharmaceutically acceptable carrier and/or the diluent and/or excipient. According to the present invention, proteinaceous compounds capable of providing the primary activation signal for T-cells in pharmaceutical and/or diagnostic compositions are monospecific or bispecific antibodies interacting with the CD-3-complex, the T-cell receptor as well as compounds including a superantigen.

A diagnostic composition comprising the multifunctional compound of the invention, the polynucleotide of the invention, and/or the vector of the invention and, optionally, a proteinous compound capable of providing the primary activation signal for T-cells and, optionally, suitable means for detection, is also a subject matter of the present invention.

In another preferred embodiment, the present invention relates to the use of the multifunctional compound of the invention, the polynucleotide of the invention and/or the vector of the invention for the preparation of a pharmaceutical composition for preventing and/or treating a malignant cell growth.

In a particularly preferred embodiment, the present invention relates to the above-described use, wherein malignant cell growth is related to malignancies of hemapoietic cells or to solid tumors.

In an even more preferred embodiment, the present invention relates to the use of the invention, wherein said malignancies of hematopoietic cells are lymphomas or leukemias.

In a most preferred embodiment, however, the present invention relates to the use of the present invention, wherein said solid tumors are carcinomas, melanomas or sarcomas.

A kit comprising the multifunctional compound of the invention and, optionally, a proteinaceous compound capable of providing the primary activation signal for T-cells, is also the subject matter of the present invention.

In another preferred embodiment, the present invention relates to the pharmaceutical composition of the invention, to the diagnostic composition of the invention or to the kit of the invention, wherein the proteinaceous compound capable of providing the primary activating signal for T-cells is a bispecific antibody interacting with the T-cell antigen CD3.

The pharmaceutical composition of the present invention may further comprise a pharmaceutically acceptable carrier, diluents and excipients. Examples of suitable pharmaceutical carriers, diluents and excipients are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Proteinaceous pharmaceutically active matter may be present in amounts between 1 ng and 10 mg per dose. Administration of the suitable compositions may be effected by different ways, e.g., by intravenous, intraperitoneal, subcutaneous, intramuscular, topical or intradermal administration. The dosage regimen will be determined by the attending physician and other clinical factors. As is well known in the medical arts, dosages for any one patent depends upon many factors, including the patient's size, body surface area, age, the particular compound to be administered, sex, time and route of administration, general health, and other drugs being administered concurrently.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

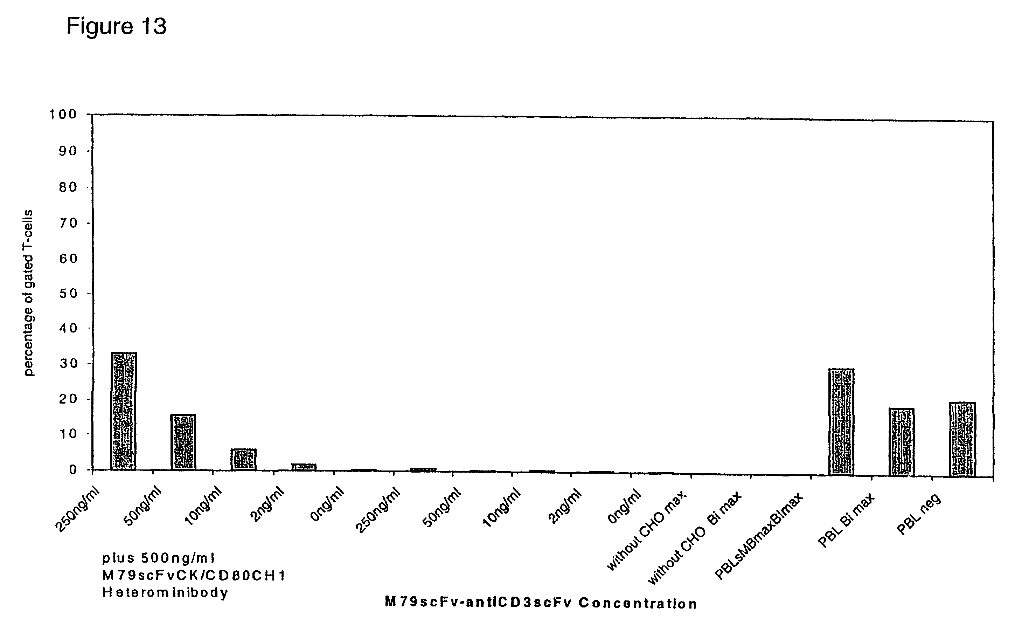

The Figures show:

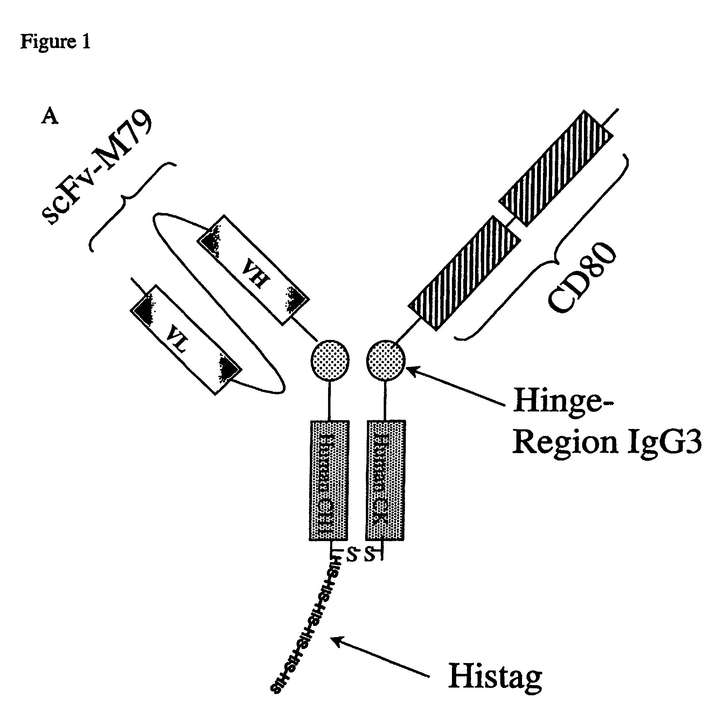

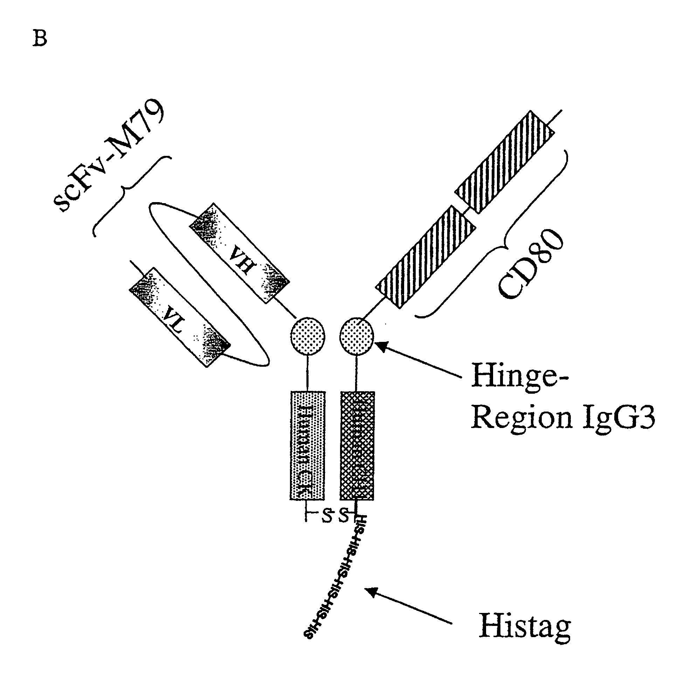

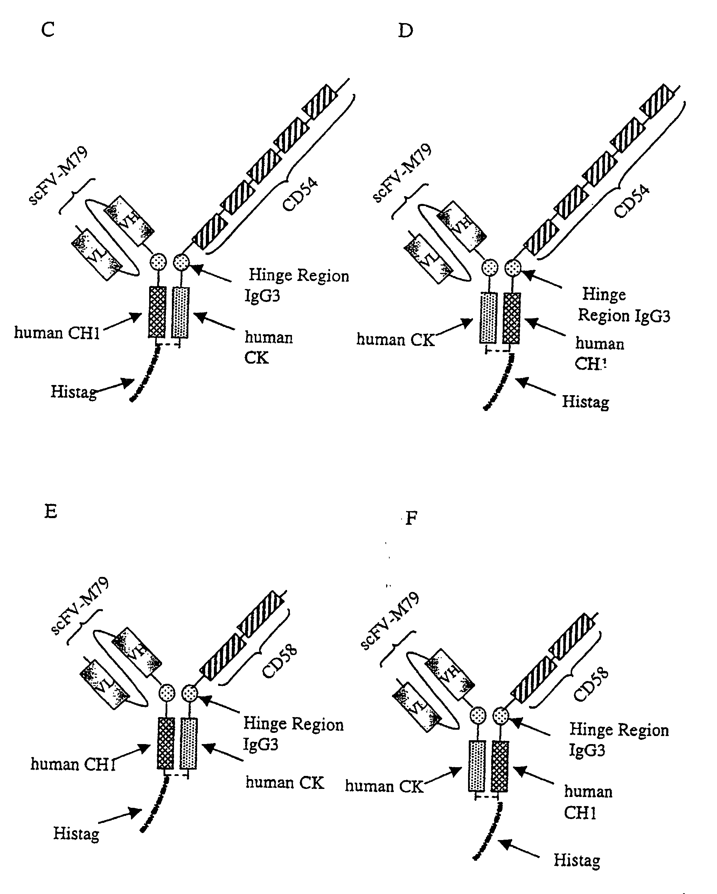

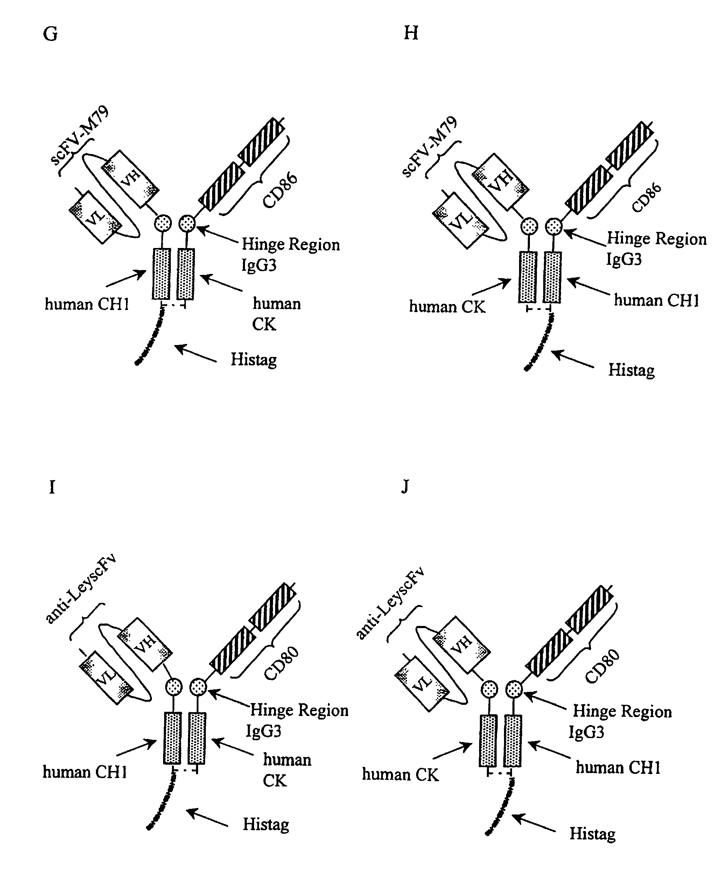

- Figure 1:: Molecular design of heterominibody M79scFvCH1/CD80CK shown on the protein level. C_{H}1 and CK indicates the first constant domain of human IgG1 heavy chain and the constant region of the human Ig-kappa light chain/ respectively, that together form the heterodimerization unit covalently joined together by a disulfide bridge (S-S). VH indicates the Ig-heavy chain variable region and VL the Ig-light chain variable region.
(B-J) Molecular design of Heterominibodies (B) M79scFvCK/CD80CH1 (C) M79scFvCH1/CD54CK (D) M79scFvCK/CD54CH1 (E) M79scFvCH1/CD58CK (F) M79scFvCK/CD58CH1 (G) M79scFvCH1/CD86CK (H) M79scFvCK/CD86CH1 (I) antiLeyscFvCH1/CD80CK (J) antiLeyscFvCK/CD80CH1 shown on the protein-level. For details see legend of Fig 1(A)

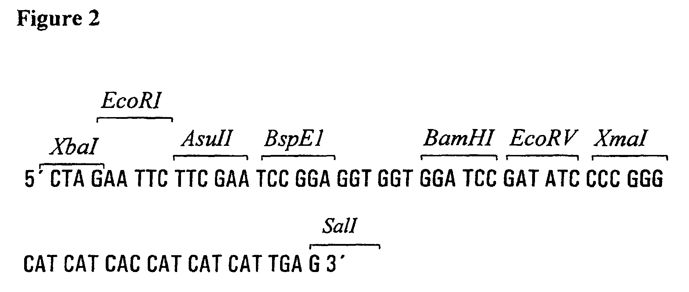

- Figure 2:: DNA-sequence designated CTI that was cloned into the multiple cloning site of the Bluescript KS vector (GenBank accession number X52327) by using the restriction sites Xbal and Sall in order to increase the number of possible cloning sites. CTI-derived restriction enzyme cleavage sites are shown.

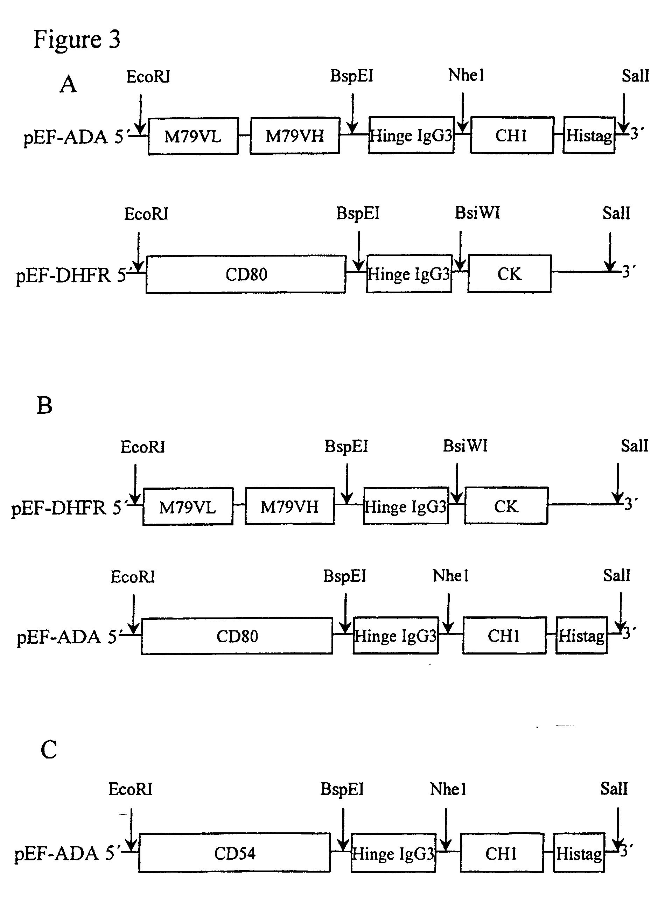

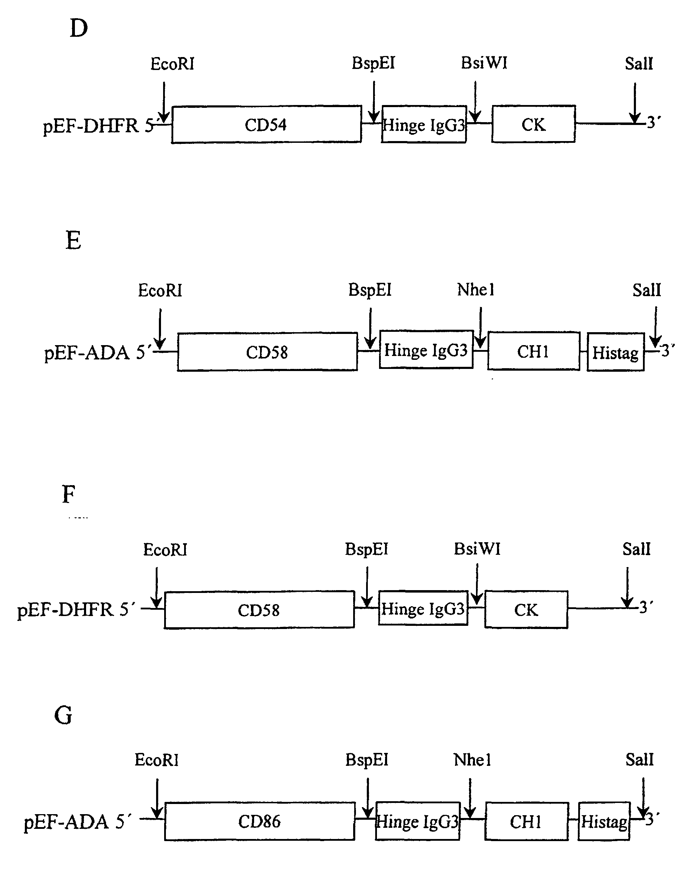

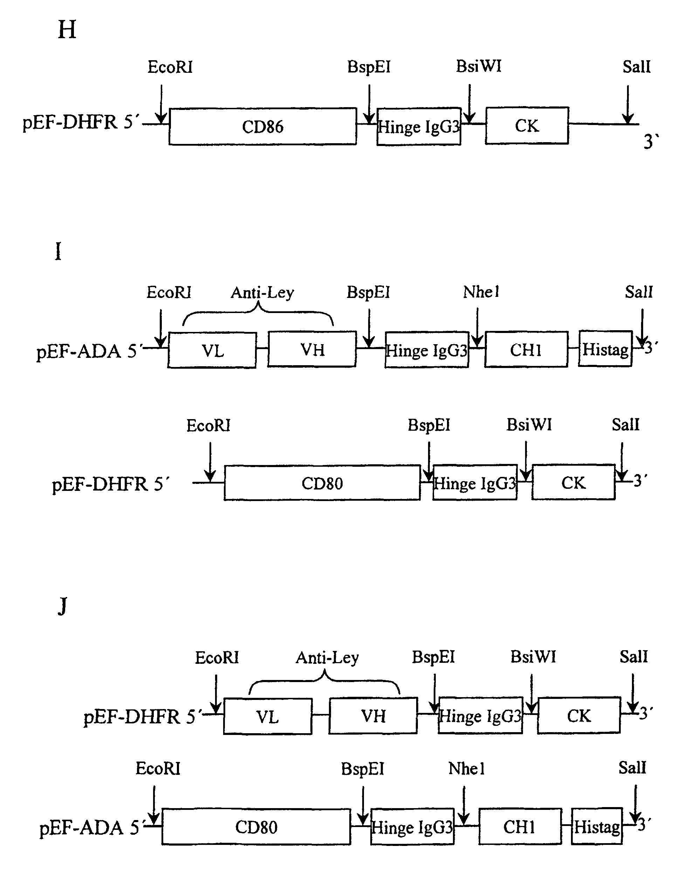

- Figure 3:: Fig 3 Molecular design of DNA-fragments encoding the single polypeptide chains of Heterominibodies (A) M79scFvCH1/CD80CK (B) M79scFvCK/CD80CH1 (C) M79scFvCH1/CD54CK (D) M79scFvCK/CD54CH1 (E) M79scFvCH1/CD58CK (F) M79scFvCK/CD58CH1 (G) M79scFvCH1/CD86CK (H) M79scFvCK/CD86CH1 (I) antiLeyscFvCH1/CD80CK (J) antiLeyscFvCK/CD80CH1 Note that some Heterominibodies share the same polypeptide chain; in these cases the corresponding polypeptide chains are only shown once. Symbols are used as in figures 1(A) and 1(B), the expression vector pEF-DHFR or pEF-ADA used for cloning and expression of individual chains is indicated. LewisY is abbreviated as Ley.

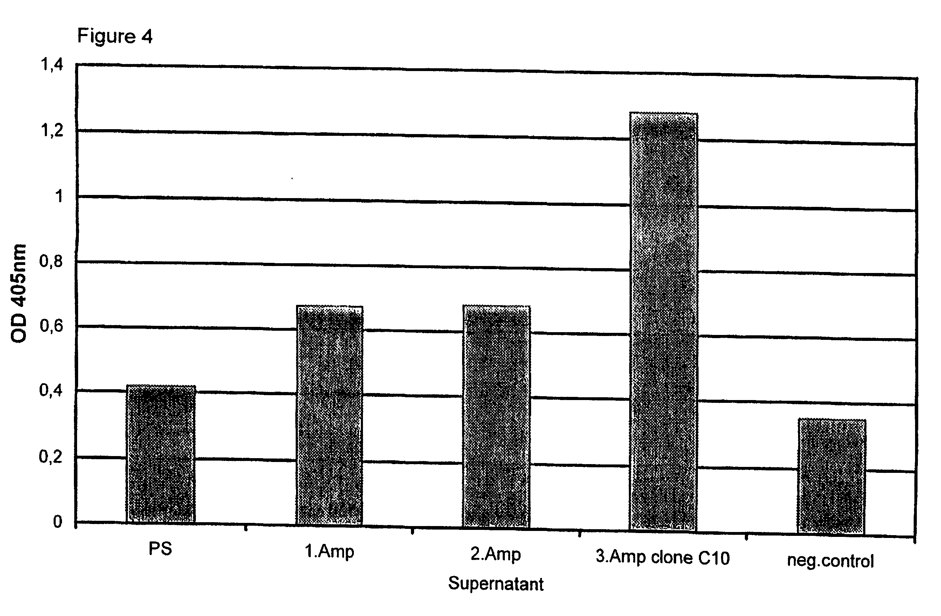

- Figure 4:: ELISA-analysis of cell-culture supematants obtained from CHO cells transfected with the expression plasmid pEF-DHFR-M79 scFv-CK pEFADA/CD80-CH1 after different amplification steps and after subcloning. 96 well ELISA plates were incubated with 50µl of soluble 17-1A antigen (50µg/ml) per well. Subsequently pure cell-culture supernatant was added as indicated. Detection was performed by a human CK biotin labeled antibody (Pierce, Cat No. 31780) diluted 1:1000 and peroxidase conjugated Avidin (Dako, Hamburg Cat No. P0347) diluted 1:1000. As negative control, well were incubated with phosphate buffered saline. The ELISA was developed by ABTS-substrate solution as described in example 2.1. OD-values were measured at 405nm using an ELISA reader.

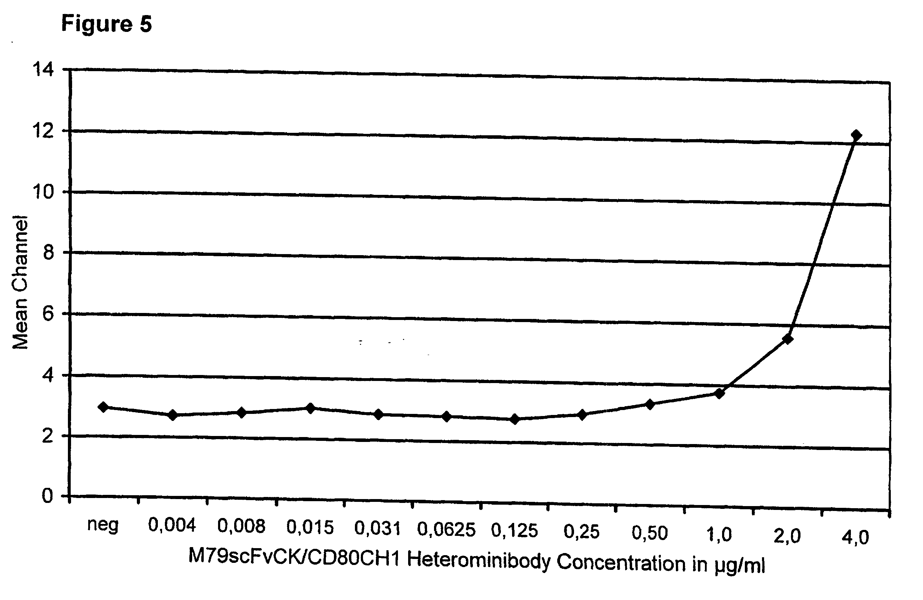

- Figure 5:: FACS analysis of heterominibody M79 scFv-CK/CD80-CH1 binding to 17-1A transfected CHO cells. 200.000 17-1A transfected CHO cells (see example 5.2) were incubated for 30 minutes with several dilutions of heterominibody M79scFv-CK/CD80-CH1 ranging from 4µg/ml to 3,9ng/ml. Thereafter, cells were washed twice in PBS and incubated for 30 minutes with a R-Pycoerythrin conjugated murine anti-human CD80 antibody (Becton Dickinson Immunocytometry Systems, San Jose CA, USA, Cat No. 340294). After two final washing steps in PBS, cells were then analyzed by flowcytrometry (FACS-SCAN Becton Dickinson Immunocytometry Systems, San Jose CA, USA).
- Figure 6:: Fig 6 DNA- as well as amino acid- sequence of antiLewisY scFv-fragment carrying a leader sequence at its N-terminus. The nucleotides from 68 to 394 encode for the Ig-light chain variable region, the nucleotides from 440 to 793 encode for the Ig-heavy chain variable domain. The restriction enzyme cleavage sites important for cloning are indicated.

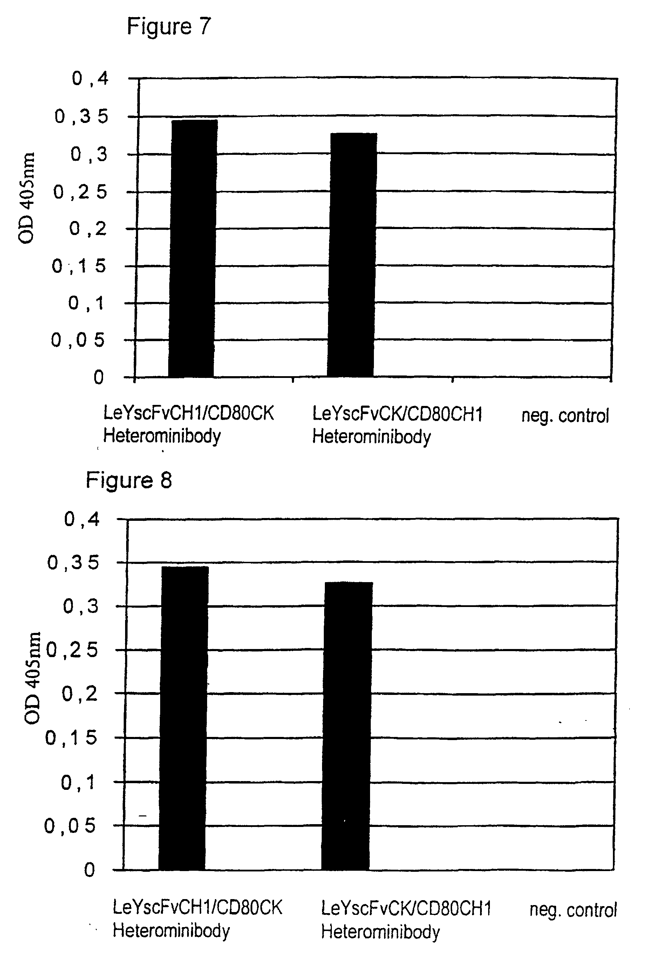

- Figure 7:: ELISA-analysis of cell-culture supernatants obtained from two different CHO-cell-transfectants, double-transfected with either the expression plasmids pEF-DHFR-anti-Lewis Y-scFv-CK and pEFADA-CD80-CH1 or with pEFDHFR-CD80-Ck and pEFADA anti-Lewis Y-scFv-CH1 after the first gene-amplification step, respectively. 96 well ELISA plates were incubated with 50µl of soluble Lewis Y-BSA conjugate (30µg/ml) per well. Subsequently pure cell-culture supernatant thereof was added as indicated. Detection was performed by a CD80-specific monoclonal antibody (Immunotech, Cat No. 1449) diluted 1:1000 and a peroxidase conjugated goat anti-Mouse IgG (Fc)-antibody (Dianova Hamburg) diluted 1:5000. As negative control, wells were incubated with phosphate buffered saline. The ELISA was developed by ABTS-substrate solution as described in example 2.1. The OD-values were measured at 405nm by an ELISA reader. LeY is used as abbreviation of anti-Lewis Y scFv.
- Figure 8:: ELISA-analysis of cell-culture supematants obtained from two different CHO-cell-transfectants, double-transfected with either the expression plasmids pEF-DHFR-anti-Lewis Y-scFv-CK and pEFADA-CD80-CH1 or with pEFDHFR-CD80-Ck and pEFADA anti-Lewis Y-scFv-CH1 after the first gene-amplification step, respectively. 96 well ELISA plates were incubated with 50µl of BSA-free anti-his tag antibody (25µg/ml) per well. Subsequently pure cell-culture supernatant was added as indicated. Detection was performed by a biotin-labeled anti-human CK antibody (Pierce, Cat No .31780) diluted 1:1000 and peroxidase conjugated Avidin (Dako, Hamburg, Cat No. P0347) diluted 1:1000. As negative control, wells were incubated with phosphate buffered saline. The ELISA was developed by ABTS -substrate solution as described in example 2.1. The OD-values were measured at 405nm by an ELISA reader. Ley is used as abbreviation of anti-Lewis Y scFv.

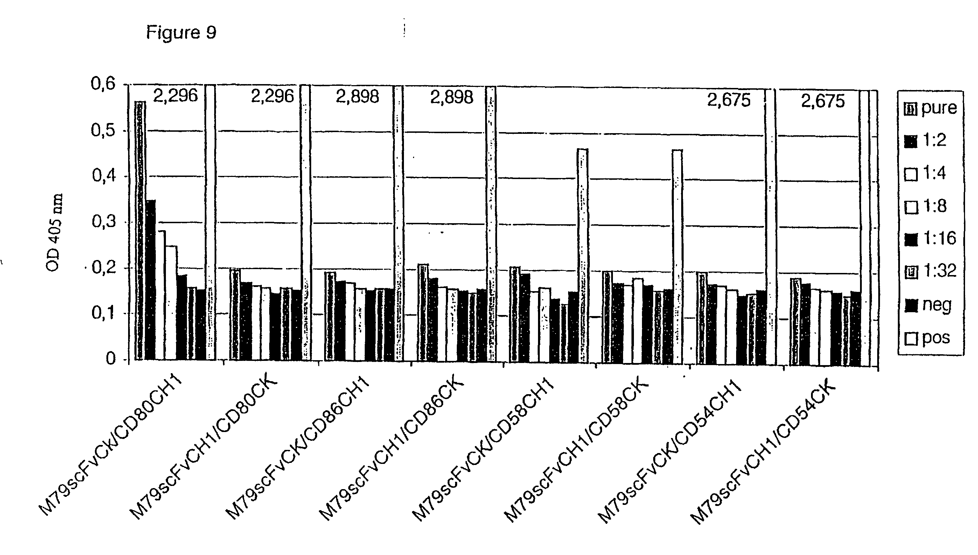

- Figure 9:: ELISA on cell-culture supernatant of both versions of heterominibody CD80, CD86, CD58, CD54 (specific detection)
Binding to the 17-1A-antigen was analyzed using recombinant 17-1A-antigen obtained by stable expression in CHO-cells as described (Mack et.al. Proc.Natl.Acad.Sci. 92 (1995)7021-7025 and example 4.4) The antigen was immobilized on 96 well U bottom ELISA plates (nunc maxisorb) at a concentration of 50µg/ml phosphate buffered saline PBS. Coating was carried out at 4°C for 12 hours with 50µl followed by washing once with (PBS) 0,05%Tween. The ELISA was then blocked for 1 hour with PBS/3%bovine serum albumin (BSA) and washed again once. Subsequently, the cell-culture supematant was added undiluted and at several dilutions (pure, 1:2, 1:4, 1:8, 1:16, 1:32) and incubated for 2 hours. Specific detection was dependent on the type of costimulatory proteins associated with the different heterobody version. Specific antibodies anti CD54, anti CD58, anti CD80 and anti CD86 were used all diluted 1:1000 (for details see table 4.4.) . After three times of washing with PBS 0,05% Tween20, a polyclonal peroxidase-conjugated goat anti-mouse IgG-antibody (Fc-specific) (Dianova Hamburg) diluted 1:5000 was added and incubated at room temperature for one hour. After four times of washing with PBS 0,05% Tween20, the ELISA was finally developed by adding the ABTS substrate as described in Example 4.4. As negative control the plates were incubated with PBS instead of heterominibody constructs. The colored precipitate was measured at 405 nm using an ELISA-reader.

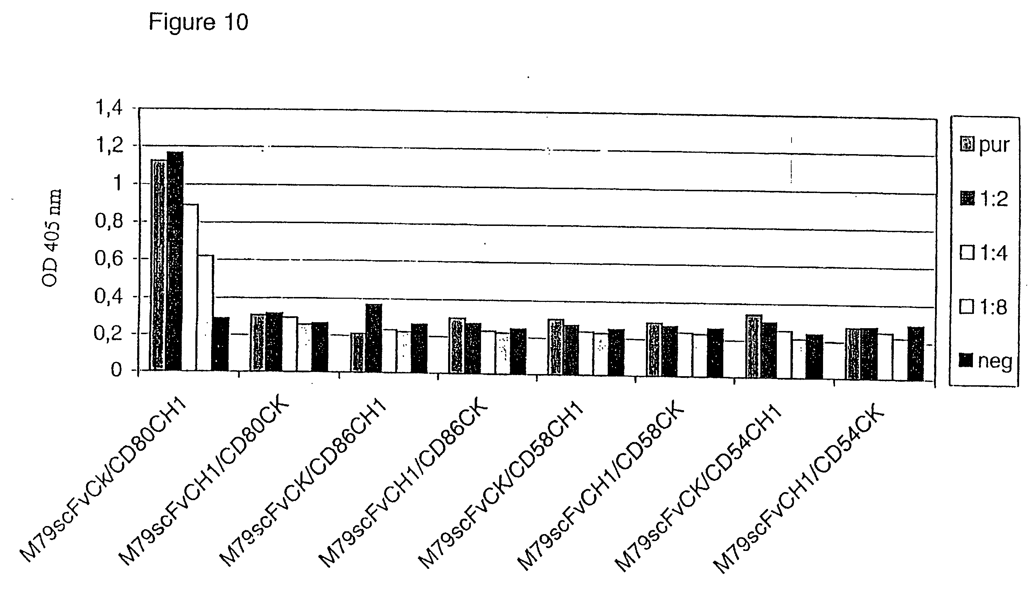

- Figure 10:: ELISA on cell-culture supernatant of both versions of heterobody CD80, CD86, CD58, CD54 ( anti human Ckappa detection)
An anti-His-tag-antibody (DIANOVA; Hamburg Cat No. DIA 910) diluted 1:40 was coated to 96-well plates as described above. Supernatants of all heterominibody versions were added pure and in dilutions 1:2, 1:4, 1:8. A biotinylated anti-human Ckappa antibody (Pierce, Cat. No. 31780) followed by peroxidase-conjugated streptavidin (1:1000) (Dako, Hamburg, Cat No. P0347) was used for detection of bound Heterominibodies (see Table 4.4). After four times of washing with PBS 0,05% Tween20, the ELISA was finally developed by adding the ABTS substrate as described in Example 4.4. As negative control the plates were incubated with PBS instead of heterominibody constructs. The colored precipitate was measured at 405 nm using an ELISA-reader.

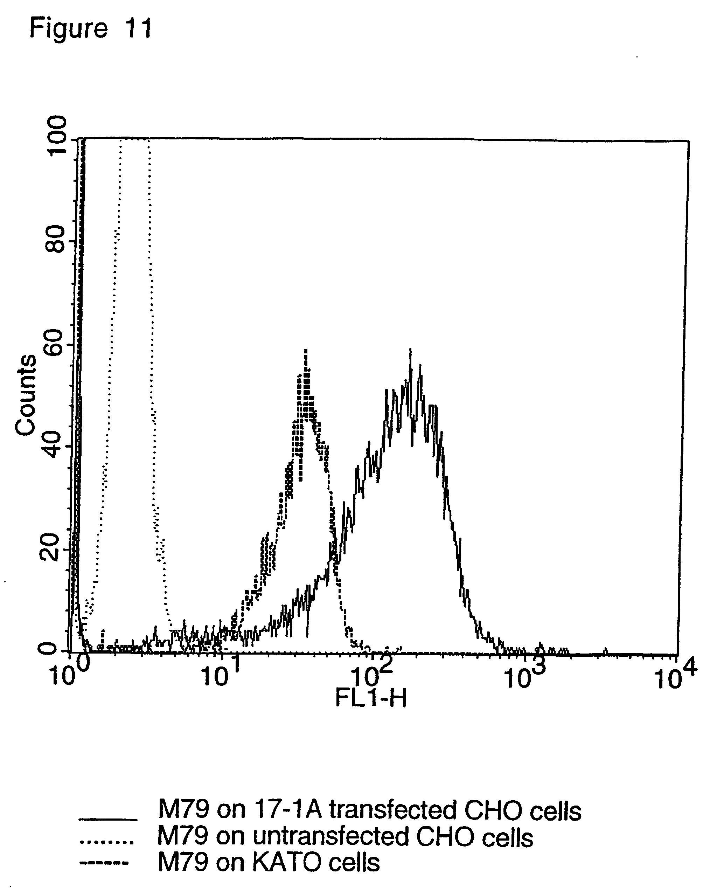

- Figure 11:: FACS-Control of the CHO cells after transfection with 17-1A. The expression of transmembrane 17-1A was increased by stepwise gene amplification induced by subsequent addition of increasing concentrations of the DHFR inhibitor MTX to a final concentration of 500nM, with the concentration steps in between 20nM and 100nM. These cells were tested for membrane expression of 17-1A by flow cytometry at a concentration of 10µg/ml of the 17-1A-specific antibody M79 (Göttinger, In6. J. Cancer 38(1986) 47-53) followed by a FITC-labeled polyclonal Goat Anti Mouse IgG + IgM (H+L) antibody diluted 1:100 in PBS. As negative control untransfected CHO cells were used whereas the 17-1A-positive human gastric cancer celline Kato, obtained from ATCC served as positive control.

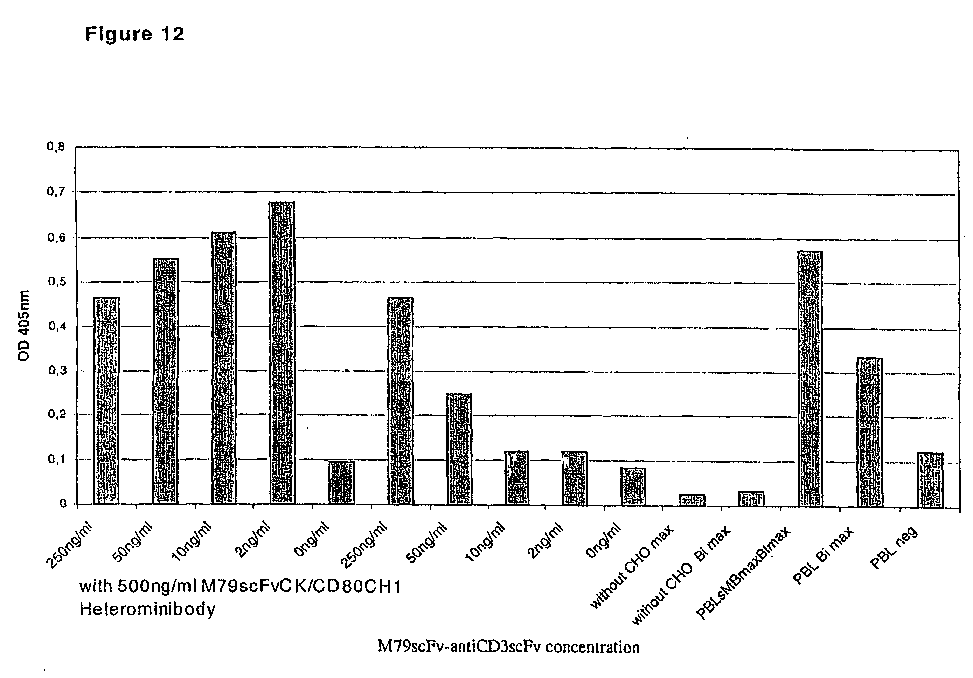

- Figure 12:: BrdU-incorporation of CD4+CD45RA+T-cells after stimulation with heterominibody M79CK/CD80CH1 and/or M79scFv-antiCD3scFv. After 3 days of stimulation the cells were incubated with BrdU for 14 hours. The assay was performed as recommended in the product description by Boehringer Mannheim Cat.No. 1647229. The OD values were measured at 450nm using an ELISA reader.
Abbreviations:
without CHO max = without 17-1A-transfected CHO-cells plus 250 ng/ml bispecific single-chain antibody M79scFv-anti-CD2scFv plus 500 ng/ml M79scFv CK/CD80CH1-heterominibody
without CHO Bimax = without 17-1A-transfected CHO-cells plus 250 ng/ml bispecific single-chain antibody M79scFv-anti-CD2scFv
PBLsMBmaxBimax = 17-1A-transfected CHO-cells plus unseparated mononuclear cells from peripheral blood plus 250 ng/ml bispecific single-chain antibody M79scFv-anti-CD3scFv plus 500 ng/ml M79scFv CK/CD80 CH1-heterominibody
PBLBimax = 17-1A-transfected CHO-cells plus unseparated mononuclear cells from peripheral blood plus 250 ng/ml bispecifc single-chain antibody M79scFv-anti-CD2scFv
PBL neg = negative control consisting of 17-1A-transfected CHO-cells plus unseparated mononuclear cells from peripheral blood
- Figure 13:: Percentage of CD4+ CD45RA+CD45R0- T-cells after 3 days of stimulation of CD4+CD45RA+T-cells with heterominibody M79CK/CD80CH1 and/or M79scFv-antiCD3 analyzed by FACS CD45RA and CD45RO expression levels were analyzed by flowcytometry after 3 days of stimulation of CD4+CD45RA+T-cells with 500ng/ml heterominibody M79CK/CD80CH1 and/or 250, 50, 10, 2 ng/ml M79scFv-antiCD3 scFv. Figure 5.4.1 shows the percentage of CD4+CD45RA-CD45RO+ T-cells of all gated cells. 100.000 cells were washed once with PBS and incubated for 30 minutes with a R-Phycoerythrin conjugated anti-human CD45RA antibody (2H4 Coulter) diluted 1:50 and with a FITC conjugated anti-human CD45RO antibody (UHCL-1 DAKO Hamburg) diluted 1:50 and washed again once with PBS. As positive control PBMCs were stimulated with 500ng/ml heterominibody M79CK/CD80CH1 and/or 250 ng/ml M79scFv-antiCD3scFv. For negative controls unstimulated PBMC, and purified CD4+CD45RO- T-cells stimulated with 500ng/ml heterominibody M79CK/CD80CH1 and/or 250 ng/ml M79scFv-antiCD3 scFv without 17-1A transfected CHO cells. Abbreviations see legend Fig. 12.

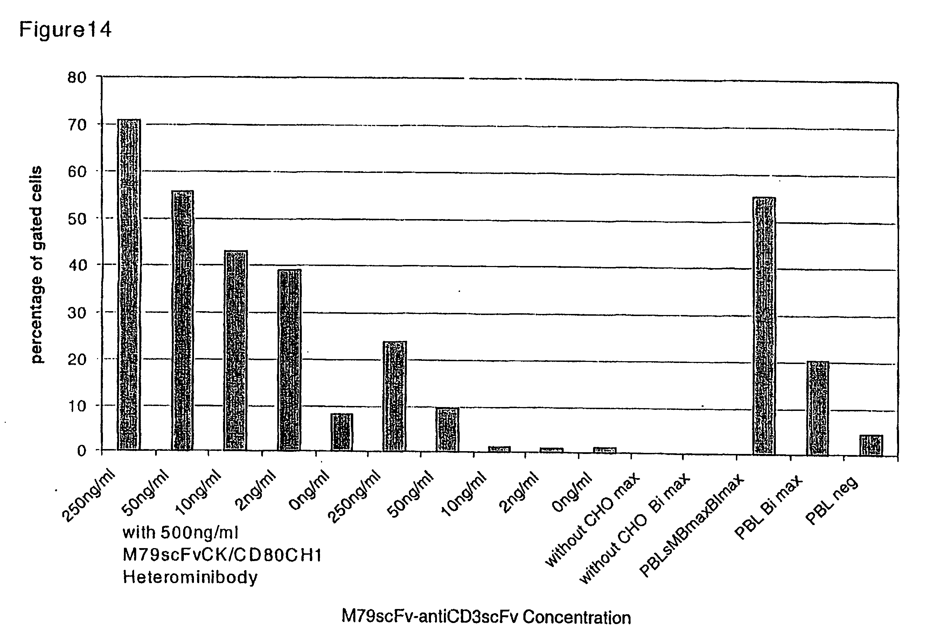

- Figure 14:: Percentage of CD4+ CD45RA-CD45R0+ T-cells after 6 days of stimulation of CD4+CD45RA+T-cells with heterominibody M79CK/CD80CH1 and/or M79scFv-antiCD3 scFv analyzed by FACS CD45RA and CD45RO expression levels were analyzed by flowcytometry after 6 days of stimulation of CD4+CD45RA+T-cells with 500ng/ml heterominibody M79CK/CD80CH1 and/or 250, 50, 10, 2 ng/ml M79scFv-antiCD3 scFv. Figure 5.4.1 shows the percentage of CD4+CD45RA-CD45RO+ T-cells of all gated cells. 100.000 cells were washed once with PBS and incubated for 30 minutes with a R-Phycoerythrin conjugated anti human CD45RA antibody (2H4 Coulter) diluted 1:50 and with a FITC conjugated anti human CD45RO antibody (UHCL-1 DAKO Hamburg) diluted 1:50 and washed again once with PBS. As positive control PBMCs were stimulated with 500ng/ml heterominibody M79CK/CD80CH1 and/or 250 ng/ml M79scFv-antiCD3 scFv. For negative controls unstimulated PBMC, and purified CD4+CD45RO- T-cells were stimulated with 500ng/ml heterominibody M79CK/CD80CH1 and/or 250 ng/ml M79scFv-antiCD3 scFv without 17-1A transfected CHO cells. Abbreviations see legend Fig. 12.

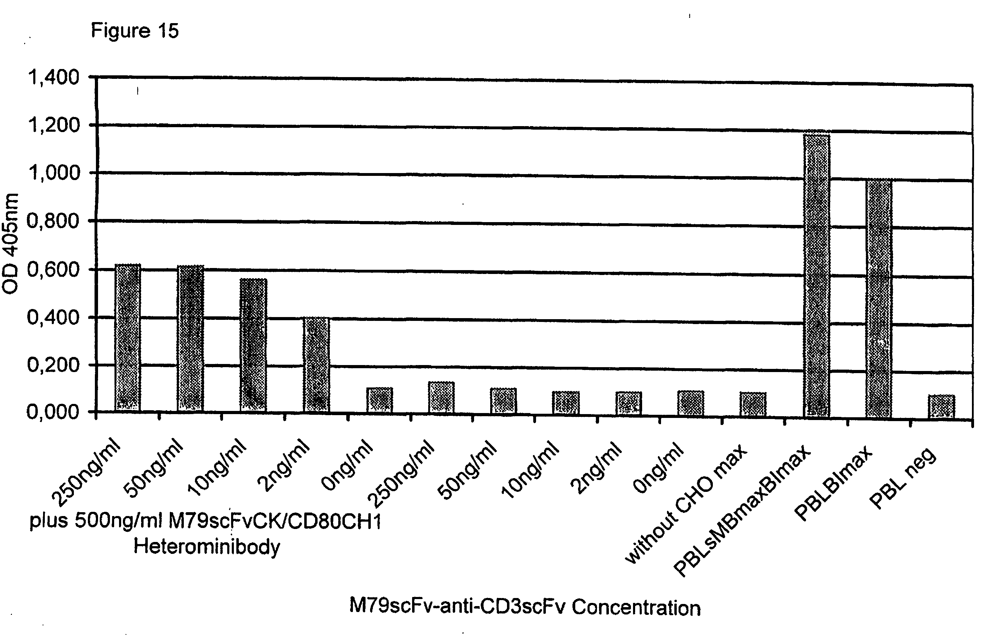

- Figure 15:: γ-IFN ELISA analysis of CD4+ CD45RA+T-cells after 3 days of stimulation with heterominibody M79CK/CD80CH1 and/or M79scFv-anti CD3 scFv. Cell-culture supernatant was diluted 1:5 prior to ELISA-analysis, the γ standard (supplied with the test-kit) was used as positive control. As negative control, wells were incubated with cell-culture-medium. The ELISA was performed according to manufacturers' manual recommendation (Genzyme DuoSet, Genzyme Diagnostics Cambridge, MA USA Cat.No. 80-3932-00) and developed by ABTS -substrate solution as described in example 2.1 The OD-values were measured at 405nm using an ELISA reader. Abbreviations see legend Fig. 12.

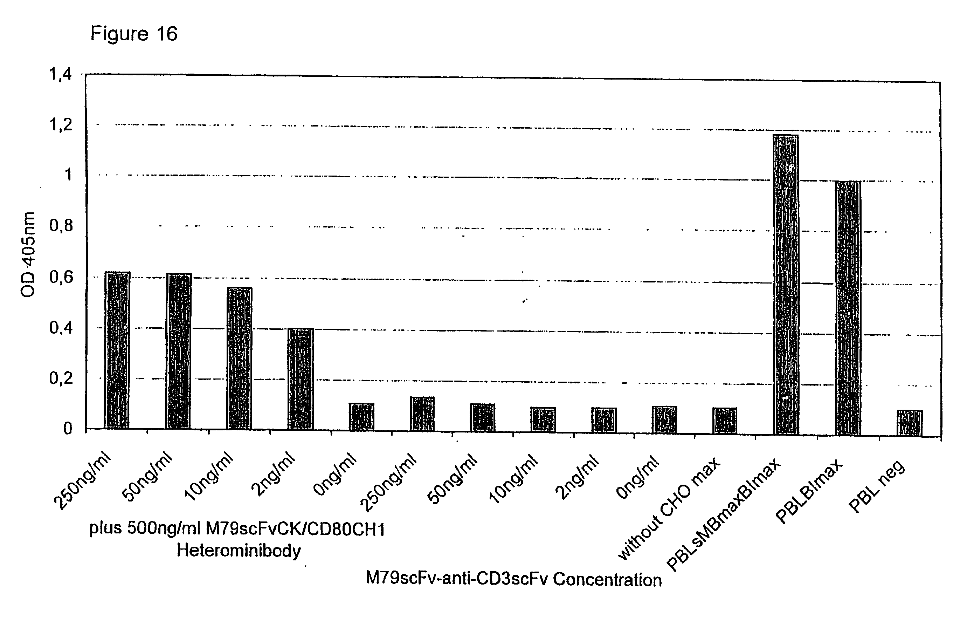

- Figure 16:: γ-IFN ELISA analysis of CD4+ CD45RA+T-cells after 6 days of stimulation with heterominibody M79CK/CD80CH1 and/or M79scFv-anti CD3 scFv. Cell-culture supernatant was diluted 1:5 prior to ELISA-analysis, the γ-IFN standard (supplied with the test-kit) was used as positive control. As negative control, wells were incubated with cell-culture-medium. The ELISA was performed according to manufacturers' manual (Genzyme DuoSet, Genzyme Diagnostics Cambridge, MA USA Cat.No. 80-3932-00) and developed by ABTS-substrate solution as described in example 2.1 The OD-values were measured at 405nm using an ELISA reader. Abbreviations see legend Fig. 12.

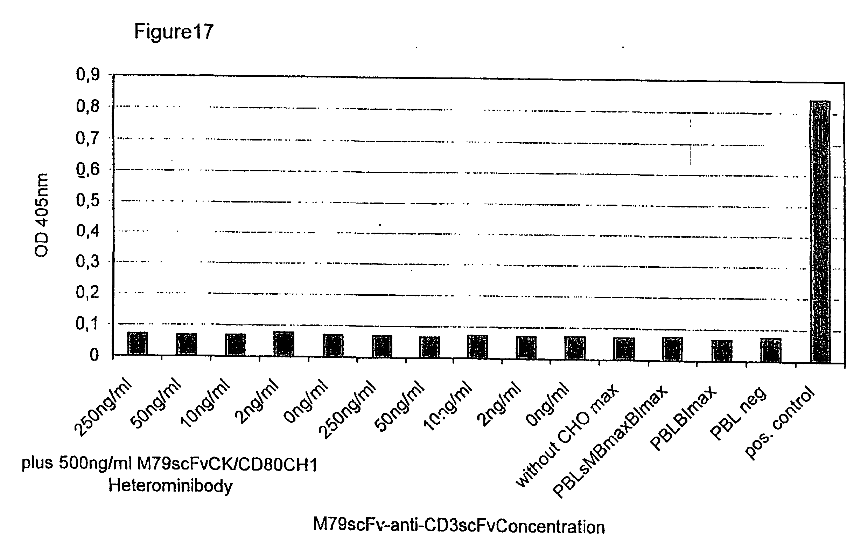

- Figure 17:: IL-5 ELISA analysis of CD4+ CD45RA+T-cells after 3 days of stimulation with heterominibody M79CK/CD80CH1 and/or M79scFv-anti CD3 scFv. Cell-culture supernatant was analyzed undiluted, the IL-5 Standard (supplied with the test-kit) was used as positive control. As negative control, wells were incubated with cell-culture-medium. The ELISA was performed according to manufacturers' manual (Genzyme DuoSet, Genzyme Diagnostics Cambridge, MA USA Cat.No. 80-5025-00) and developed by ABTS -substrate solution as described in example 2.1 The OD-values were measured at 405nm using an ELISA reader. Abbreviations see legend Fig. 12.

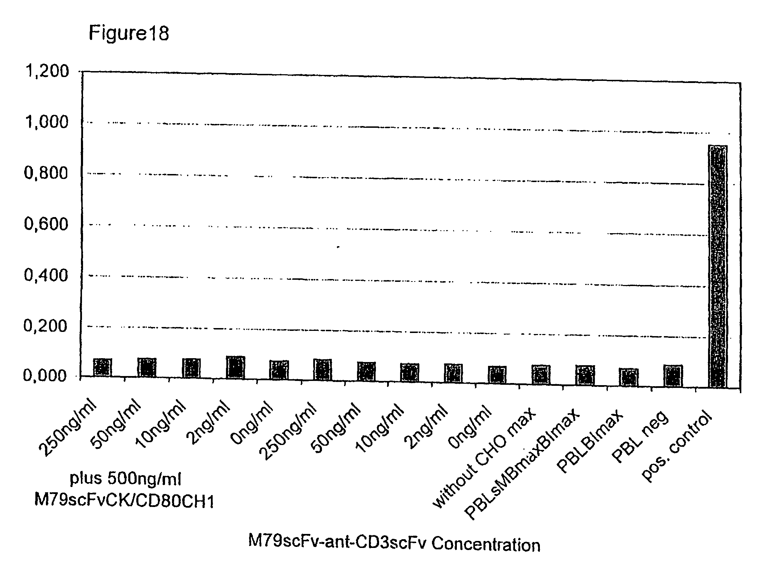

- Figure 18:: IL-5 ELISA analysis of CD4+ CD45RA+T-cells after 6 days of stimulation with heterominibody M79CK/CD80CH1 and/or M79scFv-anti CD3 scFv. Cell-culture supernatant was analyzed undiluted, the IL-5 standard (supplied with the test-kit) was used as positive control. As negative control, wells were incubated with cell-culture-medium. The ELISA was performed according to manufacturers' manual (Genzyme DuoSet, Genzyme Diagnostics Cambridge, MA USA Cat.No. 80-5025-00) and developed by ABTS -substrate solution as described in example 2.1 The OD-values were measured at 405nm using an ELISA reader. Abbreviations see legend Fig. 12.

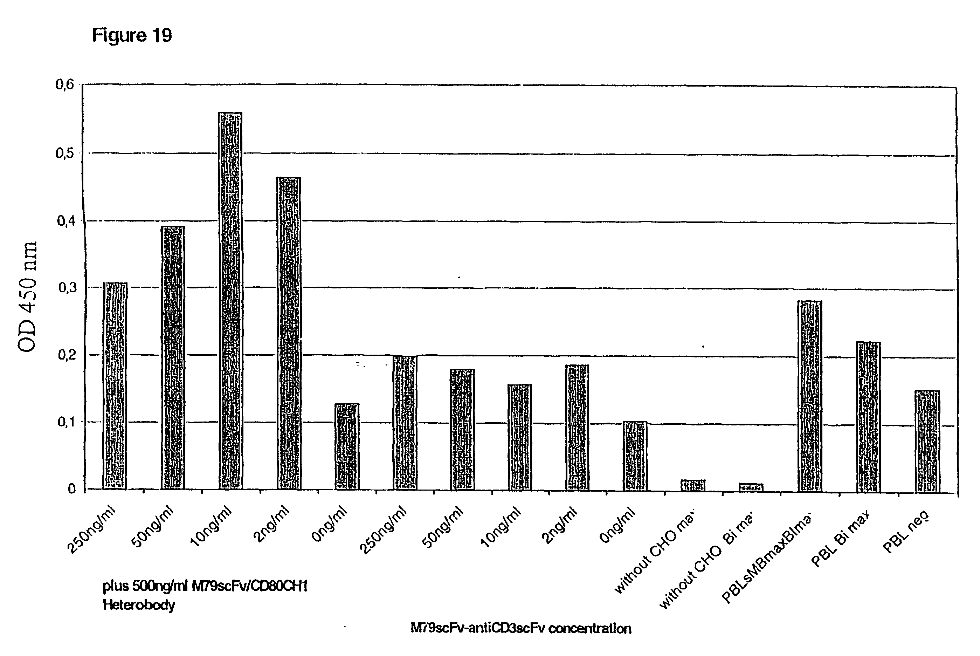

- Figure 19:: BrdU-incorporation of CD8+CD45RA+cells after 3 days of stimulation with heterominibody M79CK/CD80CH1 and/or M79scFv-anti CD3 scFv. After 3 days of stimulation cells were incubated with BrdU for 14 hours. The assay was performed as recommended in the product description by Boehringer Mannheim Cat.No. 1647229. The OD values were measured at 450nm using an ELISA reader. Abbreviations see legend Fig. 12.

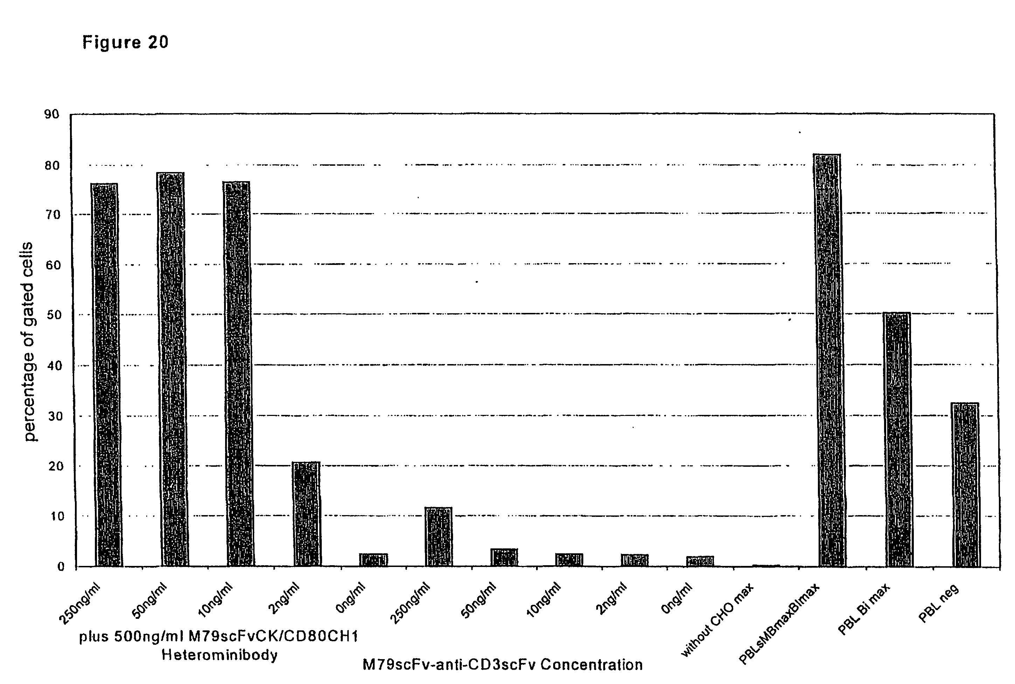

- Figure 20:: Percentage of CD8+ CD45RA-CD45R0+ T-cells after 4 days of stimulation of CD8+CD45RA+T-cells with heterominibody M79CK/CD80CH1 and/or M79scFv-antiCD3 scFv analyzed by FACS CD45RA and CD45RO expression levels were analyzed by flowcytometry after 4 days of stimulation of CD8+CD45RA with 500ng/ml heterominibody M79CK/CD80CH1 and/or 250, 50, 10, 2 ng/ml M79scFv-antiCD3 scFv. Figure 5.4.1 shows the percentage of CD8+CD45RA-CD45RO+ T-cells of all gated cells. 100.000 cells were washed once with PBS and incubated for 30 minutes with a R-Phycoerythrin conjugated anti-human CD45RA antibody (2H4 Coulter) diluted 1:50 and with a FITC conjugated anti-human CD45RO antibody (UHCL-1 DAKO Hamburg) diluted 1:50 and washed again once with PBS. As positive control PBMCs were stimulated with 500ng/ml heterominibody M79CK/CD80CH1 and/or 250 ng/ml M79scFv-antiCD3 scFv. For negative controls unstimulated PBMC, and purified CD8+CD45RO- T-cells were stimulated with 500ng/ml heterominibody M79CK/CD80CH1 and/or 250 ng/ml M79scFv-antiCD3 scFv without 17-1A transfected CHO cells. Abbreviations see legend Fig. 12.

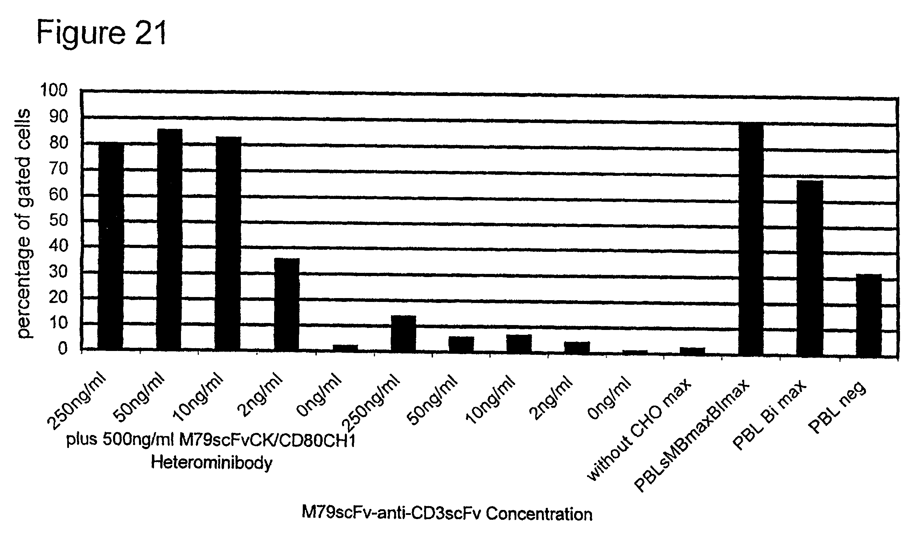

- Figure 21:: Percentage of CD8+ CD45RA-CD45R0+ T-cells after 6 days of stimulation of CD8+CD45RA+T-cells with heterominibody M79CK/CD80CH1 and/or M79scFv-antiCD3scFv analyzed by FACS CD45RA and CD45RO expression levels were analyzed by flowcytometry after 6 days of stimulation of CD8+CD45RA+T-cells with 500ng/ml heterominibody M79CK/CD80CH1 and/or 250, 50, 10, 2 ng/ml M79scFv-antiCD3 scFv. Figure 5.4.1 shows the percentage of CD8+CD45RA-CD45RO+ T-cells of all gated cells. 100.000 cells were washed once with PBS and incubated for 30 minutes with a R-Phycoerythrin conjugated anti human CD45RA antibody (2H4 Coulter) diluted 1:50 and with a FITC conjugated anti-human CD45RO antibody (UHCL-1 DAKO Hamburg) diluted 1:50 and washed again once with PBS. As positive control PBMCs were stimulated with 500ng/ml heterominibody M79CK/CD80CH1 and/or 250 ng/ml M79scFv-antiCD3 scFv. For negative controls unstimulated PBMC, and purified CD8+CD45RO- T-cells were stimulated with 500ng/ml heterominibody M79CK/CD80CH1 and/or 250 ng/ml M79scFv-antiCD3 scFv without 17-1A transfected CHO cells. Abbreviations see legend Fig. 12.

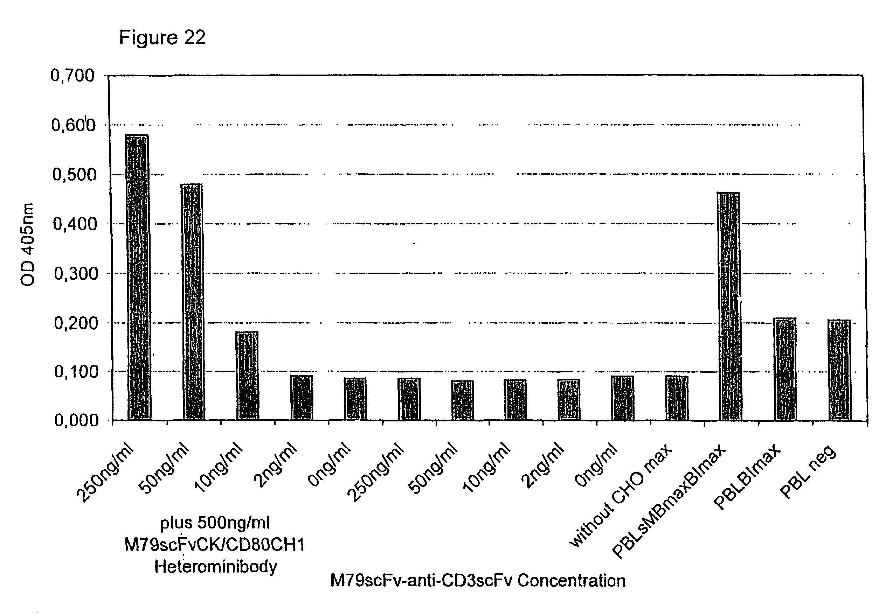

- Figure 22:: TNF-α ELISA analysis of CD8+ CD45RA+T-cells after 4 days of stimulation with heterominibody M79CK/CD80CH1 and/or M79scFv-anti CD3 scFv. Cell-culture supernatant was analyzed undiluted, the TNF-α standard (supplied with the test-kit) was used as positive control. As negative control, wells were incubated with cell-culture-medium. The ELISA was performed according to manufacturers' manual (Genzyme DuoSet, Genzyme Diagnostics Cambridge, MA USA Cat.No. 80-3933-00) and developed by ABTS -substrate solution as described in example 2.1 The OD-values were measured at 405nm using an ELISA reader. Abbreviations see legend Fig. 12.
- Figure 23:: Molecular design of heterominibody M79scFv-CK-antiCD3scFv/CD80CH1 shown on the protein level. CH1 and CK indicates the first constant domain of human IgG1 heavy chain and the constant region of the human Ig-kappa light chain/ respectively, that together form the heterodimerization unit covalently joined together by a disulfide bridge (S-S). VH indicates the Ig-heavy chain variable region and VL the Ig-light chain variable region.

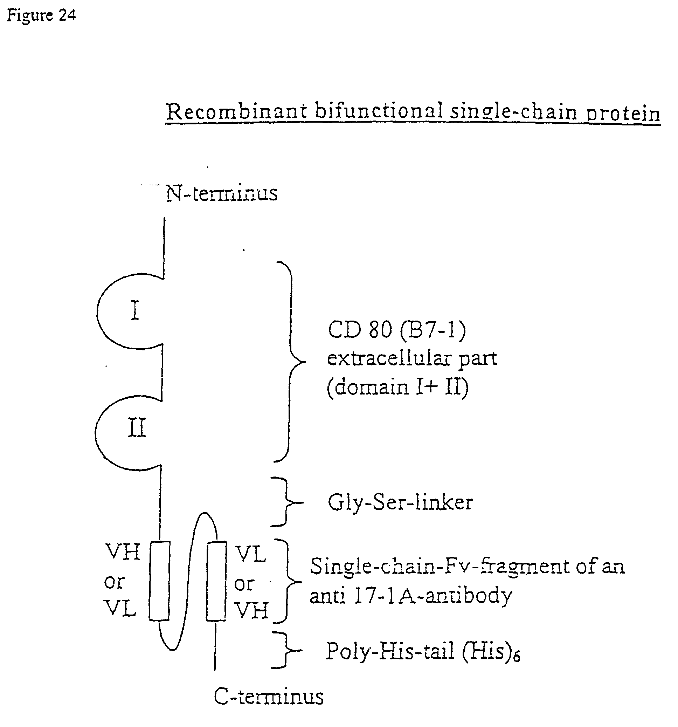

- Figure 24:: Design of various bifunctional CD80-scFv-constructs showing the construction elements on the protein-level. VH indicates the variable region of the Eg-heavy chain, VL that of the Ig-light chain.

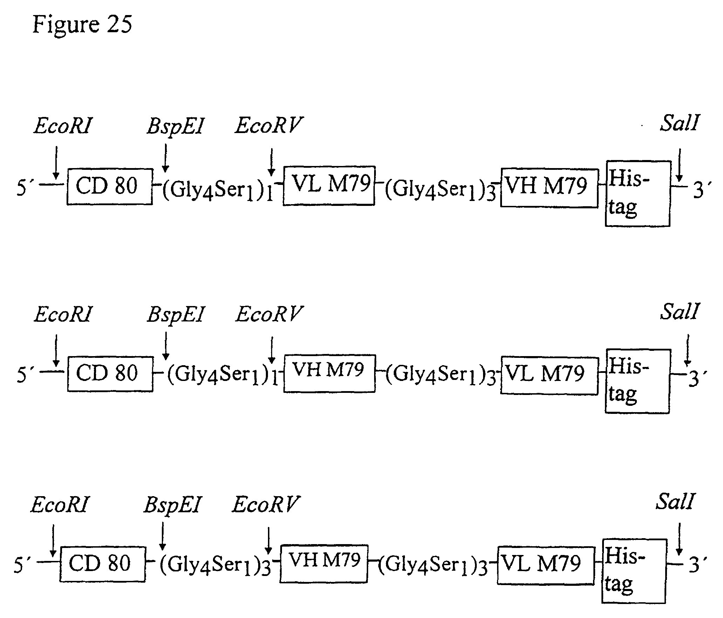

- Figure 25:: Design of various bifunctional CD80-scFv-construct showing the construction elements on the DNA-level as well as the restriction enzyme cleavage sites used.

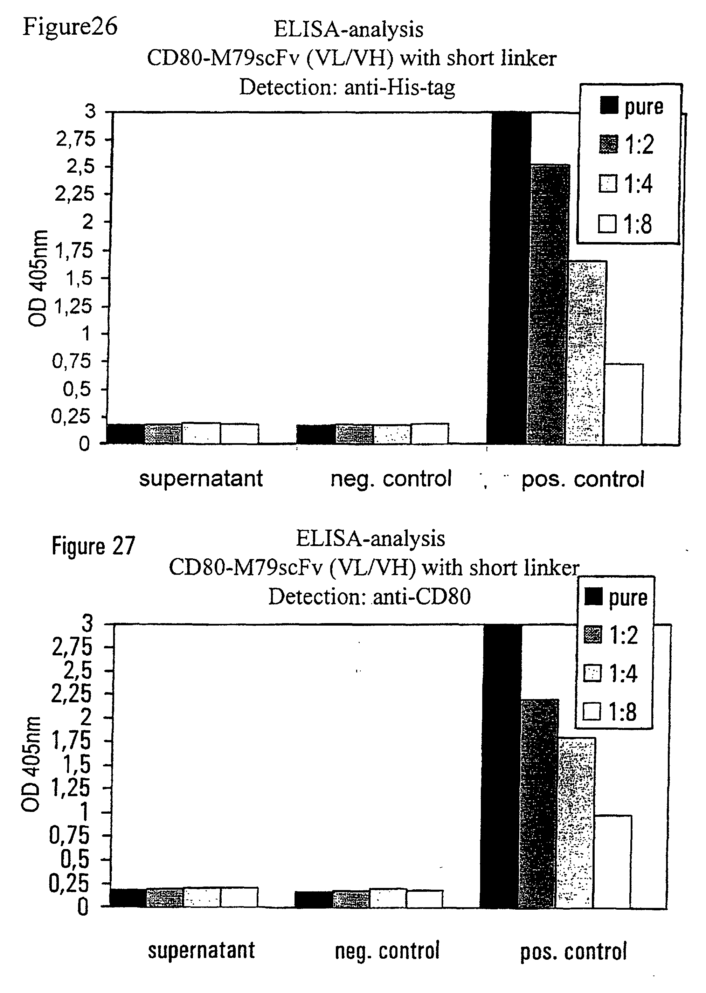

- Figure 26:: ELISA-analysis of the cell-culture supernatant obtained from CHO cells transfected with the expression plasmid pEF-DHFR+CTI+CD80-M79scFv(VL/VH) including the coding sequence of the short (Gly₄Ser₁)₁ linker. 96 well ELISA plates were incubated with 50µl of soluble 17-1A antigen (50µg/ml) per well. Subsequently pure cell-culture supernatant dilutions thereof were added as indicated. Detection was performed by a murine IgG1 anti His-tag antibody (dianova, Hamburg) diluted 1:1000 and a peroxidase conjugated polyclonal goat anti mouse IgG (Fc) antibody (dianova,Hamburg) diluted 1:5000. The anti-17-1A/anti-CD3 bispecific-single-chain antibody (Mack, Proc. Natl. .Acad. Sci. USA 92 (1995) 7021-7025) was used as positive control. As negative control, wells were incubated with phosphate buffered saline. The ELISA was developed by ABTS -substrate solution as described in example 2.1. The OD-values were measured at 405nm by an ELISA reader.
- Figure 27:: ELISA-analysis of the cell-culture supernatant obtained from CHO-cells transfected with the expression plasmid pEF-DHFR+CTI+CD80-M79scFv(VL/VH) including the coding sequence of the short (Gly₄Ser₁)₁ linker. 96 well ELISA plates were incubated with 50µl soluble 17-1A antigen (50µg/ml) per well. Subsequently pure cell-culture supernatant and dilutions thereof were added as indicated. Detection was performed by a murine IgG1-anti CD80 antibody diluted 1:1000 followed by a peroxidase conjugated polyclonal goat anti mouse IgG (Fc) antibody (dianova, Hamburg) diluted 1:5000. The anti-17-1A/anti-CD3 bispecific-single-chain antibody (Mack, Proc. Natl. Acad. Sci. USA 92 (1995) 7021-7025). was used as positive control and detected as described in Fig 26. As negative control, wells were incubated with phosphate buffered saline. The ELISA was developed by an ABTS -substrate solution as described in example 2.1. The OD-values were measured at 405nm by an ELISA reader.

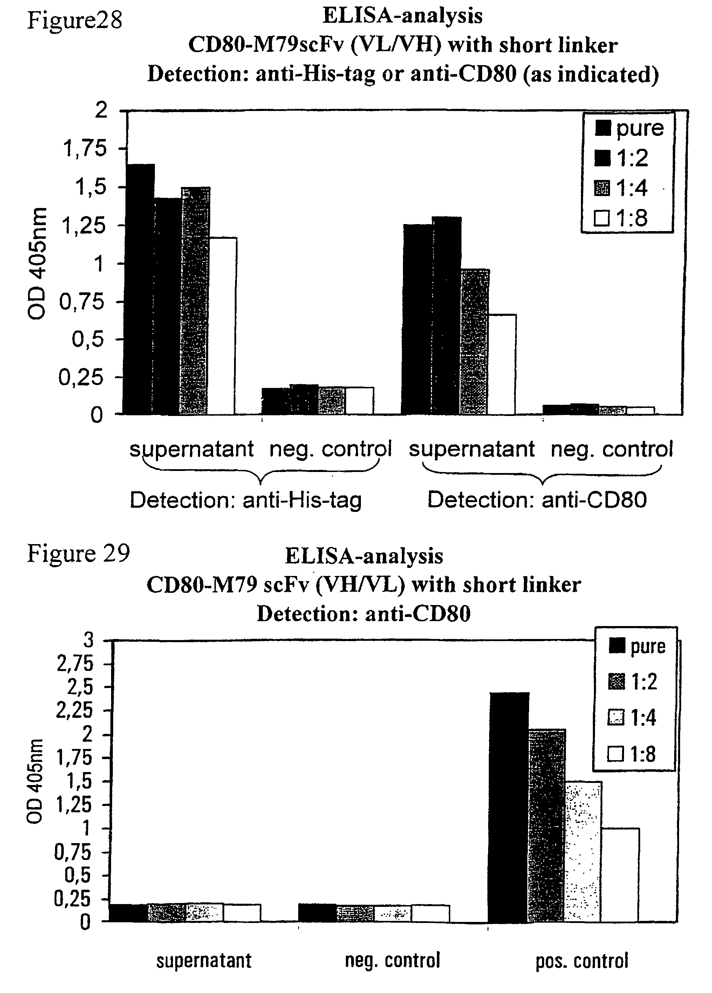

- Figure 28:: ELISA-analysis of the purified recombinant CD80-M79scFv(VL/VH)-construct with a short (Gly₄Ser₁)₁ linker obtained by purification from cell-culture supernatant using a Ni-NTA-column as described (Mack, Proc. Natl. Acad. Sci. USA 92 (1995) 7021-7025). 96 well ELISA plates were coated overnight at 4°C with pure eluate from the Ni-NTA-column and dilutions thereof as indicated. Subsequently bound recombinant protein was detected by a murine IgG1-anti CD80 antibody diluted 1:1000 or by a murine IgG1-anti His-tag antibody (dianova, Hamburg) diluted 1:1000 followed by a peroxidase conjugated polyclonal goat anti mouse IgG (Fc) antibody (dianova, Hamburg) respectively diluted 1:5000. As negative control wells were coated overnight at 4°C with 3% BSA in phosphate buffered saline. The ELISA was developed by an ABTS -substrate solution as described in example 2.1. The OD-values were measured at 405nm by an ELISA reader.
- Figure 29:: ELISA-analysis of the cell-culture supernatant obtained from CHO-cells transfected with the expression plasmid pEF-DHFR+CTI+CD80-M79scFv(VH/VL) including the coding sequence of the short (Gly₄Ser₁)₁ linker. 96 well ELISA plates were incubated with soluble 17-1A antigen (50µg/ml) per well. Subsequently pure cell-culture supernatant and dilutions: thereof were added as indicated. Detection was performed by a murine IgG1-anti CD80 antibody diluted 1:1000 followed by a peroxidase conjugated polyclonal goat anti-mouse IgG (Fc) antibody (dianova, Hamburg) diluted 1:5000. The anti-17-1A/anti-CD3 bispecific-single-chain antibody (Mack, Proc. Natl. Acad. Sci. USA 92 (1995) 7021-7025) was used as positive control and detected as described in Fig 26. As negative control wells were incubated with phosphate buffered saline. The ELISA was processed by an ABTS-substrate solution as described in example 2.1. The OD-values were measured at 405nm by an ELISA reader.

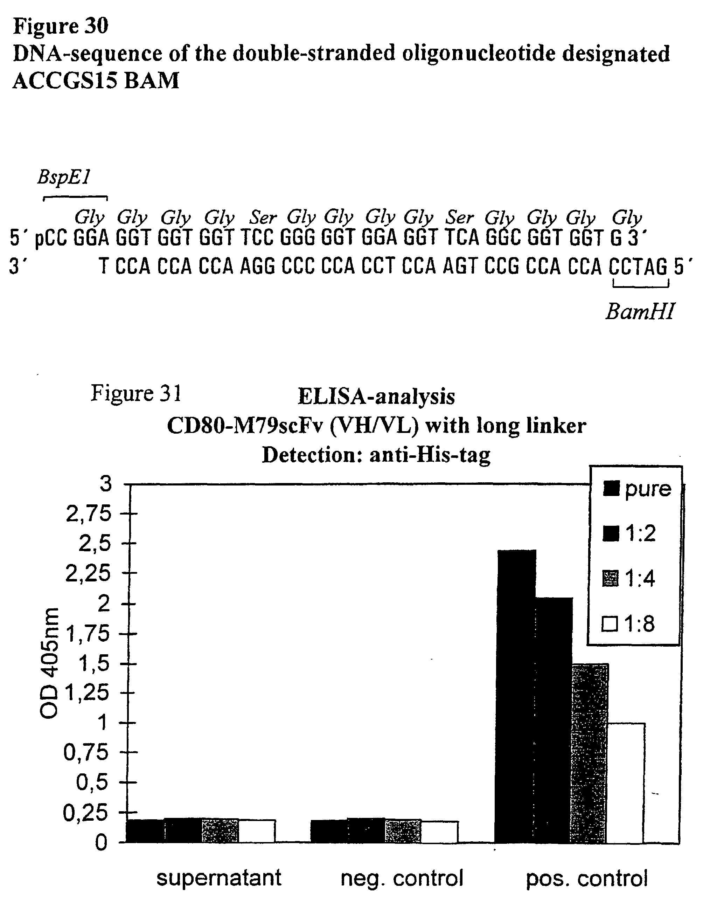

- Figure 30:: DNA-sequence of the double-stranded oligonucleotide designated ACCGS15BAM with single-stranded overhangs compatible with those of restriction enzymes BspEl and BamHl. Amino acids encoded by the nucleotide sequence are shown.
- Figure 31:: ELISA-analysis of the cell-culture supernatant and of its dilutions obtained from CHO-cells transfected with the expression plasmid pEF-DHFR+CTI+CD80-M79scFv (VH/VL) including the coding sequence of the long (Gly₄Ser₁)₃ linker. 96 well ELISA plates were incubated with 50µl soluble 17-1A antigen (50µg/ml) per well. Subsequently pure cell-culture supernatant and dilutions thereof were added as indicated. Bound protein was detected by a murine anti His-tag antibody (dianova, Hamburg) diluted 1:1000 followed by a peroxidase conjugated polyclonal goat anti mouse IgG (Fc) antibody (dianova, Hamburg) diluted 1:5000. The anti-17-1A/anti-CD3 bispecific-single-chain antibody (Mack, Proc. Natl. Acad. Sci. USA 92 (1995) 7021-7025) was used as positive control. As negative control wells were incubated with phosphate buffered saline. The ELISA was developed by an ABTS-substrate solution as described in example 2.1. The OD-values were measured at 405nm by an ELISA reader.

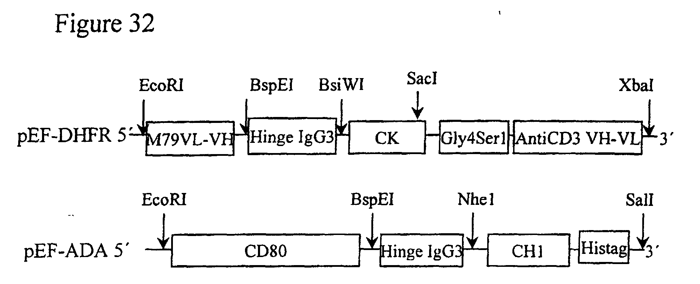

- Figure 32:: Molecular design of DNA-fragments encoding the single polypeptide chains of heterominibody M79scFv-CK-anti-CD3scFv/CD80CH1. Symbols are used as in figures 1(A) and 1(B), the expression vector pEF-DHFR or pEF-ADA used for cloning and expression of individual chains is indicated, Gly₄Ser₁ indicates a S-amino acid Glycin-Serin-Linker

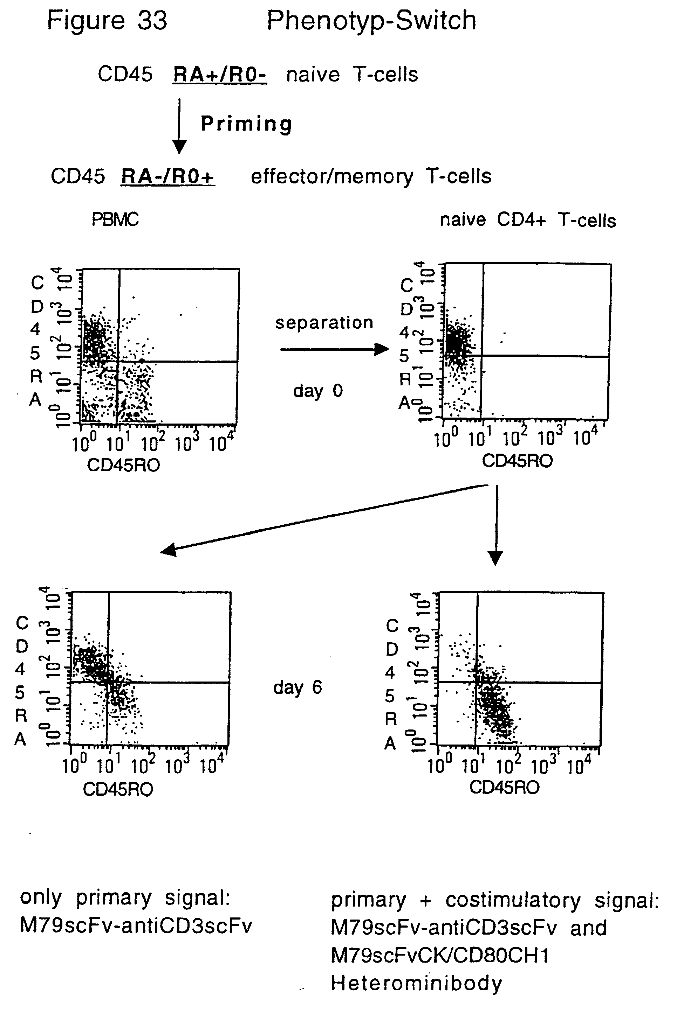

- Figure 33:: Phenotyp-switch of CD45 RA+/RO- CD4 T-cells to RA-/RO+ CD4 T-cells induced by in vitro priming. CD45RA and CD45RO expression on PBMCs and purified naive CD4+CD45RO- T-cells was analyzed by FACS on day 0. CD45RA and CD45RO expression levels of CD4+CD45RO-cells were further analyzed by flowcytometry after 6 days of stimulation with 500ng/ml heterominibody M79scFvCK/CD80CH1 and/or 250ng/ml of the bispecific single chain antibody M79scFv-anti CD3scFv in contact with irradiated 17-1A transfected CHO cells. 100.000 T-cells each were washed once with PBS and incubated for 30 minutes with a Phycoerythrin conjugated anti human CD45RA antibody (2H4 Coulter) diluted 1:50 and with a FITC conjugated anti human CD45RO antibody (UCHL-1 DAKO Hamburg) diluted 1:50 and washed again once with PBS prior to flowcytometry.

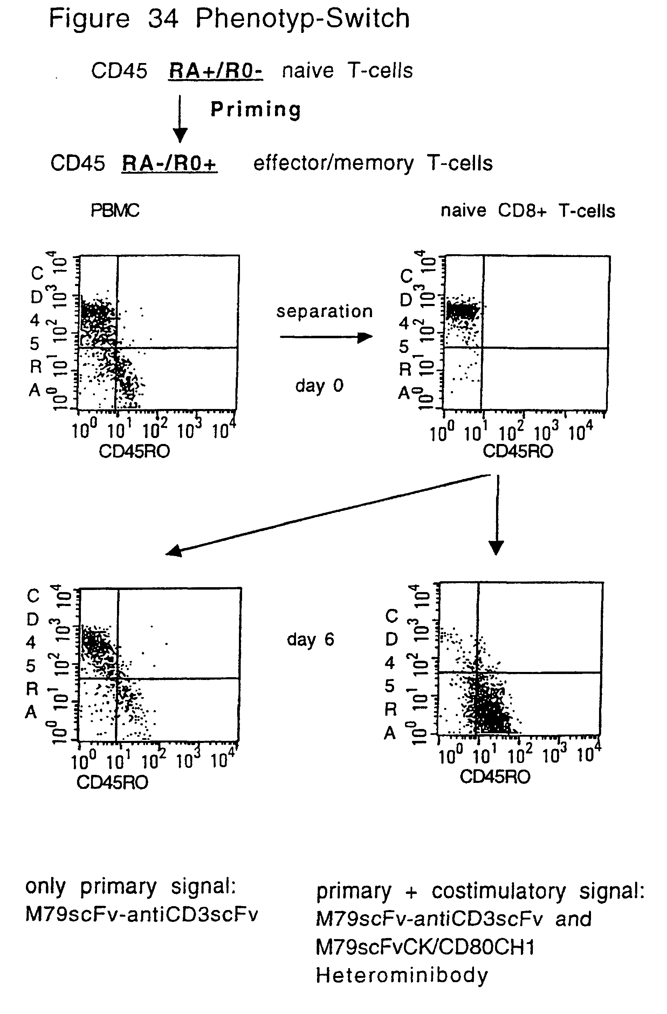

- Figure 34:: Phenotyp-switch of CD45 RA+/RO- CD8 T-cells to RA-/RO+ CD8 T-cells. CD45RA and CD45RO expression on PBMCs and purified naive CD8+CD45RO- T-cells was analyzed by FACS on day 0. CD45RA and CD45RO expression levels of CD4+CD45RO-cells were further analyzed by flowcytometry after 6 days of stimulation with 500ng/ml heterominibody M79scFvCK/CD80CH1 and/or 250ng/ml of the bispecific single chain antibody M79scFv-anti CD3scFv in contact with irradiated 17-1A transfected CHO cells. 100.000 T-cells each were washed once with PBS and incubated for 30 minutes with a Phycoerythrin conjugated anti human CD45RA antibody (2H4 Coulter) diluted 1:50 and with a FITC conjugated anti human CD45RO antibody (UCHL-1 DAKO Hamburg) diluted 1:50 and washed again once with PBS prior to flowcytometry.

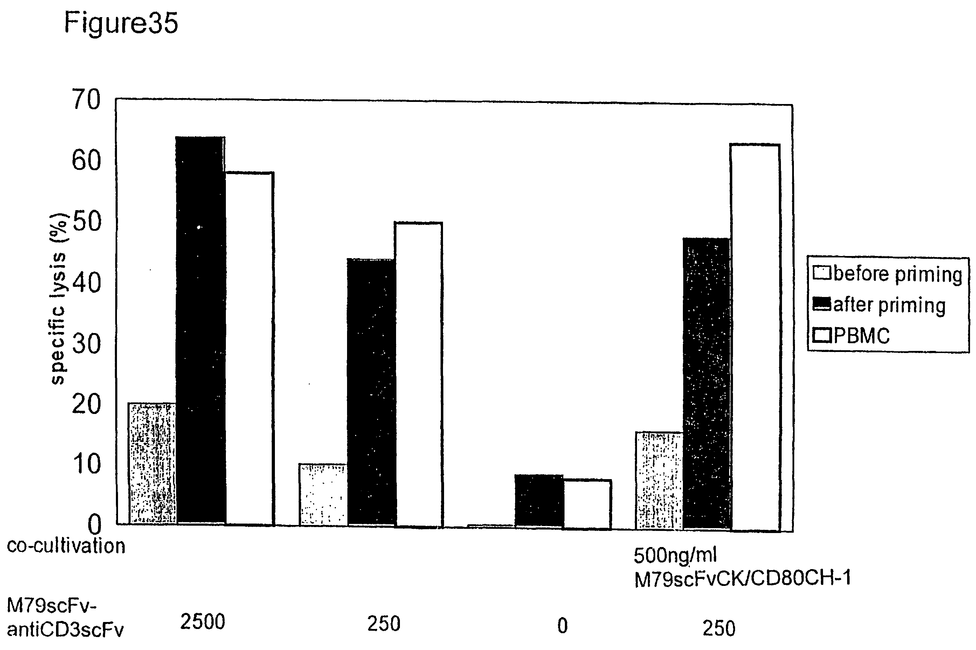

- Figure 35:: ⁵¹Cr release assay with naive CD8+ CD45RA+ CD45RO- T-cells (before priming) and T-cells derived therof by in vitro priming with heterominibody M79scFvCK/CD80CH1 and the bispecific single chain antibody M79scFv-anti CD3scFv (after priming). T-cells were redirected against 17-1A positive Kato cells by different concentrations of bispecific single chain antibody M79scFv-anti CD3scFv as indicated; optionally heterominibody M79scFvCK/CD80CH1 was present during the ⁵¹Cr release assay at a concentration of 500ng/ml. PBMC were used as positive control. Incubation time was 20h at an E:T-ratio of 20:1.

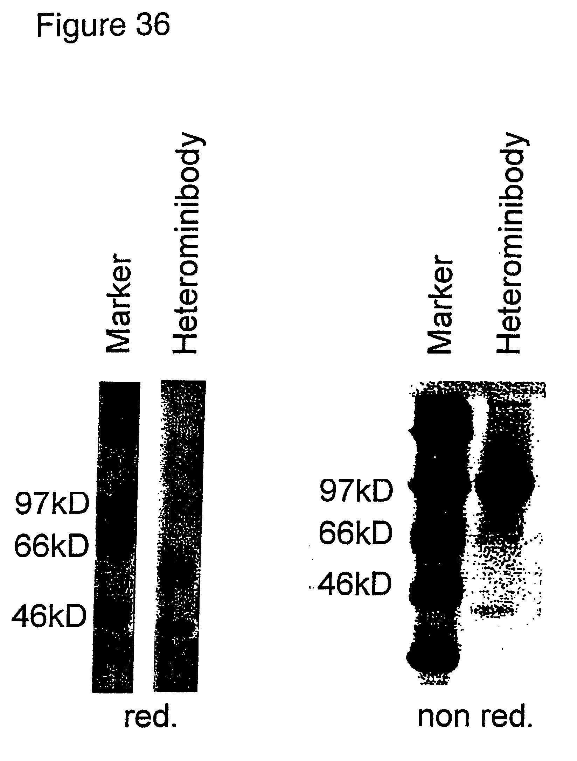

- Figure 36:: SDS PAGE (7,5%) of heterominibody M79scFvCK/CD80CH1. Coomassie staining of purified heterominibody M79scFvCK/CD80CH1 is shown under reducing (left panel) and non-reducing conditions (right panel). In both panels a molecular weight standard is shown.

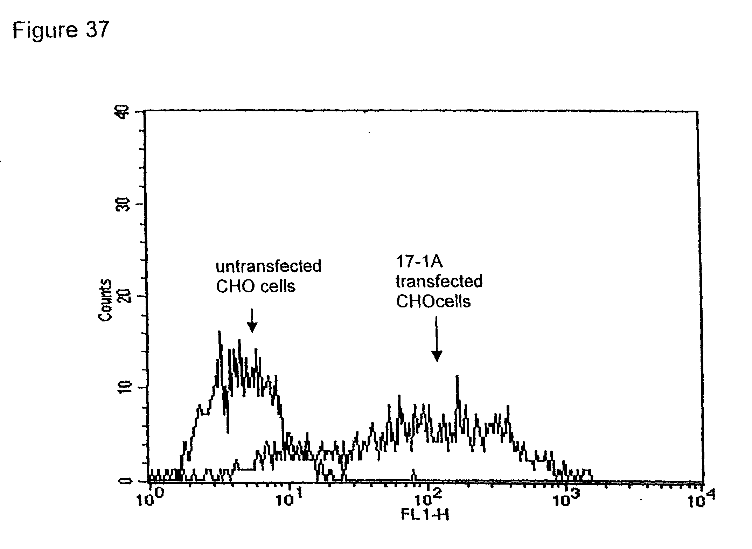

- Figure 37:: Binding of heterominibody M79scFvCK/CD80CH1 to untransfected and 17-1A transfected CHO cells. 200.000 cells of each cell-line were incubated for 30 minutes with purified heterominibody M79scFvCK/CD80CH1 at a concentration of 0.2µg/ml. Thereafter. cells were washed twice in PBS and incubated for 30 minutes with a FITC conjugated anti-human Cₖₐₚₚₐ specific antibody (Coulter 6604287) diluted 1:10. After two final washing steps in PBS, cells were then analyzed by flowcytometry (FACS-SCAN Becton Dickinson Immunocytometry Systems, San Jose CA, USA).

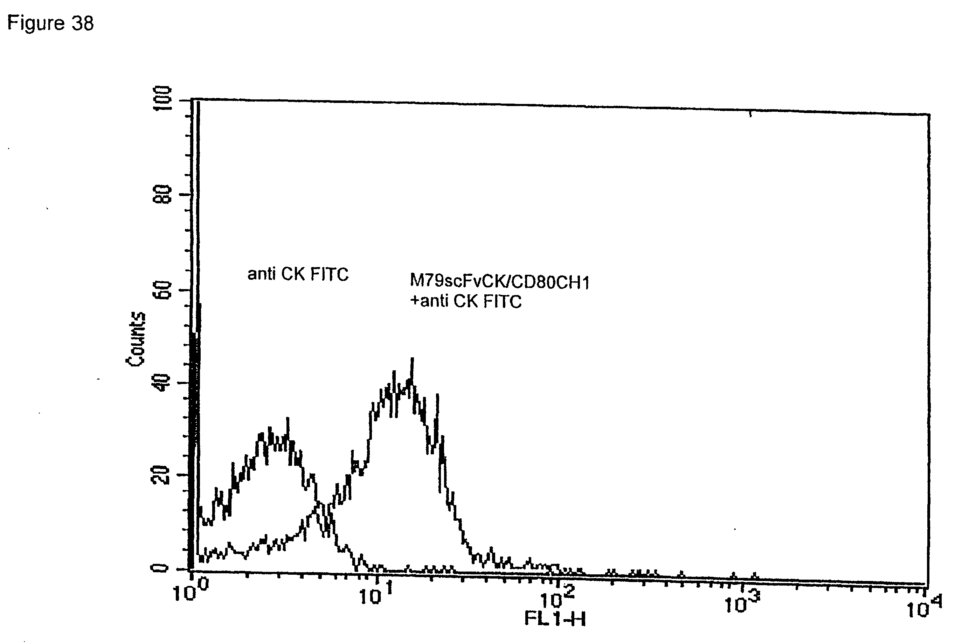

- Figure 38:: Binding of heterominibody M79scFvCK/CD80CH1 to CD28 on T cells. 100.000 purified naive CD8+CD11b-T-cells were incubated for 30 minutes with 200µg/ml heterominibody M79scFvCK/CD80CH1. Thereafter, cells were washed twice in PBS and incubated for 30 minutes with a FITC conjugated anti human Cₖₐₚₚₐ specific antibody (Coulter 6604287) diluted 1:10. After two final washing steps in PBS, cells were analyzed by flowcytometry.

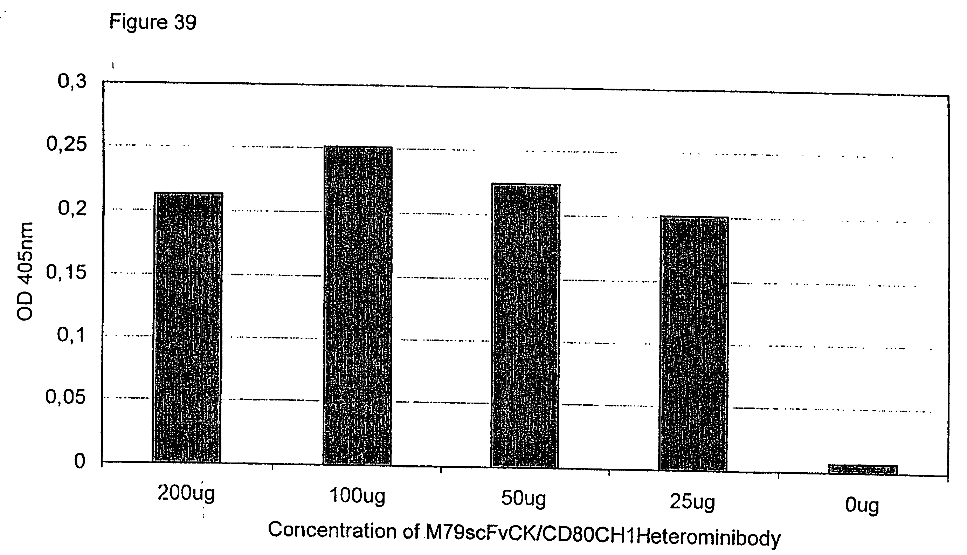

- Figure 39:: Binding of heterominibody M79scFvCK/CD80CH1 to CTLA-4 as measured by ELISA. 96 well ELISA plates were coated with 50µl/well of soluble CTLA-4/FcChimera (R&D Systems, Minneapolis MN USA, Cat No325-CT) at a concentration of 10µg/ml. Subsequently different concentrations of purified heterominibody M79scFvCK/CD80CH1 in phosphate buffered saline PBS were added. Detection was performed by a biotin-labeled anti-human Cₖₐₚₚₐ specific antibody (Pierce, Cat No 31780)) diluted 1:1000 followed by peroxidase conjugated Avidin (Dako, Hamburg, Cat No p0347) diluted 1:1000. As negative control, some wells were incubated with a CD4-lg fusion protein. The ELISA was developed by ABTS -substrate solution as described in example 2.1. The OD-values were measured at 405nm by an ELISA reader.

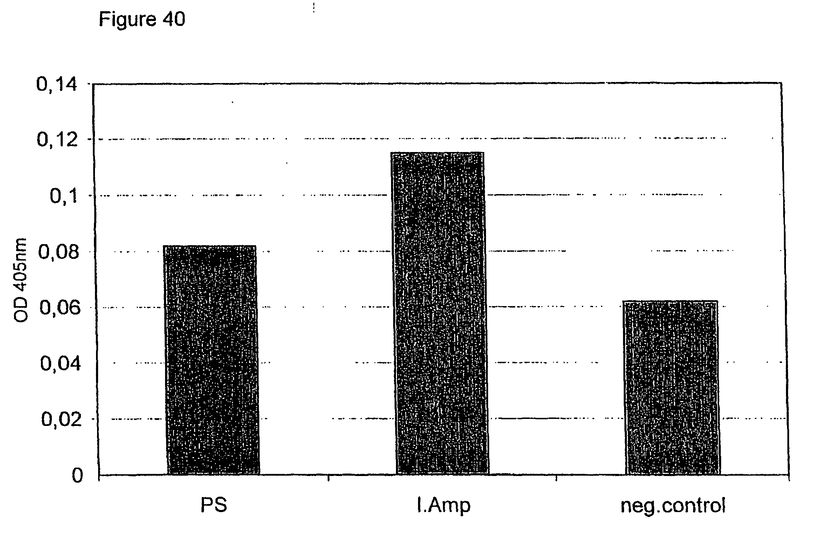

- Figure 40:: Binding of heterominibody M79scFvCK-antiCD3scFv/CD80CH1 to immobilized recombinant 17-1A antigen. 96 well ELISA plates were incubated with 50µl of soluble 17-1A antigen (50µg/ml). Subsequently, pure cell-culture supernatant of CHO cells transfected with heterominibody M79scFvCK-antiCD3scFv/CD80CH1 was added corresponding to primary selection (PS) or to first gene amplification step (1.Amp). Detection of bound heterominibody was performed by a CD80-specific monoclonal antibody (Immunotech, Cat No 1449) diluted 1:1000 and a peroxidase conjugated goat anti-mouse IgG (Fc)-antibody (Dianova, Hamburg) diluted 1:5000. As negative control, wells were incubated with phosphate buffered saline. The ELISA was developed by ABTS -substrate solution as described in example 2.1. The OD-values were measured at 405nm by an ELISA reader.

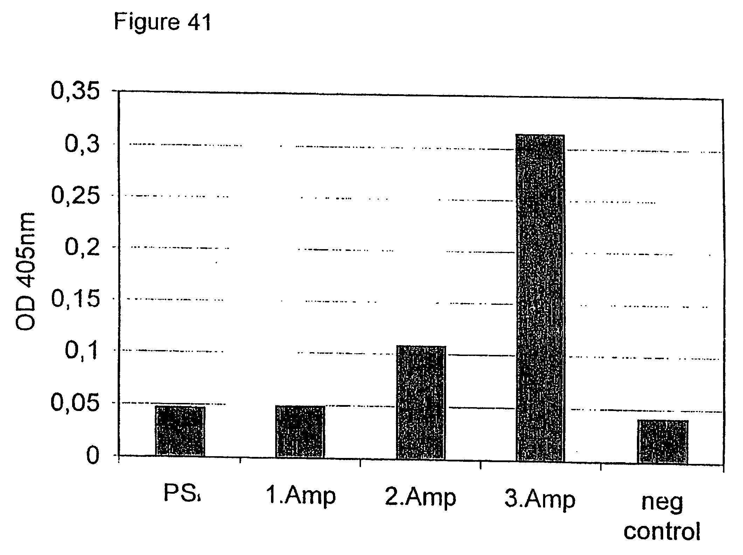

- Figure 41:: ELISA-analysis of cell-culture-supematants from CHO cells transfected with the expression plasmids pEF-DHFR-M79scFvCK-antiCD3scFv and pEF-ADA CD80CH1 corresponding to primary selection (PS) and different gene amplification steps (1.-3. Amp) as indicated. 96 well ELISA plates were incubated with 50µl/well of soluble 17-1A antigen (50µg/ml). Subsequently pure supematants containing heterominibody M79scFvCK-antiCD3scFv/CD80CH1 were added. Detection was performed by a peroxidase-labeled anti-histidine tag-antibody (Roche, 1965085) diluted 1:500. As negative control, wells were incubated with PBS. The ELISA was developed by ABTS-substrate solution as described in example 2.1. The OD-values were measured at 405nm by an ELISA reader.

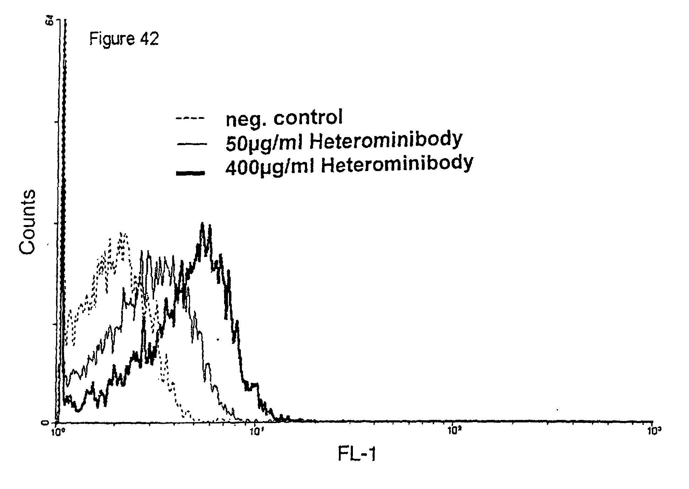

- Figure 42:: Binding of heterominibody M79scFvCK-antiCD3scFv/CD80CH1 to CD3 on human T-cells.
100.000 CD8+ CD3+CD11b+-T-cells were incubated for 30 minutes with 400µg/ml (thick black line) and 50µg/ml (thin grey line) of heterominibody M79scFvCK-antiCD3scFv/CD80CH1. Thereafter, cells were washed twice in PBS and incubated for 30 minutes with a FITC conjugated anti human Cₖₐₚₚₐ specific antibody (Coulter 6604287) diluted 1:10. As negative control cells were incubated with phosphate buffered saline (broken line) instead of heterominibody. After two final washing steps in PBS, cells were then analyzed by flowcytometry.

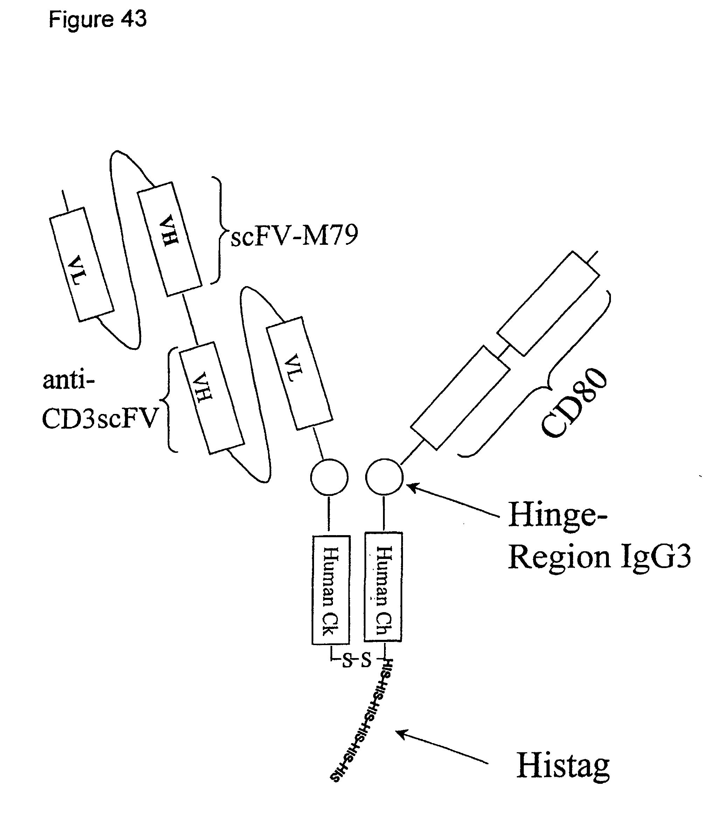

- Figure 43:: Molecular design of heterominibody bscAbM79scFv-antiCD3scFv-CK/CD80-CH1 shown on the protein level. CH1 and CK indicate the first constant domain of the human IgG1 heavy chain and the constant region of the human Ig-kappa light chain, respectively, that together form the heterodimerization unit covalently joined together by a disulfide bridge (S-S). VH indicates the Ig-heavy chain variable region and VL the Ig-light chain variable region.

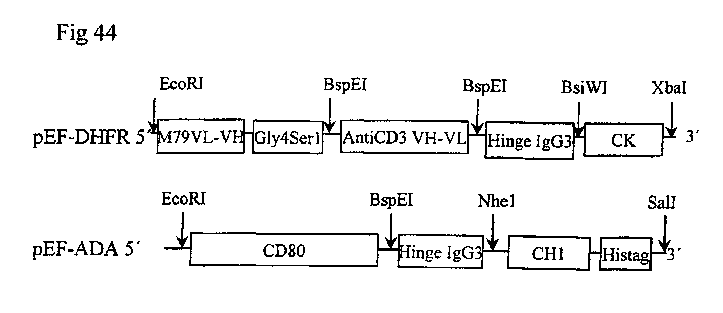

- Figure 44:: Design of both arms of heterominibody bscAb M79scFv-antiCD3scFv-CK/CD80-CH1 shown on the DNA-level; restriction enzyme cleavage sites are indicated. VH indicates the variable region of the Ig-heavy chain, VL that of the light chain.

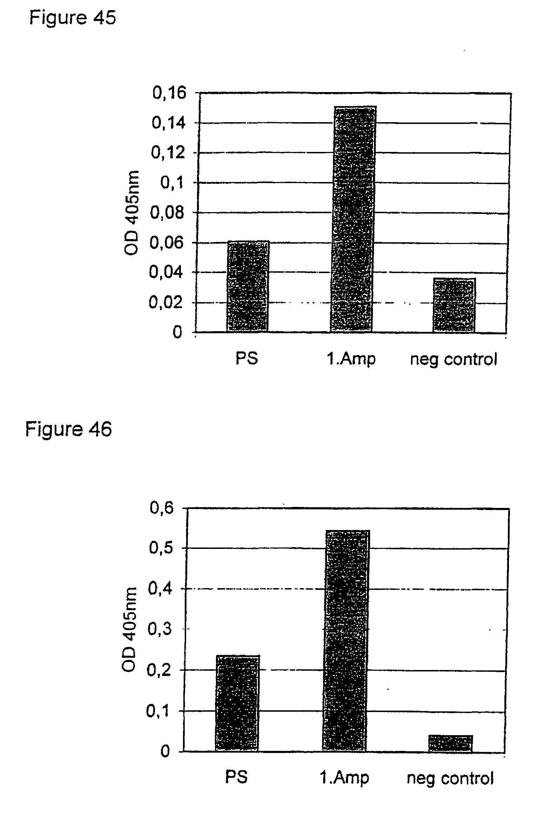

- Figure 45:: Binding of heterominibody bscAbM79scFv-antiCD3scFv-CK/CD80-CH1 to immobilized recombinant 17-1A antigen as measured by ELISA. 96 well ELISA plates were incubated with 50µl/well of soluble 17-1A antigen (50µg/ml). Subsequently pure cell-culture supernatants of CHO cells transfected with heterominibody bscAbM79scFv-antiCD3scFv-CK/CD80-CH1 were added corresponding to primary selection (PS) or to the first gene amplification step (1.Amp). Detection of bound heterominibody was performed by a peroxidase-labeled anti-histidine tag-antibody (Roche, 1965085) diluted 1:500. As negative control, wells were incubated with phosphate buffered saline instead of heterominibody. The ELISA was developed by ABTS -substrate solution as described in example 2.1. The OD-values were measured at 405nm by an ELISA reader.
- Figure 46:: ELISA-analysis of heterominibody bscAbM79scFv-antiCD3scFv-CK/CD80-CH1 carried out with an immobilized anti CD80-antibody. 96 well ELISA plates were incubated with 50µl/well of a monoclonal anti human CD80 antibody (Immunotech Cat No. 1449) diluted 1:200. Subsequently pure cell-culture supernatants of CHO cells transfected with heterominibody bscAbM79scFv-antiCD3scFv-CK/CD80-CH1 were added corresponding to primary selection (PS) or to the first amplification step (1. Amp). Detection was performed by a peroxidase-labeled anti-histidine tag-antibody (Roche, 1965085) diluted 1:500. As negative control, wells were incubated with phosphate buffered saline instead of heterominibody. The ELISA was developed by ABTS-substrate solution as described in example 2.1. The OD-values were measured at 405nm by an ELISA reader.

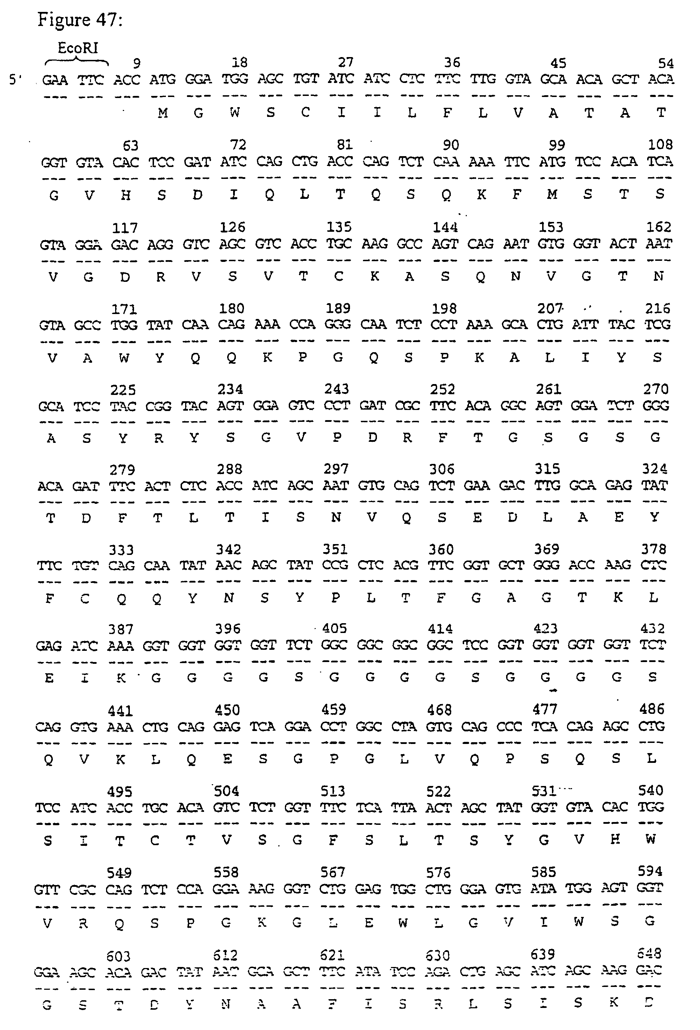

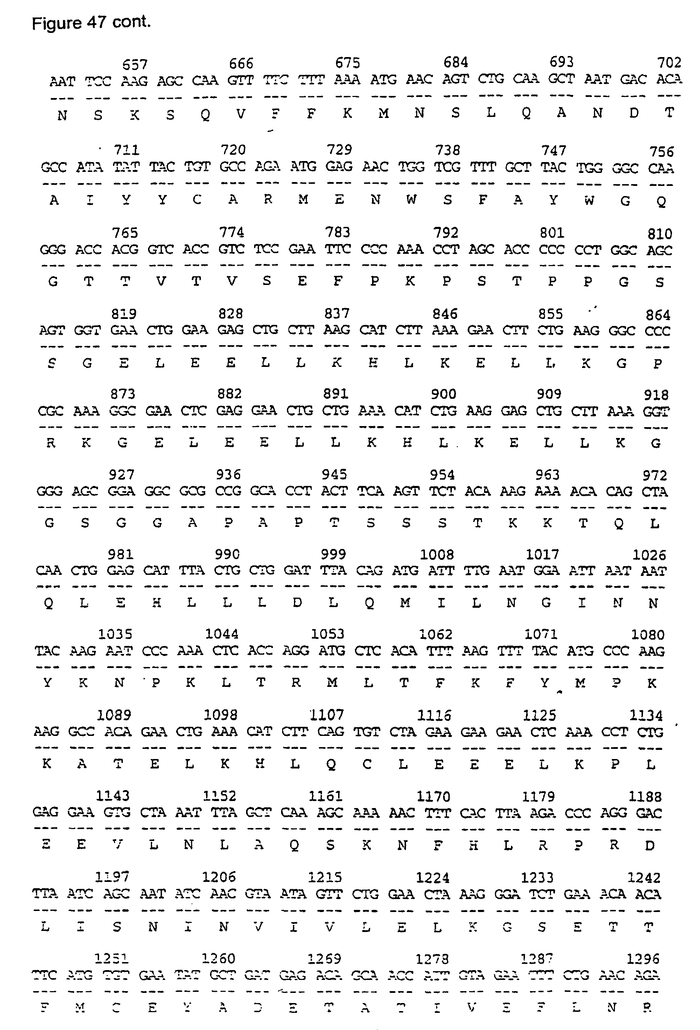

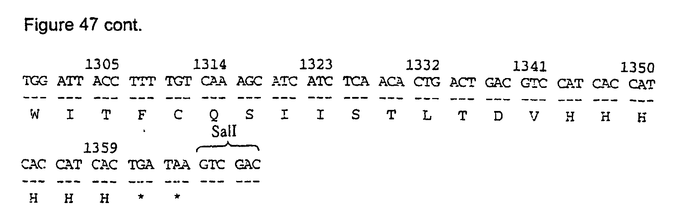

- Figure 47:: DNA- and amino acid- sequence of the M79scFv-IL2-construct containing a dimerization domain. Nucleotides 10 to 66 encode an N-terminal leader peptide. Nucleotides 67 to 387 encode the Ig-light chain variable region VL of M79scFv, nucleotides 388 to 432 encode a Glycin-Serin-linker followed by the Ig-heavy chain variable domain VH of M79scFv (nucleotide 433 to 777). At the 5'end of the murine IgG3 upper hinge region described by Pack (1993) Biotechnology 11:1271 (nucleotide 784 to 813) 6 nucleotides were added through the insertion of an EcoRl cleavage site. Nucleotides 814 to 918 encode the dHLX-dimerization domain described by Pack (1993) Biotechnology 11:1271 followed by a short peptide linker from nucleotide 919 to 936. The IL2-domain (nucleotides 937 to 1341) is followed by a C-terminal his-tag (nucleotides 1342 to 1359) and two stop codons. The restriction enzyme cleavage sites used for cloning are indicated.

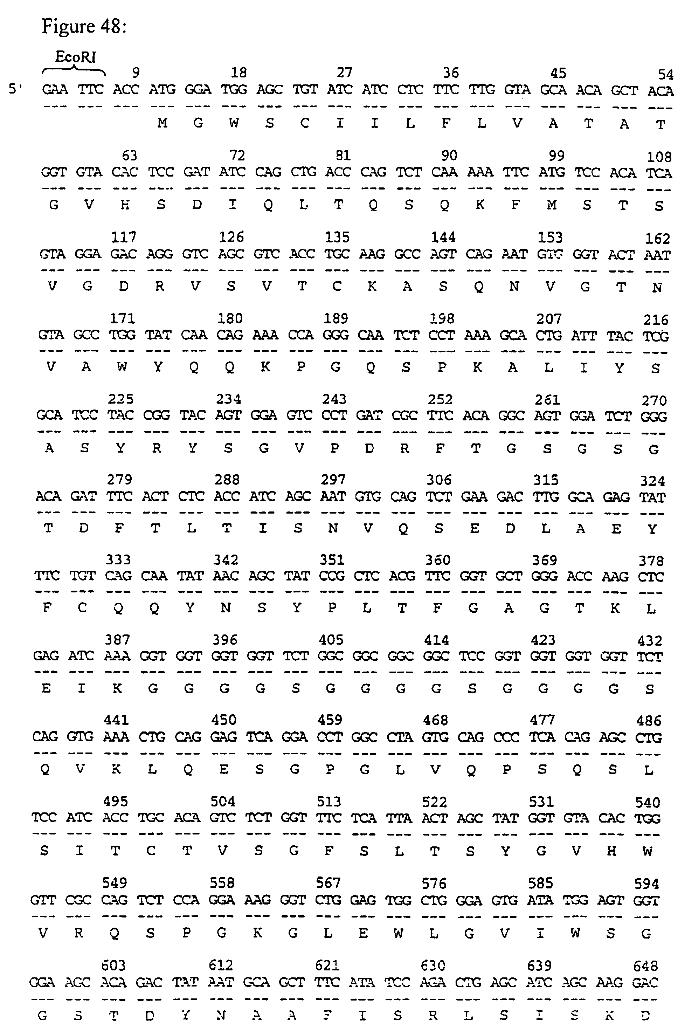

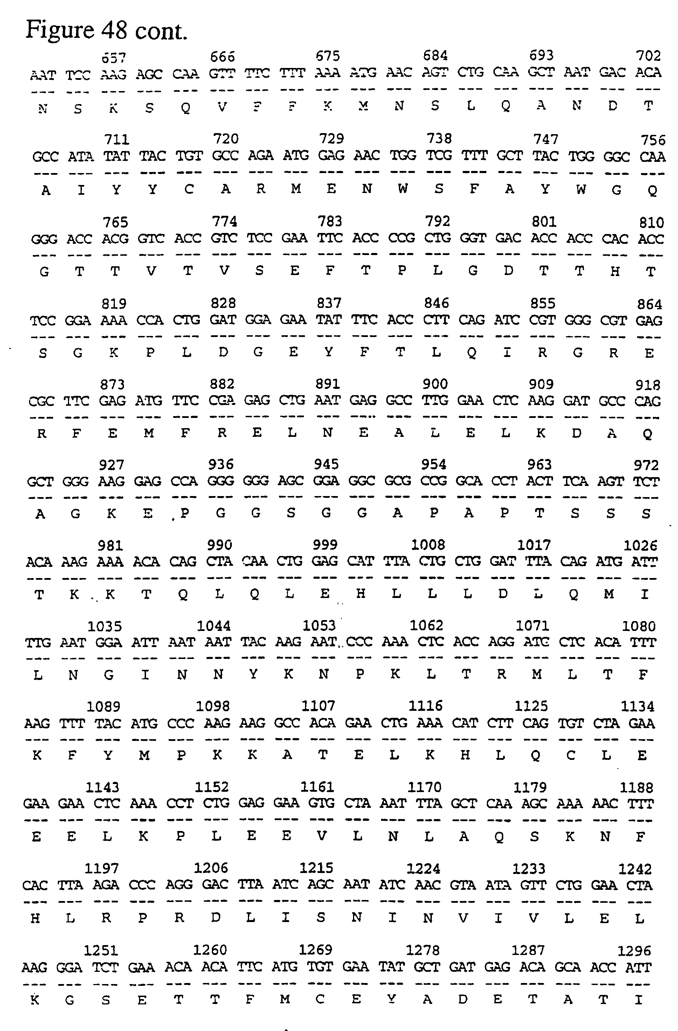

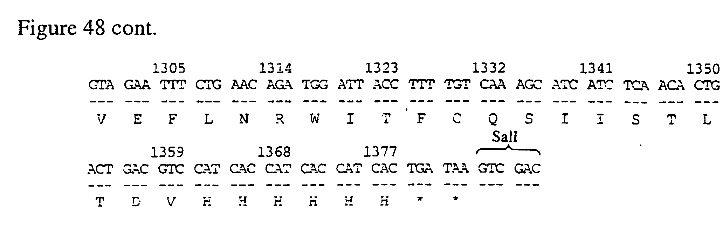

- Figure 48:: DNA- and amino acid- sequence of the M79scFv-IL2-construct containing a tetramerizations domain. Nucleotides 10 to 66 encode an N-terminal leader peptide. The nucleotides from 67 to 387 encode the Ig-light chain variable region VL of M79scFv, nucleotides from 388 to 432 encode a Glycin-Serin-linker followed by the Ig-heavy chain variable domain VH of M79scFv (nucleotide 433 to 777). At the 5'end of the human lgG3 upper hinge region described by Rheinnecker et al. J.Immunol. 157, (1996) 2989-2997 (nucleotide784 to 816) 6 nucleotides were added through the insertion of an EcoRI cleavage site. Nucleotides 817 to 933 encode the human p53 tetramerization domain (Rheinnecker et al. J.lmmunol. 157,(1996) 2989-2997) followed by a short peptide linker from nucleotides 934 to 954. The IL2-domain (nucleotides 955 to 1359) is followed by a C-terminal his-tag (nucleotides 1360 to 1377) and two stop codons. The restriction enzyme cleavage sites used for cloning are indicated.

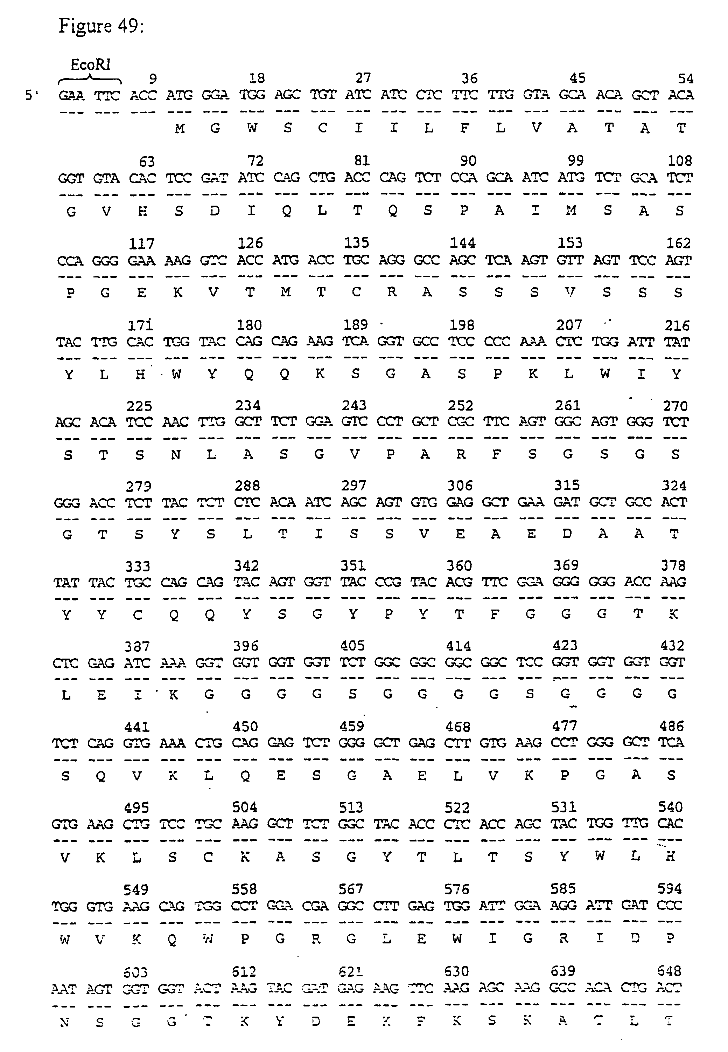

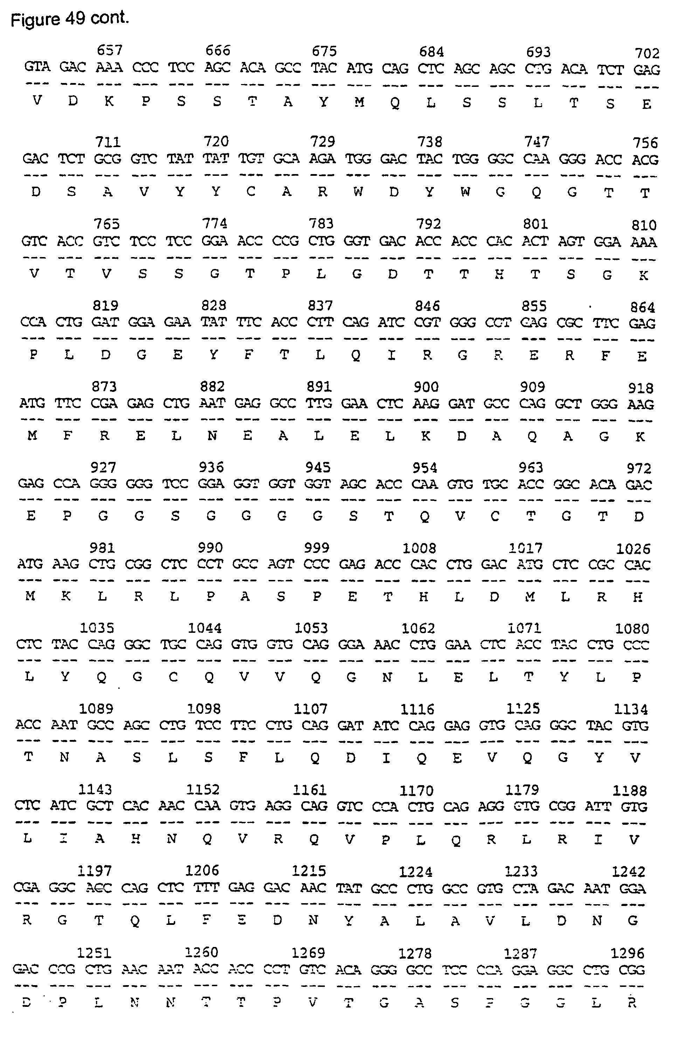

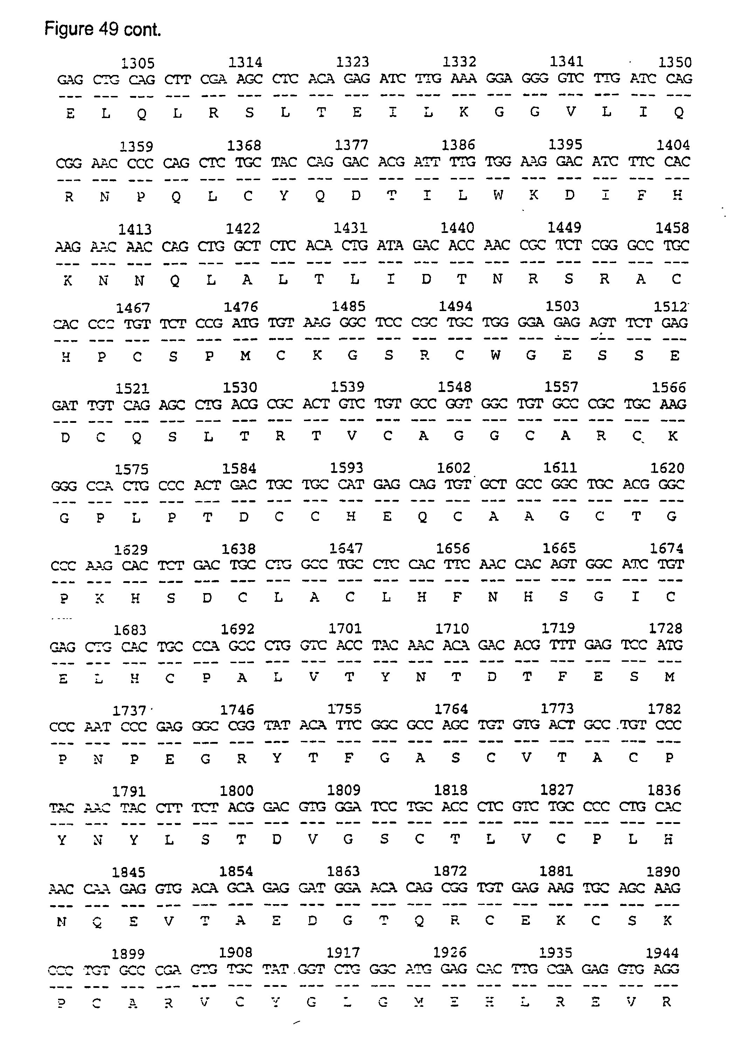

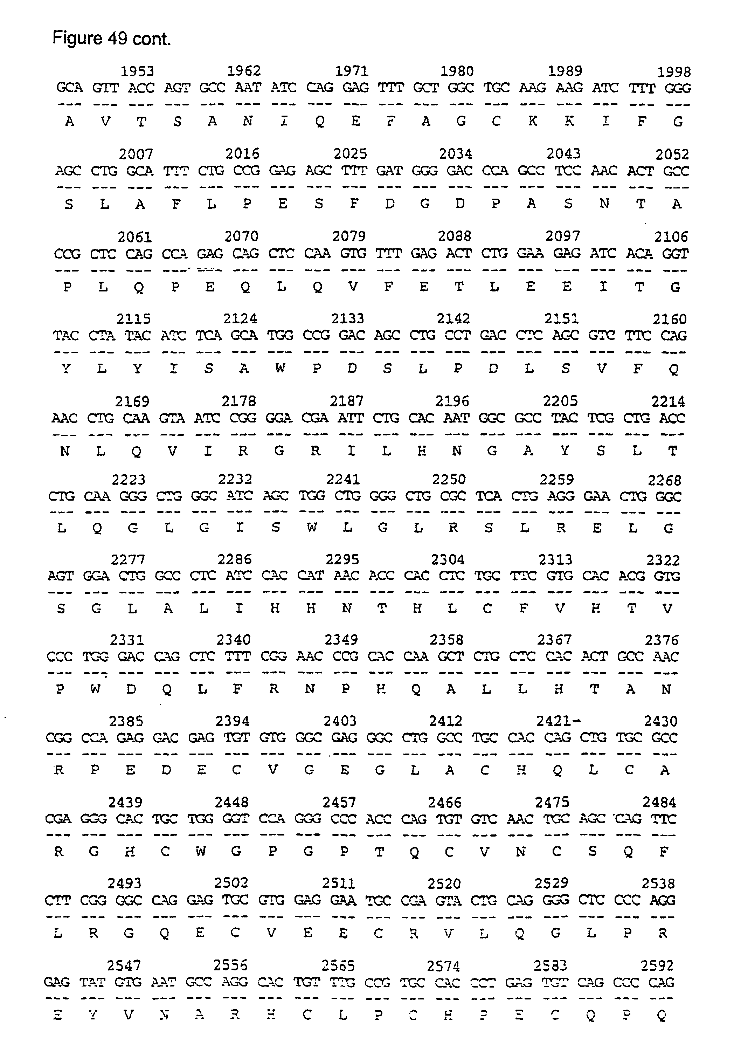

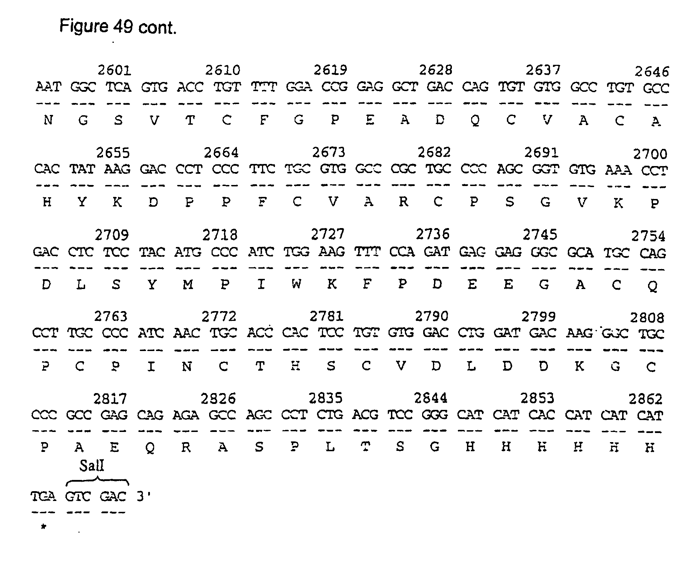

- Figure 49:: DNA- and amino acid- sequence of DC8scFv/erbB2-_{EC-}construct containing a tetramerization domain. Nucleotides from 10 to 66 encode an N-terminal leader peptide. Nucleotides 67 to 390 encode the Ig-light chain variable region VL of DC8scFv, nucleotides from 391 to 435 encode a Glycin-Serin-linker followed by the Ig-heavy chain variable domain VH of DC8scFv (nucleotide 436 to 771). At the 5'end of the human IgG3 upper hinge region described by Rheinnecker et al. J.lmmunol. 157, (1996) 2989-2997 (nucleotide775 to 807) 3 nucleotides were added thus completing with the following nucleotide triplet to form a BspEl cleavage site. Nucleotides 808 to 924 encode the human p53 tetramerization domain (Rheinnecker et al. J.Immunol. 157,(1996) 2989-2997), followed by a short peptide linker from nucleotides 925 to 945. The erbB2_{EC} domain ( nucleotides 946 to 2844) is followed by a C-terminal his-tag (nucleotides 2845-2862) and a stop codon. The restriction enzyme cleavage sites used for cloning are indicated.

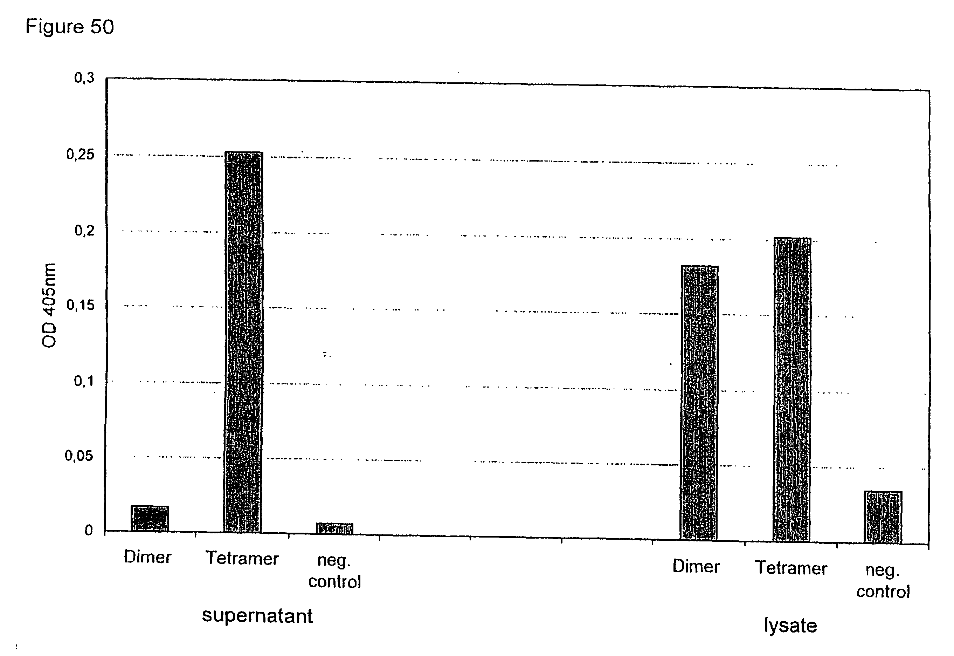

- Figure 50:: ELISA-analysis of cell-culture supernatant and lysate obtained from CHO cells transfected with the M79scFv-IL2-dimer-or tetramer construct. 96 well ELISA plates were coated with 50µl/well of soluble 17-1A antigen (50ug/ml). Subsequently pure cell-culture supernatant or the corresponding cell-lysate were added as indicated. Detection was performed with a peroxidase-conjugated anti-His-tag antibody (Roche, Cat No.1965085) diluted 1:500. As negative control, wells were incubated with phosphate buffered saline instead of M79scFv-IL-2-construct. The ELISA was developed by ABTS -substrate solution as described in example 2.1. The OD-values were measured at 405nm by an ELISA reader.

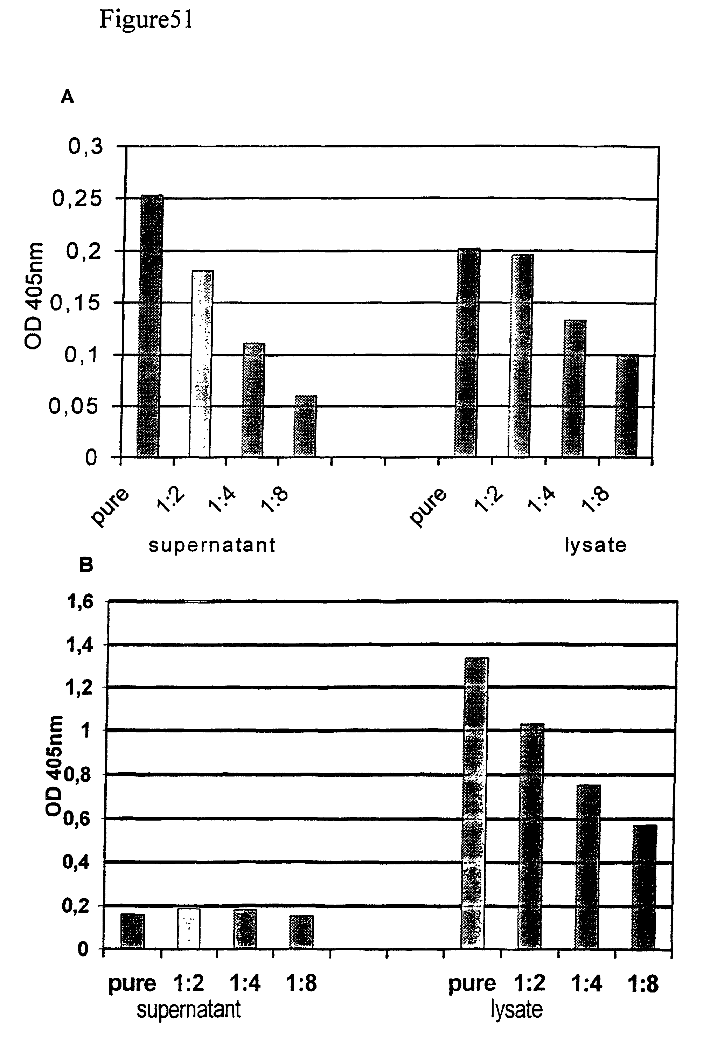

- Figure 51:: A: ELISA-analysis of cell-culture supernatant and lysate obtained from CHO cells transfected with the M79scFv-IL2-tetramer construct. 96 well ELISA plates were coated with 50µl/well of soluble 17-1A antigen (50µg/ml). Subsequently pure cell-culture supematants and the corresponding cell-lysate as well as the dilutions thereof were added as indicated. Detection was performed with a peroxidase-conjugated anti-His-tag antibody (Roche, Cat No .1965085) diluted 1:500. As negative control, wells were incubated with phosphate buffered saline instead of tetrameric M79scFv-IL2-construct. The ELISA was developed by ABTS -substrate solution as described in example 2.1. The OD-values were measured at 405nm by an ELISA reader.
- Figure 52:: Potential of the heterominibody format for addition of effector domains at its N- and C-terminal positions.
- Figure 53:: A heterominibody recognizing EpCAM with its N-terminal scFvs (HD70) and IL-2- and GM-CSF receptor-bearing cells with its C-terminally linked cytokines IL-2 and GM-CSF.

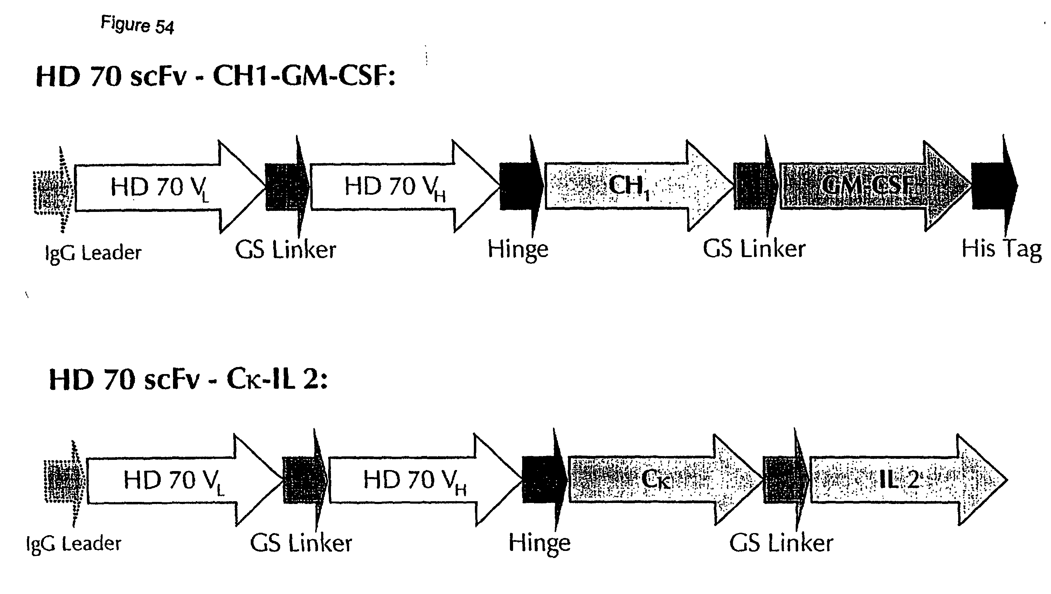

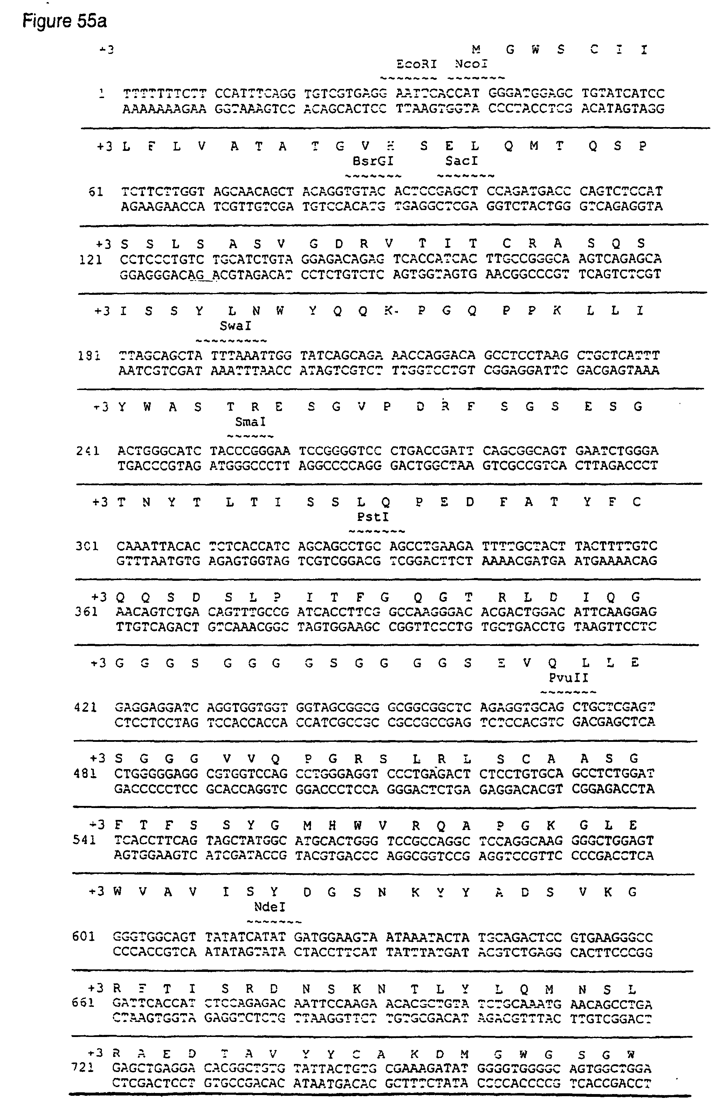

- Figure 54:: cDNA inserts of two vectors used to express the heterominibody schematically shown in Figure 53 in mammalian cells. Arrows show the various functional portions of inserts and point in 5'-3' orientation.
- Figure 55:: A. The complete nucleotide and deduced amino acid sequence encoding the HD70scFv-CH1-GM-CSF chain which is used to express the left half of the heterominibody schematically shown in figure 53 (upper part of the heterominibody schematically shown in figure 54).
B. The complete nucleotide and deduced amino acid sequence encoding the HD70scFv-Ck-IL-2 chain which is used to express the right part of the heterominibody schematically shown in figure 53 (lower part of the heterominibody scematically shown in figure 54).

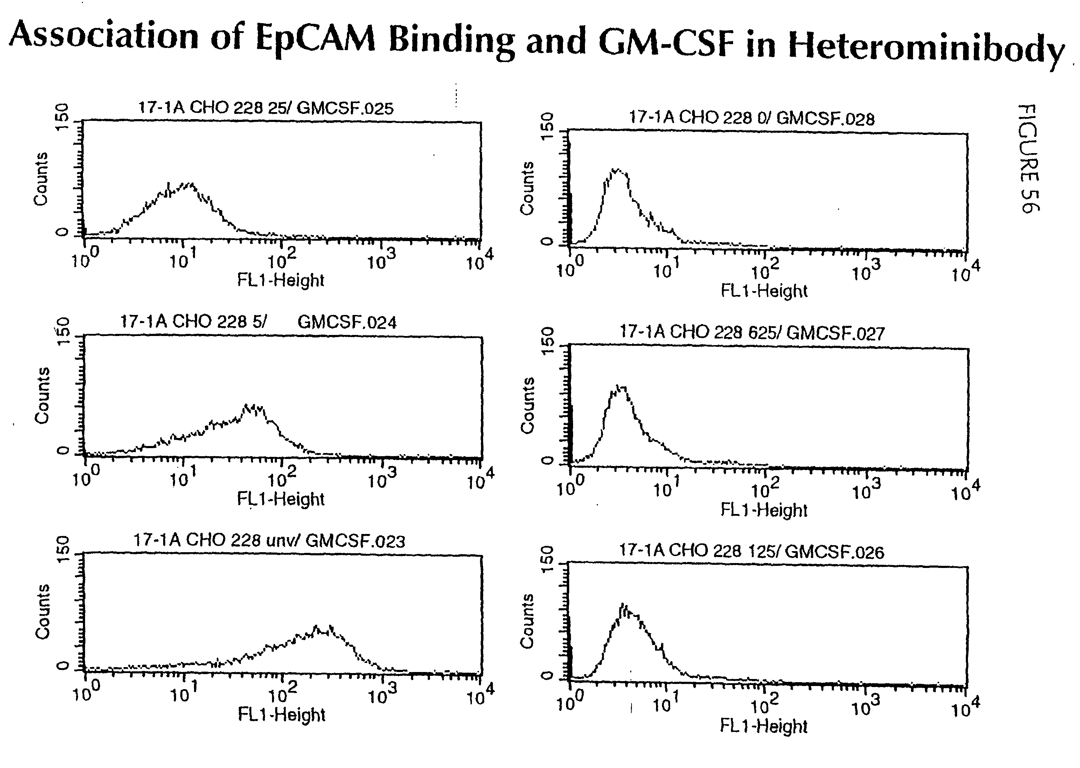

- Figure 56:: Binding of a heterminibody to CHO cells expressing human EpCAM. Expression vectors encoding the heterominibody shown in Figure 53 were stably transfected into CHO cells. Supematants from such cells were tested for production and secretion of heterominibody by FACS analysis (FACSCalibur, Beckton Dickinson) using CHO cells expressing on their surface human EpCAM. Bound heterominibody was detected by a second antibody binding to the GM-CSF portion of the particular heterominibody and a third species-specific labeled FITC-labeled antibody (see text). Upper panel: EpCAM-expressing CHO cells plus cell culture medium. Subsequent panels: Supernatants from heterominibody-expressing CHO cells were diluted in cell culture medium (in the order from top to bottom: 1:625, 1:125, 1:25, 1:5, and 1:1) and tested for increasing binding to EpCAM-positive cells.

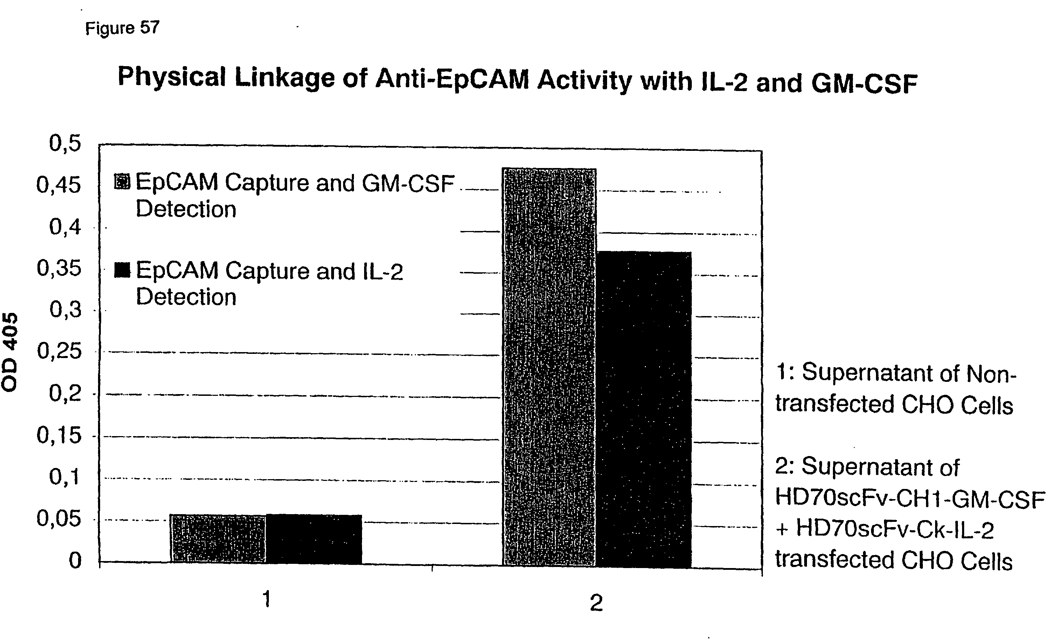

- Figure 57:: EpCAM binding activity is physically linked with GM-CSF and IL-2 immunoreactivities in supematants from heterominibody-producing CHO cells. The heterominibody shown in Figure 53 was expressed in CHO cells and resulting cell culture supernatants incubated with the extracellular domain of recombinant EpCAM immobilized to an ELISA plate. After extensive washes, bound heterominibody was analyzed for immunoreactivity with antibodies recognizing human GM-CSF or human IL-2 in an ELISA.

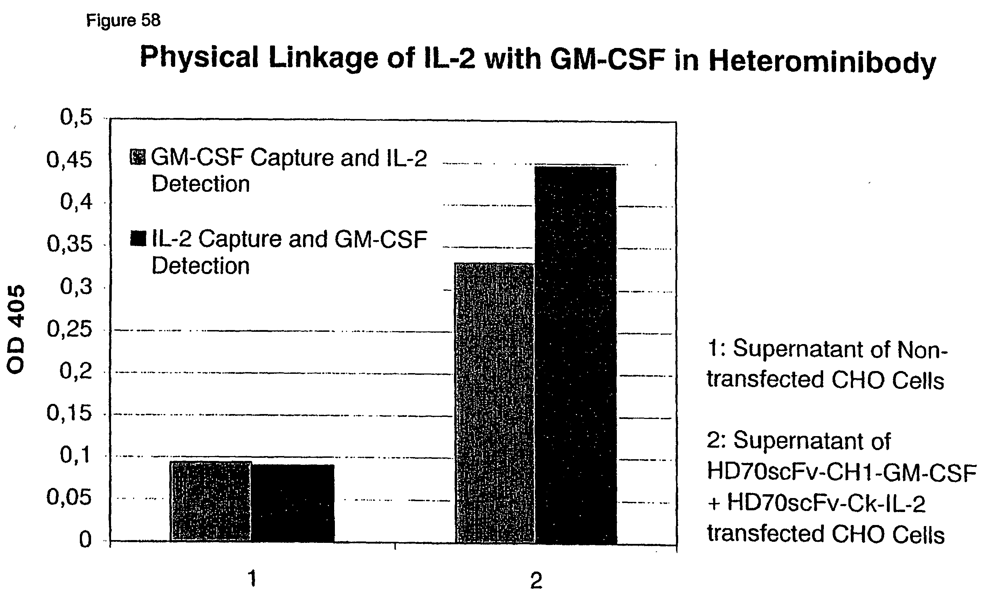

- Figure 58:: IL-2 and GM-CSF immunoreactivities are physically linked in supematants from heterominibody-producing CHO cells. The heterominibody shown in Figure 53 was expressed in CHO cells and resulting cell culture supematants incubated in an ELISA plate coated with either anti-hu-IL-2 or anti-hu-GM-CSF antibodies. Bound heterominibody was then tested for reactivity with an antibody recognizing the respective other cytokine (see examples).

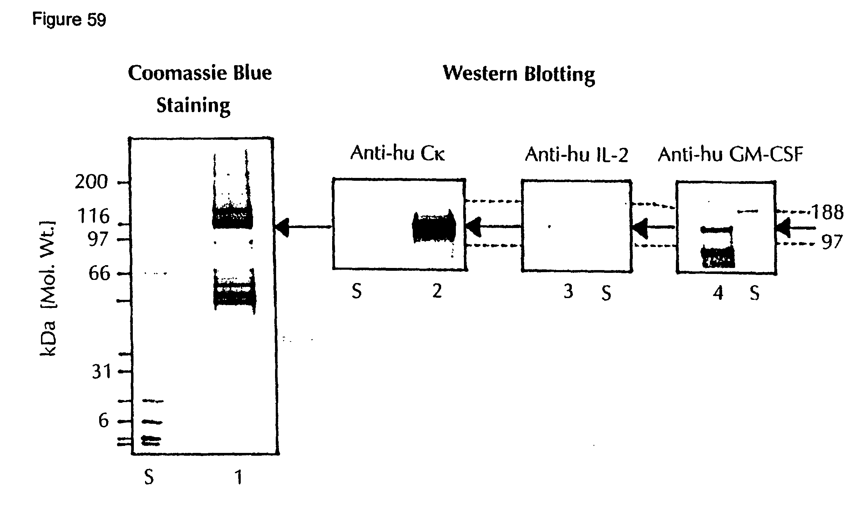

- Figure 59:: Purified heterominibody consists of covalently linked chains and immunoreacts with antibodies recognizing human C□, human IL-2 and human GM-CSF proteins in Western blots. The heterominibody shown in Figure 53 was expressed in CHO cells and purified in two steps from the cell culture supernatant. An aliquot of partially purified heterominibody was analyzed by gradient SDS-PAGE and the gel stained for proteins by Coomassie blue (lane 1). Aliquots of the supernatant were separated by SDS-PAGE followed by electroblotting onto membranes and immunostaining with antibodies recognizing human C□□(lane 2), human IL-2 (lane 3) and human GM-CSF (see examples) (lane 4). Alkaline phosphatase-conjugated streptavidin was used to visualize bound primary antibodies. The position of molecular weight standards are shown in lanes designated "S" and their sizes are given in numbers on the right and left panels. Arrows indicate the position of the 116-kDa heterominibody band. Dashed lines indicate the positions of 188 and 97 kDa markers.

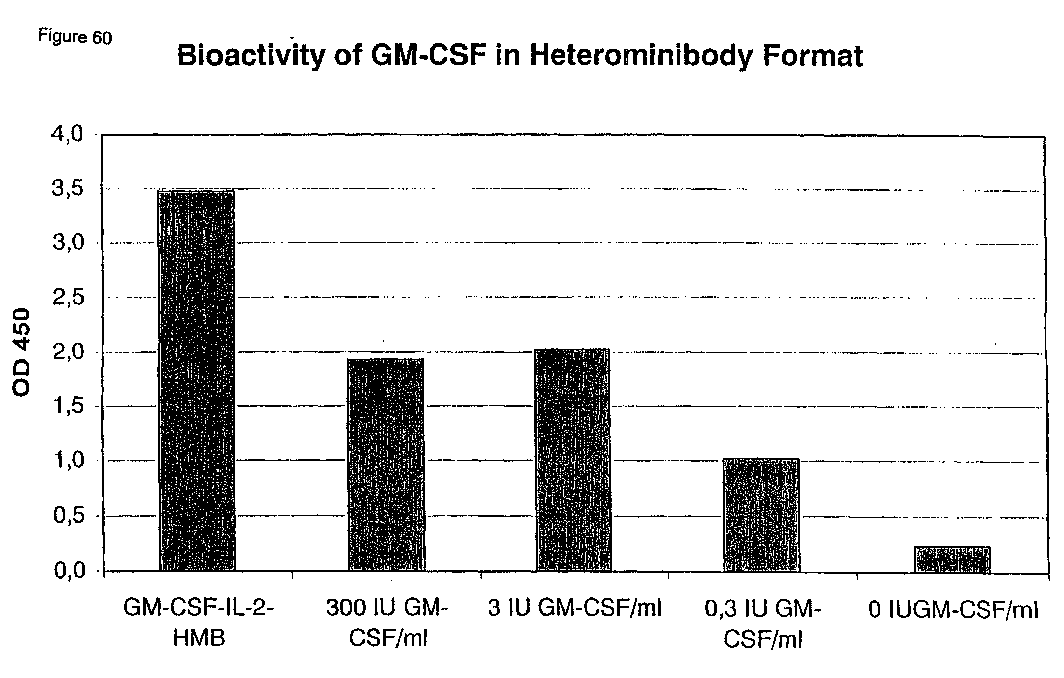

- Figure 60:: Bioactivity of heterominibody-bound human GM-CSF. The heterominibody shown in Figure 53 was produced in CHO cells, purified from cell culture supernatants and subsequently tested in a proliferation assay using the GM-CSF-responsive human erythroleukemia cell line TF-1. The heterominibody aliquot tested contained >300 IU/ml of GM-CSF as measured by titration against a human GM-CSF standard.

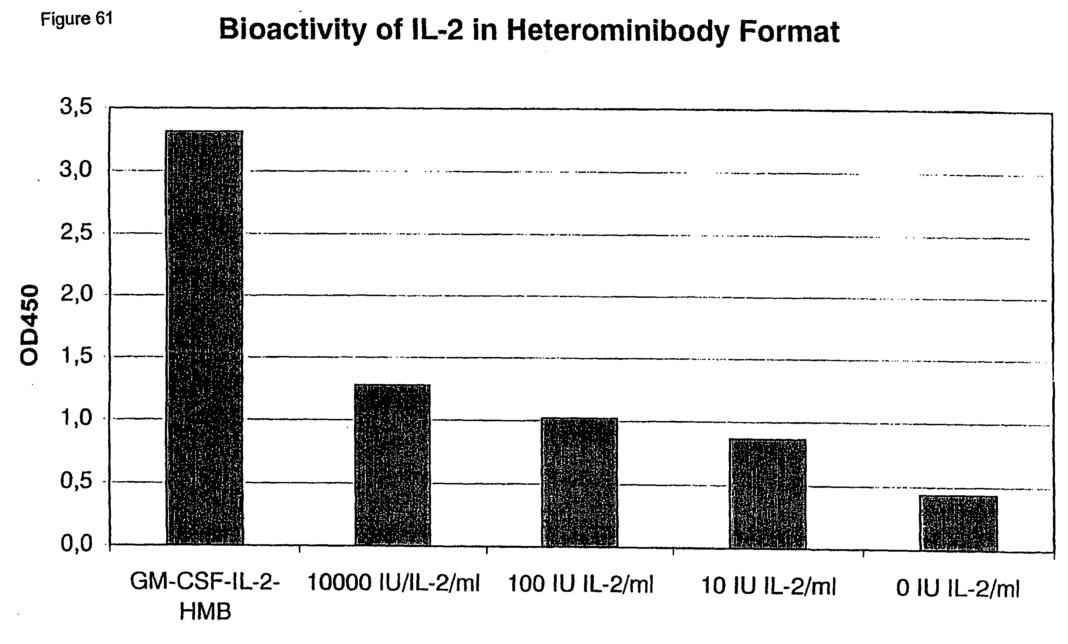

- Figure 61:: Bioactivity of heterominibody-bound human IL-2. The heterominibody shown in Figure 53 was produced in CHO cells, purified from cell culture supematants and subsequently tested in a proliferation assay using the human IL-2-responsive murine T cell line CL96. The heterominibody aliquot tested contained >10,000 IU/ml of human IL-2 as measured by titration against a human IL-2 standard.

The invention will now be described by reference to the following biological examples which are merely illustrative and are not to be construed as a limitation of scope of the present invention.

### Example 1

### Example 1.1 M79scFvCH1/CD80CK heterominibody

A protein was constructed that connects the single-chain Fv fragment (scFv) of the murine anti 17-1A antibody M79 (Göttlinger, Int. J. Cancer 38 (1986) 47-53) with the extracellular domains of human CD80 by virtue of the heterodimeric association of the immunoglobulin domains CH1 from the human γ1 heavy chain and Ck, the constant region of the human kappa light chain. For this purpose the M79scFv was connected to the human CH1 and the extracellular part of human CD80 was joined to human Ckappa, the resulting polypeptide encoding chains were inserted into separate expression vectors and both transfected into the same mammalian host cell line resulting in the CD80 heterominibody displayed in figure 1 In the following example the construction procedure is being described step by step.

### Example 1.1.1 Construction of the CD80-CK chain

First the CD80-Ckappa chain was assembled. The Ck DNA fragment was obtained by PCR using specific 5'and 3'primers. The cDNA template for this PCR was prepared by reverse transcription of the total RNA prepared from human peripheral blood mononuclear cells according to standard procedures. (Sambrook,Molecular Cloning; A Laboratory Manual, 2^{nd} Edition, Cold Spring Harbour Laboratory Press, cold Spring Habour, New York (1989)). The 5'HuCKBspE1 primer introduces the restriction cleavage sites BspE1 and BsiW1 as well as the hinge region of IgG3 (5'HuCKBspE1: 5'AAT TCC GGA ACC CCG CTG GGT GAC ACC ACC CAC ACC CGT ACG GTG GCT GCA CCA TCT GTC TTC 3') ,the 3'HuCKSalNOT primer introduces the cleavage sites Sal1 and Not1 (3'HuCKSalNOT: 5'ATA AGA ATG CGG CCG CGT CGA CTA ACA CTC TCC CCT GTT GAA GCT C-3'). The CD80 fragment was obtained by polymerase chain reaction (PCR) using specific oligonucleotide primers complementary to the 5' and 3' ends of the nucleotide sequence encoding the extracellular part of CD80 (Freeman G.J et.al. J.lmmunol.143,(1989) 2714 - 2722.). These primers also introduced an EcoRI and a BspEl cleavage site (5'CD80 Primer: 5'GCA GAA TTC ACC ATG GGC CAC ACA CGG AGG CAG 3'; 3'CD80 Primer: 5'TGG TCC GGA GTT ATC AGG AAA ATG CTC TTG CTT G 3') The cDNA template used for this PCR was prepared by reverse transcription of the total RNA prepared from the Burkitt-lymphoma cell line Raji according to standard procedures (Sambrook, Molecular Cloning; A Laboratory Manual, 2^{nd} Edition, Cold Spring Harbour Laboratory Press, Cold Spring Habour, New York (1989)).

The CD80 costimulatory protein belongs to the Ig superfamily. It is a heavily glycosylated protein of 262 amino acids. A more detailed description was published by Freeman G.J et.al. J.Immunol. 143,(1989) 2714 - 2722.

The CD80-Ckappa chain was cloned in several steps using the already existing vector pEF-DHFR-CTI-CD80-M79scFv. This vector was made as follows.
First a poly-linker designated CTI was inserted into the Bluescript KS vector (GenBank® accession number X52327) using the restriction enzyme cleavage sites Xbal and Sall (Boehringer Mannheim). The introduction of the polylinker CTI provided additional cleavage sites as well as the sequence encoding a (Gly₄Ser₁)₁ linker, a six-amino acid histidine tag and a stop codon as shown in figure 2 The vector BluescriptKS-CTI was prepared by cleavage with the restriction enzymes EcoRV and Xmal (Boehringer Mannheim and New England Biolabs) in order to ligate it (T4 DNA, Ligase Boehringer Mannheim) with the M79scFv fragment cleaved by EcoRV and BspEI (Mack et. al. Proc. Natl. Acad. Sci. 92 (1995)7021-7025) The resulting vector BluescriptKS-CTI-M79scFv again was cleaved with EcoRI (Boehringer Mannheim) and BspEl in order to insert the CD80 PCR-DNA-fragment obtained as described above and cleaved with the same enzymes. Subsequently, the whole CD80-M79scFv (VL/VH) DNA fragment was isolated by cleaving the vector BluescriptKS-CTI-CD80-M79scFv (VL/VH) with EcoRI and Sail (Boehringer Mannheim) and subsequently introduced into the eukaryontic expression vector pEF-DHFR described in Mack et.al., Proc. Natl. Acad. Sci. U.S.A. 92 (1995), 7021-7025. containing the dihydrofolatereductase gene as selection marker.
For the final step of constructing the CD80-Ckappa chain, the Ckappa fragment obtained as described above was cleaved with the restriction enzymes BspEl and Sall and cloned into the vector pEF-DHFR-CTI-M79scFv-CD80 using the same enzymes. Thereby the M79scFv fragment was replaced by Ckappa. The final plasmid pEF-DHFR-CTI-CD80-Ckappa shown in figure 3 was linearized with the restriction enzyme Ndel (Boehringer Mannheim) and transfected into CHO cells by electroporation. The electroporation conditions were 260V/960µF using a BioRad Gene Pulser™. Stable expression was performed in DHFR deficient CHO-cells as described by Kaufmann R.J. et.al. (1990) Methods Enzymol. 185, 537-566. The cells were grown for selection in nucleoside free α-MEM medium supplemented with 10% dialyzed FCS, 2 mM L-glutamine, 100U/ml Penicillin and 100ng/ml Streptomycin.

### Example 1.1.2 Construction of the M79scFv-CH1 chain

In the next step the M79scFv-CH1 chain was assembled. The CH1 fragment of the human IgG1 heavy chain was amplified by PCR from the same cDNA template used for PCR-amplification of the human Ckappa-domain. The 5' PCR-primer introduced two cleavage sites (BspE1 and Nhe1) as well as the upper Hinge region of human IgG3 (5'CH1huG1BspE1: AAT TCC GGA ACC CCG CTG GGT GAC ACC ACC CAC ACC GCT AGC ACC AAG GGC CCA TCG GTC TTC C). The 3' PCR primer introduced the cleavage sites BspE1 and Spe1 (3'CH1huG1BspE1: AAT TCC GGA ACT AGT TTT GTC ACA AGA TTT GG). The resulting PCR-fragment was prepared for cloning by cleavage with the restriction enzyme BspE1 and inserted into the above described BS-CTI vector which was cleaved with BspE1 and Xma1. In order to avoid vector self ligation, the vector was treated with alkaline phosphatase. Thereafter, the CH1 fragment was excised from BS-CTI with the restriction enzymes Sal1 and BspE1 in order to connect it with the M79 single chain Fv-fragment as described below:
The M79 antibody was described by Göttlinger et.al.(1986) Int.J.Cancer:38, 47-53. The M79 scFv fragment was obtained from the bispecific single-chain antibody (M79scFv-antiCD3scFv) described by Mack et. al. Proc. Natl. Acad. Sci. 92 (1995)7021-7025. The DNA-fragment encoding this bispecific single-chain antibody was excised from the expression vector pEF-DHFR and inserted into the expression vector pEF-ADA by using the restriction enzymes EcoRI and Sall respectively The expression vector pEF-ADA was derived from the expression vector pEF-DHFR (Mack et. al. Proc. Natl. Acad. Sci. U.S.A.92 (1995) 7021-7025) by replacing the cDNA encoding murine dihydrofolate reductase (DHFR) by that encoding murine deaminase.
In order to introduce the CH1 fragment and thereby replacing the anti-CD3scFv fragment, the vector pEF-ADA containing the bispecific single-chain antibody M79scFv-antiCD3scFv was cleaved using the same restriction enzymes as for preparation of the CH1 fragment (BspE1,Sal1). The resulting plasmid pEF-ADA-M79scFv-CH1 shown in figure 3 was linearized with Ndel and transfected into CHO cells already transfected with the expression the vector pEF-DHFR-CTI-CD80-Ck. The double transfected cells were grown for selection in nucleoside free α-MEN-medium supplemented with 10% dialyzed FCS and 2 mM L-glutamine, 100 U/ml Penicillin, 100ng/ml Streptomycin, 0,1µM deoxycoformycin (dCF) and 1x1.1-AAU-additive as described by Kaufmann-RJ (Meth.Enzym.185 (1990) 537-566). After cells were successfully grown under these conditions the concentration of dCF was increased to 0,3µM (ADA selection) and MTX was added to a final concentration of 20nM (DHFR selection) in order to obtain higher expression levels of the heterominbody due to gene amplification. ELISA-analysis of the culture supernatant of the transfected cell lines was carried out in order to determine the expression level of the heterominibody and to confirm its binding specificity for the 17-1A antigen (see example 4.4 and figures 9, 10)

### Example 1.2.1 M79scFvCK/CD80CH1 heterominibody

Another version of the CD80 heterominibody was constructed by replacing the M79scFv and the CD80 fragment by each other. Therefor the two expression plasmids pEF-DHFR-CTI-CD80-Ck and pEF-ADA M79scFv-CH1 were cleaved with EcoR1 and BspE1. The M79scFv fragment was then ligated with the pEF-DHFR-CTI-CK fragment and the CD80 fragment was ligated with the pEF-ADA-CH1-fragment. First, the vector pEF-DHFR-M79scFv-CK was transfected into CHO cells and grown for selection as described in example 1.1. The pEF-ADA-CD80-CH1 was transfected into the same CHO-cells in a second step and the resulting double transfected CHO cells were grown for selection as described above (see figure1 and 3).

### 1.2.2 Amplification, Subcloning and Purification of the M79scFvCK/CD80CH1 Heterominibody

Primary selection was carried out in nucleoside-free alpha MEM culture medium supplemented with 10% dialyzed FCS and 0,1µM deoxycoformycin (dCF) and 1x1.1-AAU-additive as described (Kaufman, Methods Enzymol. 185 (1990), 537-566). The expression of this construct was increased by gene amplification induced by stepwise increasing the concentrations of the DHFR-inhibitor methotrexate (MTX) and of the ADA-inhibitor deoxyformycin dCF.
The single amplification steps were carried out as follows:
1. Amplification 20nM MTX and 0,3µM dCF,
2. Amplification 100µM MTX and 1µM dCF
3. Amplification 500nM MTX and 3µM dCF. The cells obtained from the third amplification step were cloned by limiting dilution. Therefor the cells were seeded at a concentration of 50 cells per ml, 10 cells per ml and 2 cells per ml into 96 well flat-bottom tissue culture plates according to Current Protocols in Immunology (Coligan, Kruisbeek, Margulies, Shevach and Strober, Wiley-Interscience, 1992) under the culture conditions of the third amplification step. Positive clones from wells with single tight cluster of cells as an evidence for monoclonal growth were identified by ELISA as described in Example 2.1.

One positive clone was expanded and taken for protein production. Large scale antibody production was carried out in rollerbottles using 500 ml medium.
The M79scFvCK/CD80CH1 heterominibody was purified via its C-terminal histidine tail as described. ( Mack et. al. Proc. Natl. Acad. Sci. U.S.A.92 (1995), 7021-7025).

### Example 1.2.3 ELISA on cell-culture supernatant of CD80 Heterominibodies

To analyze the 17-1A binding properties of both CD80 heterominibody versions and to confirm the proper association of CH1 and CK two different ELISAs were carried out. Specific binding to the 17-1A-antigen was shown by incubation of culture supernatant on immobilized recombinant 17-1A-antigen and detection of bound CD80-Heterominibodies by an anti-CD80-antibody. The heterodimeric structure of the Heterominibodies was confirmed by incubation of the culture supernatant on immobilized anti-His-tag-antibody followed by a detection step with an anti-human Cₖₐₚₚₐ-antibody. For details about both ELISAs see example 4.4.,figure 9 and 10.

### Example 2: Analysis of Heterominibodies M79scFvCK/CD80CH1 and M79scFvCH1/CD80CK

### 2.1 ELISA-analysis of heterominibody M79scFvCK/CD80CH1 expressed by transfected CHO-cell lines at different steps of gene amplification

The culture supematants corresponding to primary selection, first and second amplification and third amplification plus subsequent cell-cloning were tested by ELISA. For this purpose recombinant 17-1A (Mack, Proc. Natl. Acad. Sci. USA 92 (1995) 7021-7025) was coated to 96 well U-bottom ELISA plates (Nunc maxisorb) (50µg/ml, 50µl/well) in phosphate buffered saline (PBS). Coating was performed overnight at 4°C, blocking was performed with 3% bovine serum albumin (BSA) in PBS for one hour at room temperature. Antibody constructs as culture supernatants from primary selection (PS) and from different amplification steps (1.Amp, 2.Amp, 3.Amp subcloned) (figure 4), respectively, were added and incubated for one hour at room temperature. Bound heterominibody was detected by a biotin labeled anti-human CK antibody (Pierce, Cat No.31780) diluted 1:1000 in PBS 1%BSA. After three times of washing with PBS 0,05% Tween20, Avidin Peroxidase (DAKO, Hamburg, Cat.no P0347) diluted 1:1000 was added and incubated at room temperature for one hour. After four times of washing with PBS 0,05% Tween20, the ELISA was finally developed by adding the following substrate solution: 22 mg ABTS (2,2 Azino-bis (3-Ethylbenzthiazoline-6 Sulfonic Acid) Diammonium salt) was dissolved in 10 ml 0,1M citrat buffer pH 5,1 containing 2,3mg Sodium perborate Tetrahydrate. For negative controls, the plates were incubated with PBS instead of bifunctional antibody constructs. The colored precipitate was measured at 405 nm using an ELISA-reader. As shown in figure 4, heterominibody expression continuously increased from primary selection to a cell clone from third amplification step.

### 2.2 ELISA-analysis of Heterominibodies M79scFvCK/CD80CH1 and M79scFvCH1/CD80CK by different combinations of immobilized antibodies or immobilized 17-1A-antigen with appropriate detection antibodies

### 2.2.1.ELlSA-analysis with immobilized 17-1A-antigen and an anti-CD80 detection antibody

The culture supernatants derived from the 1.Amplification step were tested. Recombinant 17-1A-antigen was coated to the ELISA plate. Bound antibody constructs were detected by a CD80-specific monoclonal antibody (Immunotech, Cat. No. 1449) diluted 1: 1000 in PBS 1% BSA followed by a polyclonal peroxidase-conjugated goat anti-mouse IgG-antibody (Fc-specific) diluted 1: 1000 in PBS 1% BSA. The ELISA procedure was performed as described above. As shown in figure 9 both heterominibody versions proved to bind to the 17-1A antigen although the M79scFvCK/CD80CH1-version showed a substantially higher expression level than the M79scFvCH1/CD80CK-version.

### 2.2.2 ELISA -analysis with immobilized anti-His-tag-antibody and an anti-human Cₖₐₚₚₐ detection antibody

The culture supernatants derived from the 1.Amplification step were tested. BSA-free anti-histidine tag-antibody (Dianova, Hamburg, Cat No DIA910) was coated to the ELISA plate. Bound antibody constructs were detected by a biotin labeled anti-human Cₖₐₚₚₐ antibody (Pierce, Cat No.31780) diluted 1:1000 in PBS followed by Avidin Peroxidase (DAKO, Hamburg, Cat. No P0347) diluted 1:1000 1%BSA. The ELISA procedure was performed as described above. As shown in figure 10 the results of this ELISA confirm the higher expression level of heterominibody M79scFvCK/CD80CH1 and together with the results of the foregoing ELISA clearly demonstrate the heterodimeric structure of the CD80-Heterominibodies.

### 2.2.3 ELISA-analysis with immobilized CTLA-4-lg fusion protein and an anti-human Cₖₐₚₚₐ detection antibody

In order to demonstrate interaction of the CD80 (B7-1)-arm of heterominibody M79scFVCK/CD80CH1 with its counterreceptor CTLA-4, a commercially available CTLA-4-Ig fusion protein (R&D Systems, Mineapolis MN USA Cat. No 325-CT) was immobilized on an ELISA-plate (coating concentration 10µg/ml) and after blocking with PBS/3%BSA incubated with different concentrations of purified heterominibody (in PBS/1%BSA); bound heterominibody was detected with a biotin labeled anti-human Cₖₐₚₚₐ antibody (Pierce, Cat.No. 31780) diluted 1:1000 in PBS/1%BSA followed by peroxidase conjugated avidin. As negative control some wells of the ELISA-plate were coated with a CD4-lg fusion protein instead of the CTLA-4-Ig fusion protein. The general ELISA procedure was performed as described above. The results shown in Fig. 39 clearly demonstrate specific binding of the heterominibody to human CTLA-4.

### Example 2.3 SDS-Proteingel

To determine the size of the purified heterominibody M79scFvCK/CD80CH1 SDS-PAGE was carried out with a 10% polyacrylamid-gel under nonreducing and reducing conditions according to Laemmli ( Laemmli, Nature 227 (1970), no. 259 680-5) gel followed by protein staining with ROTI®-Blue (Carl Roth GmbH + Co, Karlsruhe, Germany; Cat No A152.1). Compared to the molecular standard used (Rainbow™ colored protein molecular weight marker, range 14.300-2.200.000, Amersham LIFESCIENCE, Braunschweig, Germany, Cat No RPN 756) a distinct protein band could be seen under non-reducing conditions at about 115kD which is in accordance with the expected molecular weight. Under reducing conditions, two bands appeared of about 40 kD corresponding to the M79scFcCK-arm of the heterominibody and of about 60 kD corresponding to the CD80CH1-arm of the heterominibody. Thus, covalent linkage of the two heterominibody-arms via a disulfide bond could be confirmed with the "smearing" 60 kD-band demonstrating glycosylation of the CD80 extracellular domain.

### Example 2.4 Flowcytometric analysis of heterominibody M79scFvCK/CD80CH1

The binding of heterominibody M79scFvCK/CD80CH1 to native 17-1A-antigen was analyzed by flowcytometry. 200.000 17-1A transfected CHO cells (see example 5.1) were incubated for 30 minutes with several dilutions of purified heterominibody ranging from 4µg/ml to 3,9ng/ml. Thereafter, cells were washed twice in PBS and incubated for another 30 minutes with a R-Phycoerythrin conjugated murine anti-human CD80 antibody (Becton Dickinson Immunocytometry Systems, San Jose CA, USA, Cat. no. 340294). After two final washing steps in PBS, cells were analyzed by flowcytrometry (FACS-SCAN Becton Dickinson Immunocytometry Systems, San Jose CA, USA) For results see figure 5.

Alternatively, 17-1A transfected CHO-cells were incubated for 30 minutes on ice with purified heterominibody M79scFvCK/CD80CH1 at a concentration of 0,2 mg/ml. After washing in PBS, cells were incubated for 30 minutes on ice with a FITC-conjugated anti-human Cₖₐₚₚₐ antibody (Coulter, Cat. No. 6604287) diluted 1:10, washed again in PBS and subsequently subjected to flowcytometry; the result is shown in Fig. 37.

In order to demonstrate interaction of the CD80 (B7-1)-arm of heterominibody M79scFvCK/CD80CH1 with its counterreceptor CD28, a flowcytometric analysis on naive CD8⁺CD11b⁻-T cells was carried out, that were isolated as in Example 6.1; the cells were confirmed by standard flowcytometry to express CD28 but no CTLA-4, the other CD80-counterreceptor. Naive CD8⁺CD11b⁻-T cells were then incubated for 30 minutes on ice with purified heterominibody M79scFvCK/CD80CH1 at a concentration of 0,2 mg/ml. After washing in PBS, cells were incubated for 30 minutes on ice with a FITC-conjugated anti-human Cₖₐₚₚₐ antibody (Coulter, Cat. No. 6604287) diluted 1:10, washed again in PBS and subsequently subjected to flowcytometry. The result shown in Fig. 38 demonstrates binding of heterominibody M79scFVCK/CD80CH1 to CD28; binding specificity was confirmed by blocking the fluorescence signal with a commercially available CTLA-4-lg fusion protein (R&D Systems, Mineapolis MN USA Cat. No 325-CT).

### Example 3: CD80-antiLeyscFv-Heterominibodies constructs

### 3.1 Heterominibody antiLeyscFvCH1/CD80CK construct

A CD80-Heterominibody with another antigen specificity (anti-LewisY(LeY) was constructed by replacing the 17-1A-specific antigen binding-region M79scFv by the scFv-fragment of a marine monoclonal antibody directed against the LewisY-antigen (LeY), expressed on many epithelial tumor cells. For construction strategy see figure 1 and 3.For generating the antiLeyscFv-CH1-chain the vector pEF-ADA-M79scFv-CH1 described in example 1.1.2 and shown in figure 3 was cleaved with the restriction enzymes EcoRI and BspEl thus releasing the M79scFv-fragment including the N-terminal eukaryotic leader sequence. This scFv-fragment was then replaced by that of the LeY-specific antibody also carrying a eukaryotic leader at its N-terminus. The sequence of the corresponding EcoRI/BspEI-DNA-fragment is shown in figure 6. Subcloning was carried out in the E.coli strain XL-1 blue following standard methods (Sambrook, Molecular Cloning; A Laboratory Manual, 2nd Edition, Cold Spring Harbour Laboratory Press, Cold Spring Habour, NY (1989). The resulting expression plasmid pEF-ADA-anti LeYscFv-CH1 was linearized with Ndel and transfected into CHO-cells that were already transfected with the expression plasmid pEF-DHFR-CTI-CD80-Ckappa described in example 1 and shown in Figure 7.Primary selection of the resulting double transfectants was carried out as described in example 1. The expression of the Heterominibody anti-LeYscFvCH1/CD80CK was subsequently increased by gene amplification induced by the addition of the DHFR-inhibitor methotrexate (MTX) to a final concentration of 20nM, and the increase of deoxycoformycin (dCF) to a final concentration of 0,3µM described (Kaufman, Methods Enzymol. 185 (1990), 537-566).

### 3.2 Heterominibody antiLeyscFvCK/CD80CH1

For construction of heterominibody antiLeYscFvCK/CD80CH1 the M79scFv-fragment was excised from the vector pEF-DHFR-M79scFv-CK described in example 1 using the restriction enzymes EcoRI and BspEl and replaced by the EcoRI/BspEI fragment of the antigen binding region of the LeY-specific antibody as shown in figure 6. Subcloning was carried out in the E.coli strain XL-1 blue following standard methods (Sambrook, Molecular Cloning; A Laboratory Manual, 2nd Edition, Cold Spring Harbour Laboratory Press, Cold Spring Habour, NY (1989)).
The resulting expression plasmid pEF-DHFR-anti LeYscFv-CK was transfected into CHO-cells followed by transfection of the expression plasmid pEF-ADA-CD80-CH1 described in example 1. In order to increase the expression of heterominibody anti-LeYscFv-CK/CD80CH1, gene amplification was carried out as described above for heterominibody antiLeYscFvCH1/CD80CK.

### 3.3.1 ELISA-analysis of heterominibody antiLeyscFvCK/CD80CH1 and heterominibody antiLeyscFvCH 1/CD80CK with immobilized LeY-BSA-conjugate and an anti CD80 detection antibody

The culture supernatants of the corresponding transfectants harvested after the first step of gene amplification were tested by ELISA. For this purpose a commercially available BSA-conjugate of synthetic Lewis Y-antigen (Alberta Research Council, Canada) was coated to 96 well U-bottom ELISA plates (Nunc maxisorb) (30µg/ml 50µl/well) in phosphate buffered saline (PBS). Coating was performed over night at 4°C, blocking was performed with 3% bovine serum albumin (BSA) in PBS for one hour at room temperature. Culture supematants from the first amplification step (1. Amp.) (figure 7), were added and incubated for one hour at room temperature at different dilutions made in PBS containing 1% BSA.

Bound heterominibodies were detected by a CD80-specific monoclonal antibody (Immunotech, Cat No. 1449) diluted 1:1000 in PBS 1%BSA. After three times of washing with PBS 0,05% Tween20, a polyclonal peroxidase-conjugated Goat AntiMouse IgG-antibody (Fc-specific) diluted 1:5000 was added and incubated at room temperature for one hour. After four times of washing with PBS 0,05% Tween20, the ELISA was finally developed by adding the following substrate solution: 22 mg ABTS (2,2 Azino-bis (3-Ethylbenzthiazoline-6 Sulfonic Acid) Diammonium salt) dissolved in 10 ml 0,1M citrat buffer pH 5,1 containing 2,3 mg Sodium perborate Tetrahydrate. For negative controls, the plates were incubated with PBS instead of culture supernatants. The colored precipitate was measured at 405 nm using an ELISA-reader. The results are shown in figure 7 demonstrating the specificity of both heterominibody versions for the LeY-antigen and their heterodimeric structure.

### 3.3.2 ELISA-analysis of heterominibody antiLeyscFvCK/CD80CH1 and heterominibody antiLeyscFvCH1/CD80CK with immobilized anti-His-tag-antibody and an anti-human Cₖₐₚₚₐ detection antibody of the corresponding transfectants harvested after the first step of gene amplification constructs using his-coating

The culture supernatants were tested by ELISA. For this purpose, a BSA-free anti-His-tag-antibody (Dianova, Cat No 910) was coated to 96 well U-bottom ELISA plates (Nunc maxisorb) (25µg/ml 50µl/well) in phosphate buffered saline (PBS). Coating was performed over night at 4°C, blocking was performed with 3% bovine serum albumin (BSA) in PBS for one hour at room temperature. Culture supernatants from the first amplification step (1. Amp.) (figure 8), were added and incubated for one hour at room temperature at different dilutions made in PBS containing 1% BSA.
Bound Heterominibodies were detected by a biotin labeled anti-human Cₖₐₚₚₐ antibody (Pierce, Cat No.31780) diluted 1:1000 in PBS 1%BSA. After three times of washing with PBS 0,05% Tween20, peroxidase-conjugated avidin (DAKO, Hamburg Cat No P0347) was added and incubated at room temperature for one hour. After four times of washing with PBS 0,05% Tween20, the ELISA was finally developed by adding the following substrate solution: 22 mg ABTS (2,2 Azino-bis (3-Ethylbenzthiazoline-6 Sulfonic Acid) Diammonium salt) was dissolved in 10 ml 0,1M citrate buffer pH 5,1 containing 2,3 mg Sodium perborate Tetrahydrate. For negative controls, the plates were incubated with PBS instead of culture supernatant. The colored precipitate was measured at 405 nm using an ELISA-reader. The results are shown in figure 8 confirming the results of the foregoing ELISA.

### Example 4: Construction of M79scFv heterominibodies with different costimulatory proteins (CD54, CD58, CD86)

Heterominibodies containing three further costimulatory (CD86) or adhesion proteins (CD54, CD58) were constructed. CD54, CD58, CD86 were introduced into both heterominibody versions (see Example 1.1 and 1.2). The CD54, CD58 and CD86 fragments were obtained by polymerase chain reaction (PCR) using specific oligonucleotide primers complementary to the 5' and 3' ends of the nucleotide sequence encoding the extracellular part of these proteins. The cDNA template used for these PCRs were prepared by reverse transcription of the total RNA prepared from different cell lines as mentioned below according to standard procedures (Sambrook,Molecular Cloning; A Laboratory Manual, 2^{nd} Edition, Cold Spring Harbour Laboratory Press, cold Spring Habour, New York (1989)).

### 4.1 Construction of two M79scFv-CD54 heterominibody versions

The CD54 heterominibodies were constructed by replacing the extracellular part of CD80 within the heterominibodies described in example 1 by that of CD54.(figure 1). The construction strategy is described below.

### 4.1.1 Construction of the heterominibody M79scFvCH1/CD54CK

The CD54 antigen known as ICAM-1 (Intercellular adhesion molecule-1) belongs to the Ig-superfamily. It is a heavily glycosylated protein which is expressed on many lymphoid cells. e.g. dendritic cells. A more detailed description was published by Simmons D. et. al. Nature 331 (1987) 624-626. The cDNA template was obtained by reverse transcription of the total RNA from TPA-stimulated HL-60-cells. To amplify the extracellular domain of CD54, specific primers for the 5'and 3'end were used. These primers also introduced the restriction cleavage-sites EcoR1 and BspE1 (5' ICAM: CTC GAA TTC ACT ATG GCT CCC AGC AGC CCC CG and 3'ICAM: GAT TCC GGA CTC ATA CCG GGG GGA GAG CAC ). The further cloning and expression procedure was the same as described in example 1.1. for the corresponding CD80-Heterominibody resulting in double transfected CHO cells (pEF-ADA M79scFvCH1, pEF-DHFR-CTI-CD54-CK; figure 3) that proved to secrete the CD54-Heterominibody into the cell-culture medium.

### 4.1.2 Construction of the heterominibody M79scFvCK/CD54CH1

Another version of the CD54 heterominibody was constructed by replacing the M79scFv-and the CD54-fragment by each other. For this purpose the two expression plasmids pEF-DHFR-CTI-CD54-CK and pEF-ADA-M79scFv-CH1 described above were cleaved with Nde1 and BspE1, respectively. The CD54 containing fragment was cloned into the pEF-ADA vector fragment containing CH1. The resulting expression plasmid pEF-ADA-CD54-CH1 (figure 3) was transfected into transfected CHO cells (see example 1.2.1) already transfected with the pEF-DHFR-M79scFv-CK (figure 3) as described in example 1.1.1. The double transfected CHO cells were grown for selection as described in example 1

### Example 4.1.3 ELISA on cell-culture supematants of M79scFv-CD54 Heterominibodies

To analyze the 17-1A-binding specificity of both CD54 heterominibody versions and to confirm the proper association of CH1 and CK two different ELISA were carried. Specific binding to the 17-1A-antigen and the heterodimeric structure of the Heterominibodies was shown by incubation of culture supernatant on immobilized recombinant 17-1A-antigen and detection of bound CD54-Heterominibodies by an anti-CD54 antibody. The heterodimeric structure of the Heterominibodies was further confirmed by incubation of the culture supematant on immobilized anti-His-tag-antibody followed by a detection step with an anti-human Cₖₐₚₚₐ antibody. For details about both ELISAs see example 4.4, figure 9 and 10.

### 4.2 Construction of two M79scFv-CD58 heterominibody versions

The CD58 heterominibodies were constructed replacing the extracellular part of CD80 within the heterominibodies described in example 1 by that of CD58 (figure 1). The construction strategy is described below.

### 4.2.1 Construction of the M79scFvCH1/CD58CK heterominibody

CD58 also known as LFA-3 (Lymphocyte Function-Associated Antigen) is a protein belonging to the Ig-superfamily and is the counterreceptor of CD2. A more detailed description was published by Wallner B.P. et. al. J. Exp. Med 166 (1987) 923-932). The cDNA template was obtained by reverse transcription of the total RNA from U937 cells. To amplify the extracellular domain of CD58 and to introduce the restriction enzyme cleavage sites Xba1 and BspE1, specific 5'and 3'primers were used (5'LFA-3 AA TCT AGA ACC ATG GTT GCT GGG AGC GAC G and 3'LFA-3 AAG TCC GGA TCT GTG TCT TGA ATG ACC GCT GC). The further cloning and expression procedure was the same as described in example 1 except that Xbal instead of EcoRl was used due to an internal EcoRI-site within the CD58-DNA-fragment and a dam-methylase deficient E.coli-strain was used in order to prevent blocking of the BspEl site at the 3'-end of the CD58-fragment due to an overlapping dam-site. The finally resulting double transfected CHO cells (pEF-ADA-M79svFv-CH1, pEF-DHFR-CTI-CD58-CK, see figure 3) proved to secrete CD58 heterominibody into the cell-culture medium.

### 4.2.2 Construction of the heterominibody M79scFvCK/CD58CH1

Another version of the CD58 heterominibody was constructed replacing the M79scFv-and the CD58-fragment by each other (see figure 1). For this purpose two expression plasmids pEF-DHFR-CTI-CD58-Ck and pEF-ADA-M79scFv-CH1 described above were cleaved with Nde1 and BspE1, respectively. The CD58 containing fragment was cloned into the pEF-ADA vector fragment containing CH1. The resulting expression plasmid pEF-ADA-CD58-CH1 (figure 3) was transfected into CHO-cells already transfected with pEF-DHFR-M79scFv-CK as described in example 1. The double transfected CHO cells were grown for selection as described in Example 1.

### Example 4.2.3 ELISA on cell-culture supernatant of anti M79scFv-CD58-Heterominibodies

To analyze the 17-1A binding specificity of both CD58 heterominibody versions and to confirm the proper association of CH1 and CK, two different ELISAs were carried out. Specific binding to the 17-1A-antigen and the heterodimeric structure of the Heterominibodies was shown by incubation of culture supernatant on immobilized recombinant 17-1A-antigen and detection of bound CD58-Heterominibodies by an anti-CD58-antibody. The heterodimeric structure of the Heterominibodies was further confirmed by incubation of the culture supernatant on immobilized anti-His-tag-antibody followed by a detection step with an anti-human Cₖₐₚₚₐ antibody. For details about both ELISAs see example 4.4.

### 4.3 Construction of two M79scFv-CD86 heterominibody versions

The CD86 Heterominibodies were constructed by replacing the extracellular part of CD80 within the Heterominibodies described in example 1 by that of CD86 (figure 1). The construction strategy is described below.

### 4.3.1 Construction of the M79scFvCH1/CD86CK heterominibody

The CD86 costimulatory protein also known as B7-2 belongs to the Ig superfamily. It is a heavily glycosylated protein of 306 amino acids. A more detailed description was published by Freeman G. J. et. al. Science 262 (1993) 909-911. The cDNA template was obtained by reverse transcription of the total RNA from the Burkitt-Lymphoma cell line Raji. To amplify the extracellular domain of CD86 specific 5'and 3'primers (5'B7-2: 5'AAG TCT AGA AAA TGG ATC CCC AGT GCA CTA TG3', 3'B7-2: 5'AAT TCC GGA TGG GGG AGG CTG AGG GTC CTC AAG C3') were used. These primers also introduce Xba1 and BspE1 cleavage sites which were used to clone the CD86 PCR-fragment into the vector Bluescript KS-CT1-M79scFv. The further cloning and expression procedure was the same as described in example 1 except that Xbal instead of EcoRI was used due to an internal EcoRI-site within the CD86-DNA-fragment. The finally resulting double transfected CHO-cells (pEF-ADA-M79scFv-CH1, pEF-DHFR-CTI-CD86-CK) proved to secrete CD86-Heterominibody into the cell-culture medium.

### 4.3.2 Construction of the M79scFvCK/CD86CH1 heterominibody

Another version of the CD86 heterominibody was constructed by replacing the M79scFv-and the CD86-fragment by each other (figure 1). For this purpose the two expression plasmids pEF-DHFR-CTI-CD86-CK and pEF-ADA-M79scFv-CH1) were cleaved with Nde1 and BspE1, respectively. The CD86 containing fragment was cloned into the pEF-ADA vector fragment containing CH1. The resulting expression plasmid pEF-ADA-CD86-CH1 (figure 3) was transfected into CHO-cells already transfected with pEF-DHFR-M79scFv-CK as described in example 1. The double transfected CHO cells (pEF-DHFR-M79scFv-CK, pEF-ADA-CD86-CH1, figure 3) cells were grown for selection as described in example 1.

### Example 4.3.3 ELISA on cell-culture supernatant of CD86 heterominibody

To analyze the 17-1A binding specificity of CD86 heterominibody versions and to confirm the proper association of CH1 and CK two different ELISAs were carried out. Specific binding to the 17-1A-antigen and the heterodimeric structure of the Heterominibodies was shown by incubation of culture supernatant on immobilized recombinant 17-1A-antigen and detection of bound CD86-Heterominibodies by an anti-CD86 antibody. The heterodimeric structure of the Heterominibodies was further confirmed by incubation of the culture supernatant on immobilized anti-His-tag antibody followed by a detection step with an anti-human Cₖₐₚₚₐ antibody. For details about both ELISAs see example 4.4 see figure 9 and 10.

### Example 4.4 ELISA on cell-culture supernatants of M79scFv-heterominibodies

Two different ELISAs on cell-culture supernatants were performed for each M79scFv-Heterominibody:
Binding to the 17-1A-antigen was analyzed using recombinant 17-1A-antigen obtained by stable expression in CHO-celis as described (Mack et.al. Proc. Natl. Acad. Sci. 92 (1995) 7021-7025). The recombinant antigen consists of the first 264 amino acids of the native 17-1A antigen also known as GA 733-2 (Scala, Proc. Natl .Acad. Sci. 87 (1990), 3542-3546) followed by a stop codon.. The antigen was immobilized on 96 well U bottom ELISA plates (nunc maxisorb) at a concentration of 50µg/ml phosphate buffered saline PBS. Coating was carried out at 4°C for 12 hours with 50µl followed by washing once with (PBS) 0,05%Tween. The ELISA was then blocked for 1 hour with PBS/3%bovine serum albumin (BSA) and washed again once. Subsequently, the cell-culture supernatant was added undiluted and at several dilutions and incubated for 2 hours. Specific detection was dependent on the type of costimulatory proteins associated with the different heterominibody version. For specific antibodies and working dilutions see table 1. After three times of washing with PBS 0,05% Tween20, a polyclonal peroxidase-conjugated goat anti-mouse IgG-antibody (Fc-specific) was added and incubated at room temperature for one hour. After four times of washing with PBS 0,05% Tween20, the ELISA was finally developed by adding the following substrate solution: 22 mg ABTS (2,2 Azino-bis (3-Ethylbenzthiazoline-6 Sulfonic Acid) Diammonium salt) dissolved in 10 ml 0,1M citrate buffer pH 5,1 containing 2,3 mg Sodium perborate Tetrahydrate. For negative controls, the plates were incubated with PBS instead of culture supernatants. The colored precipitate was measured at 405 nm using an ELISA-reader (figure 9). The results shown in figure 9 clearly demonstrate that each of the constructed M79scFv-Heterominibodies could be detected as fully functional heterodimer in the supernatant of the corresponding transfectants.
For the second ELISA an anti-His-tag-antibody (DIANOVA; Hamburg Cat. no. DIA 910) diluted 1:40 was coated to 96-well plates as described above. Supematants of all heterominibody versions were added pure and in several dilutions. A biotinylated anti human Ckappa antibody followed by peroxidase-conjugated streptavidin (1:1000) (DAKO, Hamburg Cat. no P0347) was used for detection of bound Heterominibodies (see table 1). The ELISA was developed as described above. For results see figure 10.

### Example 5: Stimulation of naive CD4+CD45RO- T cells by M79scFcCK/CD80CH1 heterominibody

### Example 5.1 Purification of naive CD4+CD45RO- from the peripheral blood of healthy human donors blood

To analyze the biological function of the M79scFvCK/CD80CH1-heterominibody, a CD4+ T-cell stimulation experiment was performed. CD4+CD45RO- T-cells, commonly considered to be naive, were isolated from of peripheral blood of healthy donors by negative selection. First, peripheral blood mononuclear cells (PBMC) were isolated by Ficoll Density Gradient (Current Protocols of Immunology, Coligan, Kruisbeek, Margulies, Shevach and Strober, Wiley-Interscience, 1992). After washing the cells three times with phosphate buffered saline (PBS) supplemented with 2% fetal calf serum (FCS), CD4+ T-cells were purified by using commercially available CD4+-T-cell columns (R&D Systems, Minneapolis MN USA, Cat no HCD43). In the next step CD45RO+ T-cells were removed by paramagnetic Dynabeads M450 (Dynal, Hamburg, Cat.No. 110.02). For this purpose, CD4+ T-cells were incubated for 30 minutes with the murine anti-human CD45RO antibody (UHCL-1) at a concentration of 10µg/ml. Subsequently the cells were washed twice and thereafter incubated for another 30 minutes with magnetic beads conjugated with the sheep anti-mouse IgG1 antibody M450. The CD4+CD45RO+ T-cells that were quantitatively attached to magnetic beads were then removed by the application of magnet. The remaining cells were CD4+ and CD45RO- with a purity of 98% as confirmed by flowcytometry.

### Example 5.2 Stimulation of naive CD4+CD45RO- T-cells by simultaneous incubation with the M79scFvCK/CD80CH1 heterominibody and/or bispecific single chain antibody M79scFv-antiCD3scFv

CD4+CD45RO- T-cells were purified as described above. The stimulation was performed in 96-well TPP flat-bottom plates. The stimulation assay was carried out as follows. 17-1A transfected CHO-cells were used as stimulator cells. This 17-1A transfected cell-line was generated by subcloning of a DNA-fragment encoding the complete amino acid sequence of the 17-1A-antigen also known as GA733-2 (Szala, Proc. Natl. Acad. Sci. USA 87(1990) 3542-3546), into the eukaryotic expression vector pEFDHFR according to standard procedures (Sambrook, Molecular Cloning; A Laboratory Manual, 2^{nd} Edition, Cold Spring Harbour Laboratory Press, Cold Spring Habour, NY (1989); linearization of the resulting plasmid with the restriction enzyme Nde I and subsequent stable transfection into DHFR-deficient CHO cells was performed as described in example 1.1.1. The expression of transmembrane 17-1A was increased by gene amplification induced by stepwise addition of increasing concentrations of the DHFR-inhibitor Methotrexat (MTX) to a final concentration of 500nM, with the concentration steps in between being 20nM and 100nM (Kaufman, Methods Enzymol. 185 (1990), 537-566).
These cells were tested for membrane expression of 17-1A by flowcytometry using the 17-1A-specific monoclonal antibody M79 (Göttlinger, Int. J. Cancer 38 (1986) 47-53) at a concentration of 10 µg/ml followed by a polyclonal goat anti mouse IgG + IgM (H+L) antibody diluted 1:100 in PBS. As negative control untransfected CHO cells were used whereas the 17-1A-positive human gastric cancer cell-line Kato, obtained from ATCC served as positive control. Results are shown in figure 11.
Before using these cells for T-cell stimulation they were irradiated with 14000 rad, washed twice in PBS, 2%FCS, counted, diluted in medium (for details see below) and seeded into 96-well plates at a number of 25.000 cells per well. 50.000 CD4+CD45RO-T-cells cells were added to each well thus resulting in a T-cell/stimulator cell ratio of 2:1. M79scFvCK7CD80Cm-heterominibody and/or the bispecific single chain antibody M79scFv-antiCD3scFv were added in several concentrations as shown in (Table 2) Cells were grown in RPMI 1640 medium supplemented with 10% human AB serum, 100U/ml Penicillin, 100mg/ml Streptomycin, 2mM Glutamin, 1mM sodium pyruvat, 10mM HEPES-buffer, 50µM Mercaptoethanol at 37°C 6% CO2 and 100% humidity for up to 12 days.

### 5.3 BrdU-Proliferationassay

In order to measure the proliferation kinetics of CD4+CD45R0- T-cells simultaneously stimulated by M79scFvCK/CD80CH1-Heterominibody and the bispecific single chain antibody M79scFv-antiCD3scFv or stimulated with the bispecific single chain antibody alone, a BrdU proliferation assay was performed. For details see product description by Boehringer Mannheim Cat.No. 1647229. The results shown in figure 12 clearly demonstrate a substantially increased cell proliferation induced by heterominibody plus the bispecific single chain antibody compared to that induced by the bispecific antibody alone.

### 5.4 Flowcytometric analysis of CD4+CD45R0- T-cells stimulated with the M79scFvCK/CD80CH1 heterominibody and/or the bispecific single chain antibody M79scFv-antiCD3scFv

CD45RO and CD45RA expression levels on stimulated T-cells were analyzed by flowcytometry (FACS Scan, Becton Dickinson) on day 3 and 6 of the stimulation experiment. Stimulated T-cells as well as controls (see example 5.1) were incubated for 30 minutes with different combinations of antibodies listed in table 4.

T-cells were incubated with the following three antibody combinations: anti CD45RO FITC/ anti CD45RA PE, anti CD45RO FITC / Isotyp IgG1 PE, anti CD45RA PE/ Isotyp IgG2a FITC. The percentage of primed T-cells that switched to the surface phenotype CD45R0+/CD45RA- depending on the concentrations of heterominibody and bispecific antibody is shown in figures 13 and 14 corresponding to a stimulation time of 3 and 6 days, respectively. The final result of T-cell stimulation after 6 days is also shown in Figure 33.

### 5.5 INF-γ ELISA analysis of cell culture supernatant of stimulated CD4+ T-cells

In order to confirm in vitro priming of CD4+ T-cells by the combination of heterominibody and bispecific single chain antibody, the INF-γ-concentration in the T-cell culture supernatant was determined using a semi-quantitative INF-γ ELISA (Genzyme DuoSet, Genzyme Diagnostics Cambridge, MA USA Cat No. 80-3932-00) was performed according to manufacture's manual.. Since INF-γ is typically secreted by primed TH1- but not by naive CD4+ T-cells, the results shown in figures 15 and 16 demonstrate that T-cell priming has occurred in the presence of both heterominibody and bispecific single chain antibody but not with the bispecific antibody alone. Furthermore the secretion of INF-γ by the primed CD4+T-cells strongly indicates the differentiation of these cells into TH1-phenotype.

### Example 5.6 IL-5 ELISA -analysis of cell culture supernatant of stimulated CD4+ T-cells

A second ELISA was performed analyzing the IL-5 secretion of stimulated CD4+ cells. However the IL-5 ELISA (Genzyme DuoSet, genzyme Diagnostics Cambridge, MA USA Cat.No. 80-5025-00) of T-cell culture supernatant did not detect any IL-5 secretion as shown in figures 17 and 18 Since IL-5 is typically secreted is by primed CD4+ T-cells of the TH2-phenotype, the combined T-cell stimulation by the M79scFvCK/CD80CH1 heterominibody and the bispecific single chain antibody M79scFv-antiCD3scFv proved to induce no priming of TH2 T-cells at all.

### Example 5.7 IL-4 ELISA-analysis of cell culture supernatant of stimulated CD4+-T cells

Similar to the IL-5 analysis described in Example 5.6, T cell culture supematants were analyzed by ELISA (Pharmingen,Cat. No. 2629KI) for the precence of IL-4, another cytokine typically secreted by primed CD4+-T cells of the TH2-phenotype. Since no IL-4 could be detected, stimulation of naive CD4⁺-T cells with M79SCFVCK/CD80CH1-Heterominibody and the bispecific single chain-antibody M79scFv-antiCD3scFv was confirmed to induce no priming of TH2-cells.

### Example 6: Costimulation of CD8+CD45RO- T cells by theM79scFVCKCD80CH1-Heterominibody

### Example 6.1 Purification of naive CD8+CD45RO- T-cells from the peripheral blood of healthy human donors

To analyze the biological function of the M79scFv-CK/CD80-CH1 Heterominibody, a CD8+ T-cell stimulation experiment was performed. CD8+CD45RO- T-cells, commonly considered to be naive, were isolated from peripheral blood of healthy donors by negative selection. At first, peripheral blood mononuclear cells (PBMC) were isolated by Ficoll Density Gradient (Current Protocols of Immunology, Coligan, Kruisbeek, Margulies, Shevach and Strober, Wiley-Interscience, 1992). After washing the cells three times with phosphate buffered saline (PBS) supplemented with 2% fetal calf serum (FCS), CD8+T-cells were purified, using commercially available CD8+-T-cell columns (R&D Systems, Minneapolis MN USA, Cat No HCD8C-1000). In addition to the manufacture's protocol 1µg/ml murine anti human CD11b antibody was added to the supplied antibody cocktail in order to remove the suppressor T-cells that are CD11b+/CD28⁻. In the next step CD45RO+ T-cells were removed by use of paramagnetic Dynabeads M450 (Dynal, Hamburg, Cat.No. 110.02) For this purpose CD8+T-cells were incubated for 30 minutes with the murine anti-human CD45RO antibody (UHCL-1) at a concentration of 10µg/ml. Subsequently, the cells were washed twice and thereafter incubated for another 30 minutes with magnetic beads conjugated with the sheep anti-mouse IgG1 antibody M450. The CD8+CD45RO+T-cells that were quantitatively attached to magnetic beads were then removed by the application of a magnet The remaining cells were CD8+CD45RO-CD28+. with a purity of 95-98%.

### Example 6.2 Stimulation of naive CD8+CD45R0-T-cells by simultaneous incubation with the M79scFvCK/CD80CH1-Heterominibody and/or the bispecific single chain antibody M79scFv-anti CD3scFv

CD8+CD45RO- were stimulated as described in example 5using construct concentrations as displayed in table 3

BrdU Proliferationassay as well as FACS analysis were performed as described in example 5. For results see figures 19, 20, 21 and 34.

### Example 6.3 TNF-α ELISA analysis of cell culture supernatant of stimulated CD8-T-cells

In order to confirm in vitro priming of CD8+T-cells by the combination of heterominibody and bispecific antibody, the TNF-α-concentration in the T-cell culture supernatant was determined using a semiquantitative TNF-α ELISA (Genzyme DuoSet, Genzyme Diagnostics Cambridge, MA USA Cat No. 80-3932-00) which was carried out according to manufacture's manual. Since TNF-α is typically secreted by primed but not by naive CD8+T-cells the results shown in figure 22 demonstrate that T-cell priming has occurred in the presence of both hetrominibody and bispecific antibody but not with the bispecific antibody alone.

### Example 6.4 Cytotoxic activity of in vitro primed CD8+-T lymphocytes

During the priming process, initially naive CD8+-T cells usually acquire the capability of mediating target cell cytotoxicity in a CD80 (B7-1) independent manner. CD80 (B7-1) independence is thus an excellent biologic marker for primed T-cells. Therefore, initially naive CD8⁺CD11 b⁻CD45RO⁻-T cells that had been primed for 6 days as described in Example 6.2 were used as effector cells in a ⁵¹Cr-release test; T cell cytotoxicity was redirected against 17-1A-positive Kato cells by the bispecific single chain antibody M79scFv-antiCD3scFv. As shown in Fig. 35, in vitro primed CD8⁺-T cells proved to be highly cytotoxic against Kato cells in a 20h-⁵¹Cr release test even exceeding fresh PBMC in cytotoxic activity. In contrast naive CD8⁺-T cells exhibited no significant cytotoxic effects. As expected, due to CD80 (B7-1) independence, in vitro primed CD8⁺-T cells did not show increased redirected cytotoxicity in the presence of heterominibody M79scFVCK/CD80CH1. Thus, priming of naive CD8⁺-T cells by virtue of heterominibody M79scFVCK/CD80CH1 in combination with an appropriate primary signal could be clearly demonstrated by a third parameter in addition to the change of CD45RA/RO-phenotype and cytokine secretion. The ⁵¹Cr-release assay was carried out as described by Mack, Proc. Natl. Acad. Sci. USA 92 (1995) 7021-7025.

### Example 7: Heterominibody M79scFvCKantiCD3scFv/CD80CH1

Another version of the CD80 heterominibody shown in Figure 23 was constructed by adding an antiCD3scFv-fragment via a Glycin₄Serin₁-linker to the C-terminus of the M79scFvCK-polypeptide chain described in example 1. For this purpose, the DNA-fragment encoding this polypeptide chain was excised from the expression plasmid pEF-DHFR-M79scFv-CK described in example 1 and subcloned in the vector pMa (Stanssens, Nucleic Acids Res.17(1998)441-4454) using the restriction enzymes EcoRI and Sall (Boehringer, Mannheim). The antiCD3scFv-fragment was PCR-amplified from the DNA template encoding the bispecific single chain antibody M79scFv-antiCD3scFv described by Mack, Proc. Natl. Sci. U.S.A. 92 (1995) 7021-7025 by using the following primers. The 5'primer VHTR66CKSAC (5'-CCT GAG CTC GCC CGT CAC AAA GAG CTT CAA CAG GGG AGA GTG TGG AGG TGG TGG ATC CGA TAT C-3'), introduced a Sacl-site and the 3'primer VLTR66SalNotXba introduced the cleavage sites Sall, Notl and Xbal (5'-ATT CTA GAG CGG CCG CGT CGA CTA TTT CAG CTC CAG CTT GGT CCC AGC -3') The resulting PCR fragment was cloned according to standard procedures (Sambrook, Molecular Cloning; A Laboratory Manual, 2^{nd} Edition, Cold Spring Harbour Laboratory Press, cold Spring Habour, New York (1989)) into the pMa-vector by using the restriction enzyme cleavage sites Sacl and Xbal (Boehringer Mannheim). In the final step the whole M79scFvCKantiCD3scFv-fragment shown in Figure 32 was excised from the pMa-vector by the restriction enzymes EcoRI and Sall and subcloned into the eukaryotic expression vector pEF-DHFR described by Mack, Proc. Natl. Acad. Sci. 92 (1995)7021-7025 The resulting expression plasmid was transfected into DHFR-deficient CHO-cells, prior to transfection with the expression-plasmid pEF-ADA-CD80-CH1 described in example 1. Double transfection and selection of the CHO-cells as well as production and purification of the heterominibody was carried out as described in Example 1.

To demonstrate binding of heterominibody M79scFVCKantiCD3scFv/CD80CH1 to the 17-1A-antigen and to confirm the presence of correctly folded CD80 as well as the heterodimeric structure of the heterominibody an ELISA was carried out with cell culture supematants after primary selection and after the first gene amplification step; recombinant 17-1A-antigen was immobilized and bound heterominibody detected with an anti-CD80 antibody as described in Example 1.2.3. The results are shown in Fig. 40.

The increase of the expression level due to gene amplification was also monitored by ELISA; therefor recombinant 17-1A-antigen was immobilized, incubated with culture supernatant containing heterominibody M79scFVCKantiCD3scFv/CD80CH1 and bound heterominibody detected with a peroxidase-conjugated anti-his-tag antibody (Roche, Cat.No. 1965085) diluted 1:500. The results are shown in Fig. 41. The general ELISA procedure was performed as described in Example 4.4.

Interaction of the C-terminally located scFv-fragment to CD3 on human T cells was confirmed by flowcytometry. For this purpose, CD11b-positive CD8⁺-T cells were isolated, because this T cell subset is known to be CD28-negative. Isolation of CD11b-positive CD8⁺-T cells was carried out with commercially available CD8⁺-T cell columns as described in Example 6.1 except that no CD11b-antibody was added to the manufacturer's antibody cocktail. In the next step, CD11b-positive T cells were positively selected by using paramagnetic Dynabeads M450 (Dynal, Hamburg; Cat.No. 110.02); for this purpose CD8⁺-T cells were incubated for 30 minutes with a murine anti-human CD11b-antibody at a concentration of 10µg/ml. Subsequently, the cells were washed twice and thereafter incubated for another 30 minutes with magnetic beads conjugated with a sheep anti-mouse IgG antibody. The CD11b-positive CD8⁺-T cells attached to the magnetic beads were then isolated by the application of a magnet and were then confirmed by standard flowcytometry to express neither CD28 nor CTLA-4. The CD11b-positive CD8⁺-T cells were then incubated for 30 minutes on ice with purified heterominibody M79scFVCKantiCD3scFv/CD80CH1 at different concentrations. After washing in PBS, cells were incubated with a FITC-conjugated anti-human Cₖₐₚₚₐ-antibody (Coulter Cat.No. 660287) diluted 1:10, washed again in PBS and subsequently subjected to flowcytometry. The results shown in Fig. 42 clearly demonstrate binding of the heterominibody to human CD3 on T cells, since both counterreceptors of CD80 are absent from the particular T cell subset used in this experiment. Furthermore, these results exemplify functionality of a polypeptide with receptor or ligand function when located at a C-terminal position within a heterominibody.

A second antiCD3scFv-derivative of heterominibody M79scFVCK/CD80CH1 was obtained by inserting a PCR-DNA-fragment encoding the anti-CD3scFv-fragment of the bispecific single chain antibody (bscAb) M79scFv-antiCD3scFv described by Mack, Proc. Natl. Acad. Sci. USA 92 (1995) 7021-7025 into the BspEl restriction site of the expression plasmid pEF-DHFR-M79scFv-CK (see Example 1.2.1). The antiCD3scFv-fragment was PCR-amplified from the DNA template encoding bscAbM79scFv-antiCD3scFv using the 5'primer VHTR66BspEI (5'-GTC ACC GTC TCC TCC GGA G-3') and the 3'primer VLTR66BspEI (5'-GTG TCC GGA TTT CAG CTC CAG CTT GGT CC-3') and digested with BspEl prior to cloning into pEF-DHFR-M79scFv-CK. The correct orientation of the cloned fragment was checked by PCR using the primers 5'VHTR66BspEI (5'-GTC ACC GTC TCC TCC GGA-G3') hybridizing immediately upstream of the M79scFv-DNA-sequence within the expression vector pEF-DHFR and 3'Hinge-Seq (5'-GGT GTG GGT GGT GTC ACC-3'). The resulting expression plasmid pEF-DHFR-bscAbM79scFv-antiCD3scFv-CK (see Fig. 44) was transfected together with the expression plasmid pEF-ADA-CD80-CH1 into CHO cells as described in Example 1.2.1. It is noteworthy, that in this case the polypeptide with receptor or ligand function fused to the N-terminus of the Cₖₐₚₚₐ-domain of the resulting heterominibody (see Fig. 43) is identical with the bispecific single chain antibody M79scFv-antiCD3scFv (Mack, Proc. Natl. Acad. Sci. USA 92 (1995) 7021-7025). Gene amplification as well as production and purification of the heterominibody was carried out as described in Example 1.2.2.
In order to confirm correct folding of the CK-arm of heterominibody bscAbM79scFv-antiCD3scFv-CK/CD80-CH1 as well as the heterodimeric structure of the recombinant molecule, binding to immobilized 17-1A-antigen was shown by ELISA, that was carried out with cell culture supematants after primary selecion and after the first gene amplification step. Bound heterominibody was detected with a peroxidase-conjugated anti-His-tag antibody (Roche, Cat.No. 1965085) diluted 1:500. The results are shown in Fig. 45. The general ELISA procedure was performed as described in Example 4.4.
In order to confirm the presence of correctly folded CD80 within the heterominibody bscAbM79scFv-antiCD3scFv-CK/CD80-CH1 the following ELISA with cell culture supematants after primary selection and after the first gene amplification step was carried out:
A monoclonal antibody against human CD80 (Immunotech, Cat.No. 1449) diluted 1:200 was immobilized on an ELISA-plate followed by incubation with cell culture supematants. Subsequently, heterominibody captured by anti-CD80 antibody was detected with a peroxidase-conjugated anti-His-tag antibody (Roche, Cat.No. 1965085) diluted 1:500. The results are shown in Fig. 46. The general ELISA procedure was performed as described in Example 4.4.
In summary, these results exemplify, that the heterominibody format can also carry polypeptides with more complex protein structures and/or more complex receptor or ligand function like e.g. bispecific single chain antibodies.

### Example 8: CD80-M79scFv constructs

### 8.1 CD80 - M79 scFv (VL/VH) construct with short (Gly₄Ser₁)₁ linker

A protein was constructed that consists of the single-chain Fv fragment(scFv) of the murine anti 17-1A antibody M79 and the extracellular part of the human costimulatory protein CD80 (B7-1) connected by a (Gly₄Ser₁)₁ linker (Figure24). The M79 antibody was obtained as described by Göttlinger et. al.(1986) Int.J.Cancer:38, 47-53. The M79 scFv fragment was cloned as described by Mack et. al. Proc. Natl. Acad. Sci. 92 (1995)7021-7025. The complete plasmid was cloned in several steps. First a poly-linker designated CTI was inserted into the Bluescript KS vector (GenBank® accession number X52327) using the restriction enzyme cleavage sites Xbal and Sall (Boehringer Mannheim). The introduction of the polylinker CTI provided additional cleavage sites as well as the sequence encoding the (Gly₄Ser₁)₁, linker a six-amino acid histidine tag and a stop codon as shown in Figure 2 .The vector Bluescript KS + CTI was prepared by cleavage with the restriction enzymes EcoRV and Xmal (Boehringer Mannheim and New England Biolabs) in order to ligate it (T4 DNA, Ligase Boehringer Mannheim) with the M79 scFv fragment cleaved by EcoRV and BspEl (). The resulting vector Bluescript KS+CTI+M79 scFv again was cleaved with EcoRI (Boehringer Mannheim) and BspEl in order to insert the CD80 DNA-fragment which was previously prepared using the same enzymes. Prior to subcloning, the CD80 fragment was obtained by polymerase chain reaction (PCR) using specific oligonucleotide primers complementary to the 5' and 3' ends of the nucleotide sequence encoding the extracellular part of CD80 (Freeman G.J et al. J.Immunol.143,(1989) 2714 - 2722.). These primers also introduced an EcoRI and a BspEl cleavage site (5'CD80 Primer: 5'GCA GAA TTC ACC ATG GGC CAC ACA CGG AGG CAG 3'; 3'CD80 Primer: 5'TGG TCC GGA GTT ATC AGG AAA ATG CTC TTG CTT G 3') The cDNA template used for this PCR was prepared by reverse transcription of the total RNA prepared from the Burkitt-lymphoma cell line Raji according to standard procedures (Sambrook, Molecular Cloning; A Laboratory Manual, 2^{nd} Edition, Cold Spring Harbour Laboratory Press, cold Spring Habour, New York (1989)).

The CD80 costimulatory protein belongs to the Ig superfamily. It is a heavily glycosylated protein of 262 amino acids. A more detailed description was published by Freeman G.J et. al. J.Immunol.143,(1989) 2714 - 2722.

In the last step, the whole CD80-M79scFv (VL/VH)DNA fragment (figure 25) was isolated by cleaving the vector Bluescript KS+CTI+CD80+M79scFv (VL/VH) with EcoRI and Sall (Boehringer Mannheim) and subsequently introduced into the eukaryotic expression vector pEF-DHFR described in Mack et. al. Proc. Natl. Sci. U.S.A. 92 (1995) 7021-7025. containing the dihydrofolatereductase gene as selection marker. The final plasmid was linearized with the restriction enzyme Ndel (Boehringer Mannheim) and transfected into CHO cells by electroporation. The electroporation conditions were 260V/960µF using a BioRad Gene Pulser™. Stable expression was performed in DHFR deficient CHO-cells as described by Kaufmann R. J. et. al. (1990) Methods Enzymol. 185, 537-566. The cells were grown for selection in nucleoside free α-MEM medium supplemented with 10% dialyzed FCS and 2 mM L-glutamine. For production of the bifunctional CD80-M79 scFv (VL/VH)construct, cells were grown in rollerbottles (Falcon) for 7 days in 300ml culture medium. The protein was purified via its His-tag attached to the C-terminus (see figure24) by using a Ni-NTA-column (Mack et. al. Proc. Natl. Acad. Sci. 92 (1995)7021-7025).To analyze the binding properties different ELISA were performed:

### 8.1.1 ELISA with cell culture supernatant using anti-His-tag detection

Binding to the 17-1A-antigen was analyzed using soluble 17-1A-antigen obtained as described (Mack et. al. Proc. Natl. Acad. Sci. 92 (1995)7021-7025) by stable expression in CHO-cells of the DNA encoding the first 264 amino acids of the 17-1A antigen also known as GA 733-2 (Scala, Proc. Natl. Acad. Sci. 87 (1990) 3542-3546) followed by a stop codon.. The antigen was immobilized on 96 well U bottom ELISA plates (nunc maxisorb) at a concentration of 50µg/ml phosphate buffered saline PBS. Coating was carried out at 4°C for 12 hours with 50ul followed by washing once with (PBS) 0,05%Tween. The ELISA was then blocked for 1 hour with PBS/3%bovine serum albumin (BSA) and washed again once. Now the cell-culture supernatant was added undiluted and at several dilutions and incubated for 2 hours. As detection system a murine IgG1 anti His-tag antibody (dianova, Hamburg) diluted 1:200 and a peroxidase conjugated polyclonal goat anti mouse IgG (Fc) (dianova, Hamburg) antibody were applied sequentially. The ELISA was developed by adding ABTS-substrate solution (2'2 Azino-bis (3-Ethlbenzthiazoline-6-Sulfonic Acid), SIGMA A-1888, Steinheim) as described in example 2.1 . The result was measured by an ELISA-Reader at OD 405 nm; results are shown in Figure 26. Obviously no binding activity could be measured. As negative controls, the plates were incubated with PBS instead of antibody constructs. As positive control served the anti-17-1A/anti-CD3 bispecific-single-chain antibody described previously (Mack et. al. Proc. Natl. Acad. Sci. 92 (1995) 7021-7025).

### 8.1.2 ELISA with cell culture supernatant using anti-CD80 detection

Immobilization of 17-1A-antigen, blocking and the incubation of cell culture supematants was performed as described above. Detection was carried out with a murine lgG1 anti-CD80-antibody diluted 1:1000 (dianova, Hamburg) followed by a peroxidase conjugated polyclonal goat anti-mouse IgG (Fc)-antibody diluted 1:5000 (dianova, Hamburg). The ELISA was developed with ABTS-substrate solution and OD-values were measured as described above, however, again no 17-1A-binding activity could be detected. As positive control, the anti-17-1A/anti-CD3 bispecific-single-chain antibody (Mack et. al. Proc. Natl. Acad. Sci. 92 (1995)7021-7025) was used and detected with the described anti-His-tag antibody. Results are shown in Figure 27.

### 8.1.3 ELISA-analysis of purified recombinant CD 80-M79scFv-construct

As the ELISAs with cell-culture supematants detecting specific antigen binding were all negative, soluble CD80-M79scFv was obtained by protein purification from supernatant of a roller bottle culture (300ml) in order to exclude the possibility that no recombinant protein was secreted into the supemantant. The purification was carried out using a Nickel-NTA-column as described (Mack, M et. al. Proc. Natl. Sci. U.S.A. 92 (1995) 7021-7025). ELISA wells were coated with the protein eluted from the Nickel-NTA-column. Detection of the bifunctional CD80-M79scFv-construct was performed independently of its 17-1A-antigen binding activity by using either an anti His-tag antibody (see example 8.1.1.) as well as an anti-CD80 antibody (see example 8.1.2.) in separate experiments followed by an anti-mouse IgG(Fc) antibody, respectively. Development of the ELISA as well as the measurement of the OD-values was carried out as described above. The results are shown in Fig 28., confirming the presence of the CD80-M79scFv-construct in the cell culture supernatant.

### 8.2. CD80 - M79 scFv (VH/VL) construct with (Gly₄Ser₁)₁ linker

To change the arrangement of the Ig variable regions within the M79scFv fragment from VL/VH to VH/VL a two step fusion PCR using oligonucleotide primers 5'VHB5RRV:AGG TGT ACA CTC CGA TAT C(A,C)A (A,G)CT GCA G(G,C)A GTC (A;T)GG, 3'VHGS15: 5'GGA GCC GCC GCC GCC AGA ACC ACC ACC ACC TGA GGA GAC GGT GAC CGT GGT CCC TTG GCC CCA G 3', 5'VLGS15: 5'GGC GGC GGC GGC TCC GGT GGT GGT GGT TCT GAC ATT CAG CTG ACC CAG TCT CCA3' and 3'VLBspEl: 5'AAT CCG GAT TTG ATC TCG AGC TTG GTC CC3' was performed according to the procedure described by Mack et al. Proc. Natl. Acad. Sci. 92 (1995) 7021-7025 (see also example 2.1.) The PCR-fragment encoding the VH/VL-scFv-fragment was cleaved with the restriction enzymes EcoRV/BspEl and inserted into the vector Bluescript KS + CTI already prepared by cleavage with EcoRV/Xmal (see example 8.1.).Next, the inverted M79scFv (VH/VL) fragment was excised with the restriction enzymes BspEI/Sall and introduced into the plasmid pEF-DHFR+CTI + CD80-M79scFv (VL/VH) using BspEl/Sall thus replacing the M79scFv- VL/VH fragment (see Fig 25). Transfection and cell culture procedures were carried out as described above. Analysis of antigen binding was performed using the described 17-1A-ELISA (example 8.1.2.). However, no 17-1A binding activity of the alternatively arranged CD80-M79scFv-construct could be detected. Results are shown in Fig 29.

### 8.3. CD80 - M79 scFv (VHNL) construct with a long (Gly₄Ser₁)₃ linker

First, the M79scFv (VHNL) fragment was obtained by a two step fusion PCR as described in example 8.2. The PCR fragment encoding the VH/VL-scFv-fragment was cleaved with the restriction enzymes EcoRV/BspEI and subcloned into the Bluescript KS +CTI vector cleaved EcoRV/Xmal (see example 8.1). In a further step a longer Glyin-Serin linker (Gly₄Ser₁)₃ consisting of 15 amino acids was introduced. Therefor, another oligonucleotide linker (ACCGS15BAM) which was designed to encode the (Cly₄Ser₁)₃ linker and to provide BspEl and BamHl compatible overhangs had to be inserted into the Bluescript KS + CTI + M79 scFv (VH/VL)(example 8.2). The nucleotide sequence of the linker is shown in Fig 30.

The plasmid Bluescript KS + CTI + M79 scFv (VH/VL) including the coding sequence of the (Gly₄Ser₃)₃ linker was cleaved with BspEI and Sall and the resulting DNA-fragment (Gly₄Ser₁)₃+M79scFv (VH/VL) was inserted into the BspEl/Sall-cleaved vector pEF-DHFR that contains the CD80-coding fragment (example8.1) thus replacing the M79scFv (VL/VH) fragment together with the short (Gly₄Ser₁)₁ linker(see Fig 25). For transfection and cell culture procedure example 8.1.. Antigen specific binding was analyzed by 17-1A ELISA as described above (example 8.1.1) and detection of functional recombinant protein in the cell-culture supernatant was performed with an anti His-tag antibody followed by an anti mouse IgG (Fc) antibody (compare example 8.1.1) The anti-17-1A/anti-CD3 bispecific-single-chain antibody (Mack et. al. Proc. Natl. Acad. Sci.. 92 (1995) 7021-7025) served as positive control. Development of the ELISA and measurement of the OD values was carried out as described above(example 8.1.1) However, no antigen binding was detectable. Results are shown in Fig 31.

### Example 9: Unpredictable expression results in mammalian host cells of recombinant proteins carrying oligomerization domains established in E.coli

In order to find out, whether the feasibility of polypeptide oligomerization strategies in mammalian host cells can be predicted by their successful use in E.coli, two different oligomerization domains characterized in bacterial expression systems were tested in fusion proteins expressed by mammalian cells (Pack (1993) Biotechnology 11: 1271; Rheinnecker (1996) J. Immunol. 157: 2989). The first fusion protein, that was tested, consists of two functional domains, the 17-1A-specific M79scFv-fragment at the N-terminus and human IL-2 at the C-terminus; between these two functional domains, either a dimerization or a tetramerization domain was inserted resulting in the polypeptide chains shown in Figures 47 and 48, respectively. The corresponding DNA-fragments were cloned into the expression vector pEF-DHFR with the restriction enzymes EcoRl and Sall and transfected into DHFR-deficient CHO-cells as described (Mack, Proc. Natl. Acad. Sci. USA 92 (1995) 7021-7025) (note that both DNA-sequences shown in Figures 47 and 48 carry an internal recognition site for EcoRI, thus requiring partial digestion with this restriction enzyme). Culture supernatants of the transfected CHO-cells as well as the corresponding cell lysates prepared according to standard procedures were tested by ELISA. Immobilized recombinant 17-1A-antigen was incubated with culture supernatant or cell lysate and bound construct detected with a peroxidase-conjugated anti-His-tag antibody (Roche, Cat.No. 1965085) diluted 1:500. The general ELISA procedure was performed as described in Example 4.4. The results shown in Fig. 50 demonstrate, that only the tetrameric but not the dimeric construct is found in the supernatant and thus produced as secretable and fully functional protein in mammalian cells, although both are well detectable in the cell lysate. In conclusion, the dimerization domain proved to be not generally applicable for oligomerization strategies in mammalian host cells.
In order to further test the promising tetramerization domain, it was inserted into another fusion protein that closely resembles the M79scFv-IL-2-construct. This polypeptide consists of the DC8scFv-fragment (Schakel, Eur. J. Immunol. 28 (1998), 4084-4093) at the N-terminus and the extracellular domain of human erbB2 at the C-terminus (note that the extracellular domain of human erbB2 is as well secreted by mammalian cells as human IL-2). The complete polypeptide chain is shown in Fig. 49. The corresponding DNA-fragment was also cloned into the expression vector pEF-DHFR with the restriction enzymes EcoRI and Sall and transfected into DHFR-deficient CHO-cells as described (Mack, Proc. Natl. Acad. Sci. USA 92 (1995) 7021-7025). Culture supernatant of the transfected CHO-cells as well as the corresponding cell lysate prepared according to standard procedures were tested by an erbB2 specific Sandwich-ELISA based on two anti-erbB2 antibodies (chimeric 7C1 and murine Her81) that recognize different epitopes allowing simultaneous binding to the antigen. Chimeric 7C1, immobilized on an ELISA-plate was incubated with several dilutions of culture supernatant or cell lysate thus capturing tetrameric DC8scFv-erbB2_{EC}-construct, that could then be detected with murine Her81 followed by an Fc-specific peroxidase-conjugated polyclonal goat anti-mouse IgG antibody (Dianova, Hamburg; Cat.No.115-035-071) diluted 1:5000. As positive control equivalent dilutions of culture supernatant and cell lysate of the tetrameric M79scFv-IL-2-construct was analyzed in parallel with the ELISA described above; results are shown in Fig. 51A. The general ELISA procedure was performed as described in Example 4.4.
The results shown in Fig. 51B demonstrate, that in contrast to the tetrameric M79scFv-IL-2-construct, the closely related tetrameric DC8scFv-erbB2_{EC}-construct is not detectable in the supernatant and thus not produceable as secretable and fully functional protein in mammalian host cells, although it is undoubtedly present in the cell lysate. In conclusion, the tetramerization domain also failed to demonstrate general applicability for oligomerization strategies in mammalian host cells.
Although both oligomerization domains are well established for polypeptide oligomerization in E.coli and are envisaged in the literature to be suited for fusion proteins like that tested in this example (Rheinnecker (1996) J. Immunol. 157: 2989), they clearly failed to generally meet this goal in mammalian host cells. Thus, it appears as a common principle, that successful use of oligomerization domains in E.coli does not predict their general applicability in mammalian host cells.

### Example 10: Design of a heterominibody with effector domains present in all four positions

The heterominibody format principally allows the addition of four distinct effector domains to the two N- and two C-terminal positions present in the core of the disulfide-linked C_{H}1 and C kappa domains (see Figure 52). While the previous examples showed that effector domains, such as single chain Fvs (scFvs) and co-stimulatory molecules, retain their functionality at the N-termini of the heterominibody, it is not clear whether two effector domains can be functionally linked at a time to the C-terminal positions of C_{H}1 and Ck (kappa). Improper folding, steric hinderance by the N-terminal sequences or mutual hinderance may perturb the activity of effector domains linked to the C-termini of heterominibodies, or prevent the expression of such heterominibodies in mammalian cells.
In order to demonstrate that, firstly, heterominibodies can be made with all four positions being linked to effector domains and, secondly, that two C-terminally added domains retain their biological activity, the heterominibody shown in Figure 53 was designed. In this molecule, a single chain Fv ("HD70 scFv" corresponding to nucleotides 96 to 842 as depicted in either of the two nucleotide sequences shown in Figure 55a and 55b. The corresponding antibody HD70 is described in WO 98/46645) is N-terminally linked to the human C_{H}1 domain which bears at its C-terminus the human inflammatory cytokine granulocyte/macrophage colony-stimulating factor (GM-CSF), plus a hexahistidine sequence for ease of purification.
For the second polypeptide chain, the human Ck domain also had a HD70 scFv linked to its N-terminus but the human inflammatory cytokine interleukin-2 (IL-2) linked to its C-terminus. In order to provide a flexible linkage, a serine-glycine linker was put in between the C_{H}1 and Ck domains and the C-terminally linked cytokines. The HD70 scFv specifically recognizes the human epithelial cell adhesion molecule (EpCAM; also called 17-1A antigen).
Plasmid expression vectors encoding HD70--CH1--GM-CSF and HD70--Ck--IL-2 chains were constructed. The previously mentioned mammalian expression vectors were used (pEF-DHFR; pEF-ADA). The arrangement of the various domain-encoding DNA sequences in the two expression vectors is shown in Figure 54. For proper secretion in mammalian cells, a sequence encoding a peptide leader from IgG was added at the 5'-ends of the constructs. The entire nucleotide and amino acid sequences of the two coding sequences for HD70--CH1--GM-CSF and HD70--Ck--IL-2 chains is shown in Figure 55.

### Example 11: Production and characterization of an anti-EpCAM heterominibody with two distinct cytokines linked to its C-termini

In order to test whether the heterominibody shown in Figure 53 is expressed and secreted, chinese hamster ovary (CHO) cells were sequentially transfected using Superfect (Qiagen) with the vectors encoding the two chains of the heterominibody and stably expressing cells selected as described previously. Cell culture supematants from stably transfected CHO cells were analyzed by FACS for the presence of an activity binding to CHO cells stably expressing human EpCAM (see previous examples). For detection of heterominibody binding to EpCAM-positive CHO cells, a mouse-anti-human GM-CSF antibody (Biosource, Cat. No. AHC 2812) was used. The second antibody was detected by a FITC-labeled sheep anti-mouse antibody (Sigma, Cat. No. F6257). No binding was seen with the supematant from non-transfected CHO cells (Figure 56, upper panel). Increasing amounts of supernatant from stably transfected CHO cells caused an increasing shift of the mean value of fluorescence to the right indicative of an increasing amount of heterominibody binding to EpCAM-positive CHO cells (Figure 56, lower panels). This shows that at least one of the chains was expressed and secreted while maintaining a functional EpCAM-specific scFv in its N-terminus.
Next it was tested whether the EpCAM activity in the supernatant of transfected CHO cells is physically linked with immunoreactivity for the cytokines GM-CSF and IL-2. To this end, an ELISA was established which captures anti-EpCAM scFvs by their binding to the recombinant, extracellular domain of EpCAM (Micromet) that was used to coat the ELISA plates. Bound molecules were then tested by specific antibodies for the presence of immunoreactivity for human IL-2 (Pharmingen Cat. No. 18951D + FITC-labeled goat anti-rat antibody (Sigma Cat. No. F6258)) and human GM-CSF (Biosource, Cat. No. AHC 2812 + FITC-labeled sheep anti-mouse antibody (Sigma Cat. No. F6257)), respectively. As shown in Figure 57 (columns 2), the bound EpCAM indeed captured an activity which was strongly immunoreacting with antibodies to both IL-2 and GM-CSF. These reactivities were not observed in the supernatant of non-transfected CHO cells (columns 1). These data show that both heterominibody chains were expressed and secreted, and that the cytokines which are C-terminally linked to the heterominibody chains are in a confirmation that can be recognized by their respective antibodies.
An important feature of the heterominibody format is the tight physical linkage of two polypeptide chains. In order to investigate whether IL-2 and GM-CSF which are part of different polypeptide chains (see Figure 53) are indeed physically linked in a heterominibody, an ELISA was established in which the molecules contained in the CHO cell culture supematant are captured to the ELISA plate by an antibody to human GM-GSF (Biosource, see above) and, subsequently, analyzed for reactivity with an anti-IL-2 antibody (Pharmingen, see above, and alkaline phosphatase conjugated streptavidin, DAKO Cat. No. D0396). Likewise, capturing was performed with an anti-IL-2 antibody (see above) and subsequent detection using a biotinylated anti-GM-CSF antibody (Biosource Cat. No. AHC 2919) followed by alkaline phosphatase conjugated streptavidin (DAKO Cat. No. D0396). The results shown in Figure 58 (columns 2) demonstrate that, in fact, antibodies to GM-GSF or IL-2 are capable of precipitating IL-2 or GM-CSF immunoreactivities, respectively. This shows that GM-CSF and IL-2 were both tightly associated within one molecule. Supernatant form control CHO cells did not show any of the reactivities (Figure 58, columns 1).
Another important feature of the heterominibody format is the covalent linkage of the two chains by a disulfide bond. In order to demonstrate that the expression of the two chains in CHO cells resulted in a covalently joined molecule that displays reactivities for both IL-2 and GM-GSF, heterominibody was purified from stably transfected CHO cells via cation exchange and cobalt chelate affinity chromatography. As shown in Figure 59 (lane 1), this purification yielded one prominent band with an apparent molecular weight of 116 kDa upon non-reducing SDS-PAGE followed by Coomassie blue staining. The identity of this band with the properly assembled heterominibody was demonstrated by Western blotting using a biotinylated antibody specific for the human C_{κ} domain (Pierce; Cat No: 31780). The antibody was detected by a streptavidinlalkaline phosphatase conjugate (DAKO, Cat. No. D0396). The immunostaining for Cκ comigrated with the Coomassie blue-stained 116-kDa band (Figure 59, lane 2). The molecular weight of 116 kDa is consistent with the sum of calculated molecular weights of the HD70-CH1--GM-CSF (54.4 kDa) and HD70--Ck--IL-2 (55.4 kDa) chains. Carbohydrate moieties linked to the N-glycosylation sites in the two VH HD70 domains may account for the difference of 6.2 kDa. As shown by Western blotting, the heterominibody molecule of 116 kDa was also immunoreactive with antibodies specific for human IL-2 (Figure 59, lane 3) and human GM-CSF (lane 4). These data show that it is possible to produce a properly assembled heterominibody molecule in CHO cells which is immunoreactive for two distinct cytokines.
The production of pharmaceutically useful heterominibodies requires that N- and C-terminally attached effector domains retain their proper biological activities. In order to test whether the C-terminally attached cytokines GM-CSF and IL-2 were still biologically active in the heterominobody, their activity was determined in a proliferation assay. Both IL-2 and GM-CSF can initiate the proliferation of immune cells bearing their respective high-affinity receptors. Purified heterominibody was added to cell cultures of TF-1 (a human erythroleukemia line; provided by DSMZ, Braunschweid, Germany) and CL96 (a murine T cell line, described in: Marcucci (1981) Nature 291, 79-81), which proliferate in response to human GM-CSF and human IL-2, respectively. In both cases, cell proliferation as measured by the reagent WST-1 (Boehringer Mann heim, Cat.No 1644807), was induced by GM-CSF in TF-1 cells (Figure 60) and by IL-2 in CL96 cells (Figure 61) in a form where the cytokines are physically linked to the heterominibody's C-termini (Figures 53). This shows that both effector functions in the two C-terminal positions of the heterominibody can retain their full biological activity.
In summary, it was shown that the heterominibody format allows the production of multivalent, highly active molecules that can have up to four functional effector domains attached to either their N- and C-terminal positions. It is envisaged that yet other effector domains can be added to the N- and C-termini of the attached effector domains (see Figure 52).

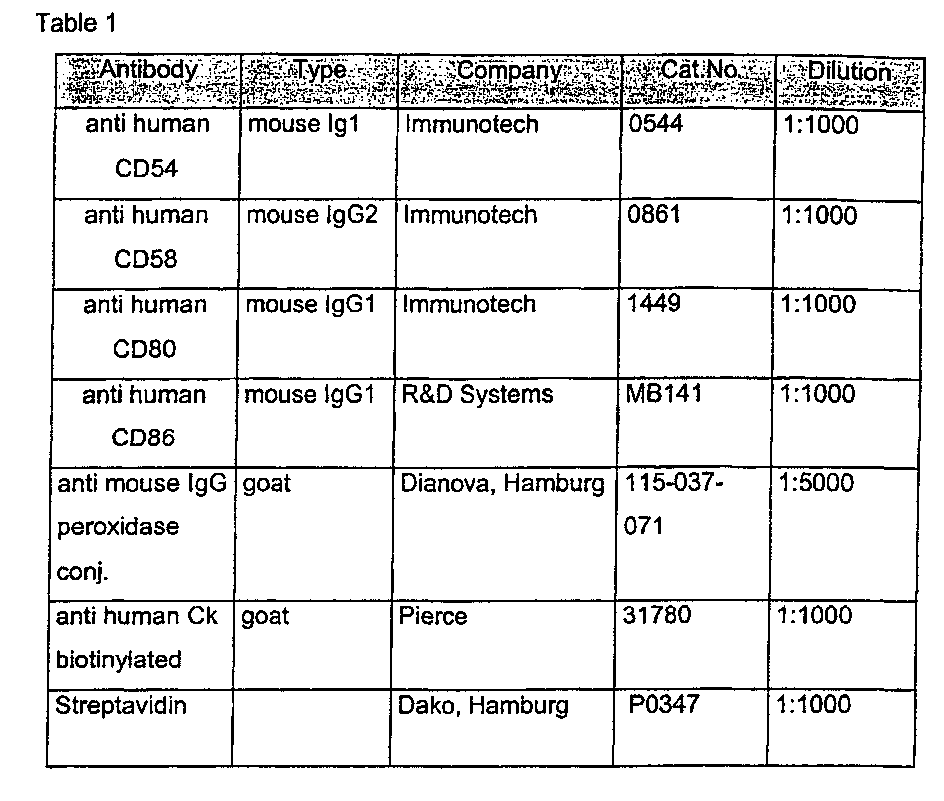

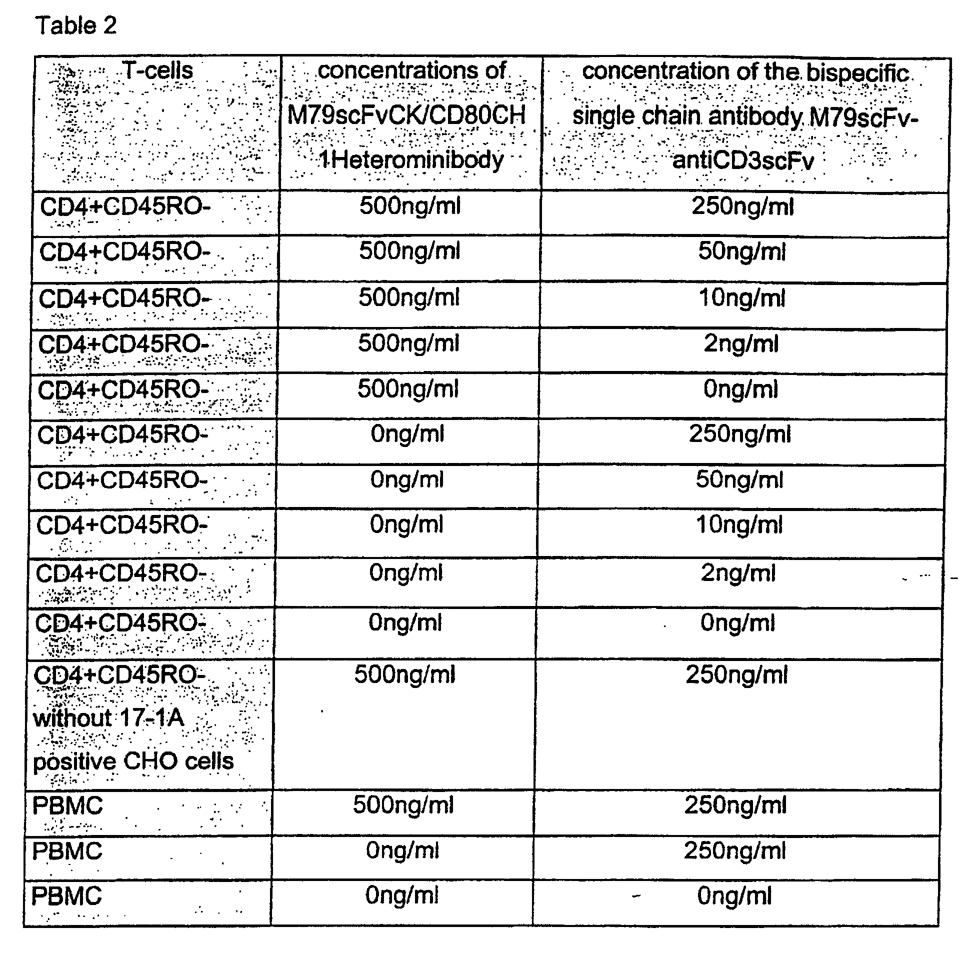

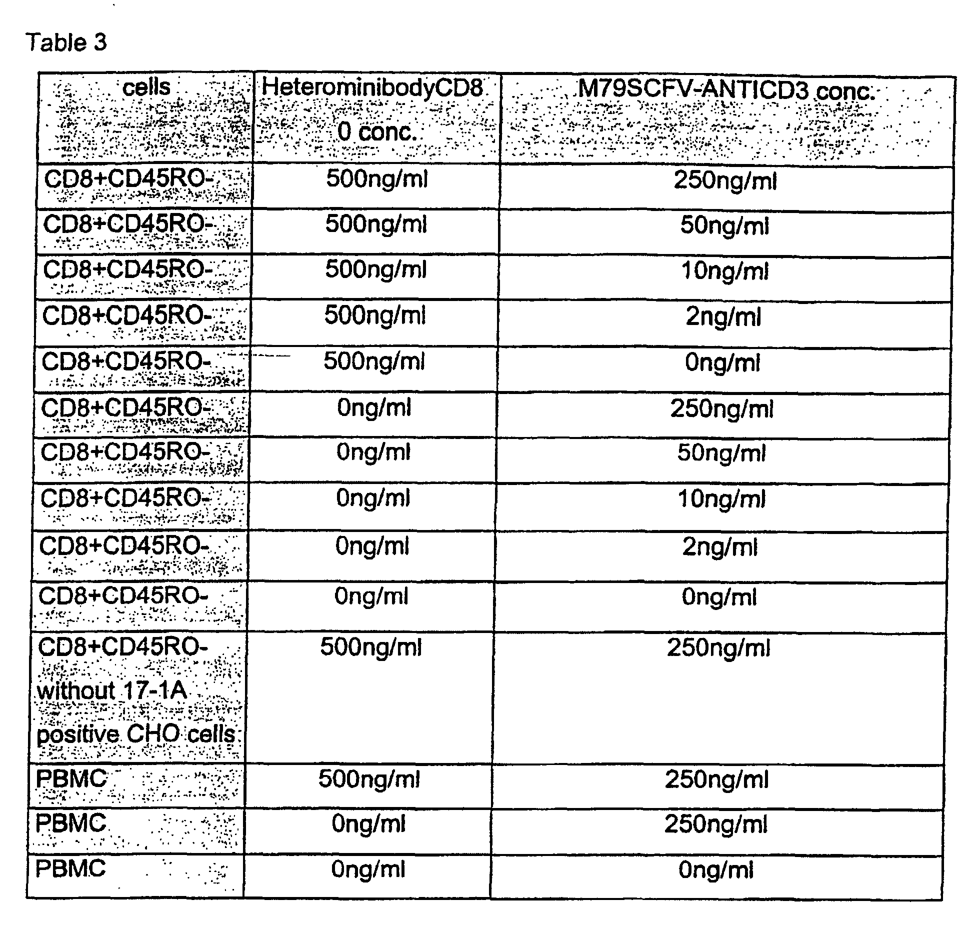

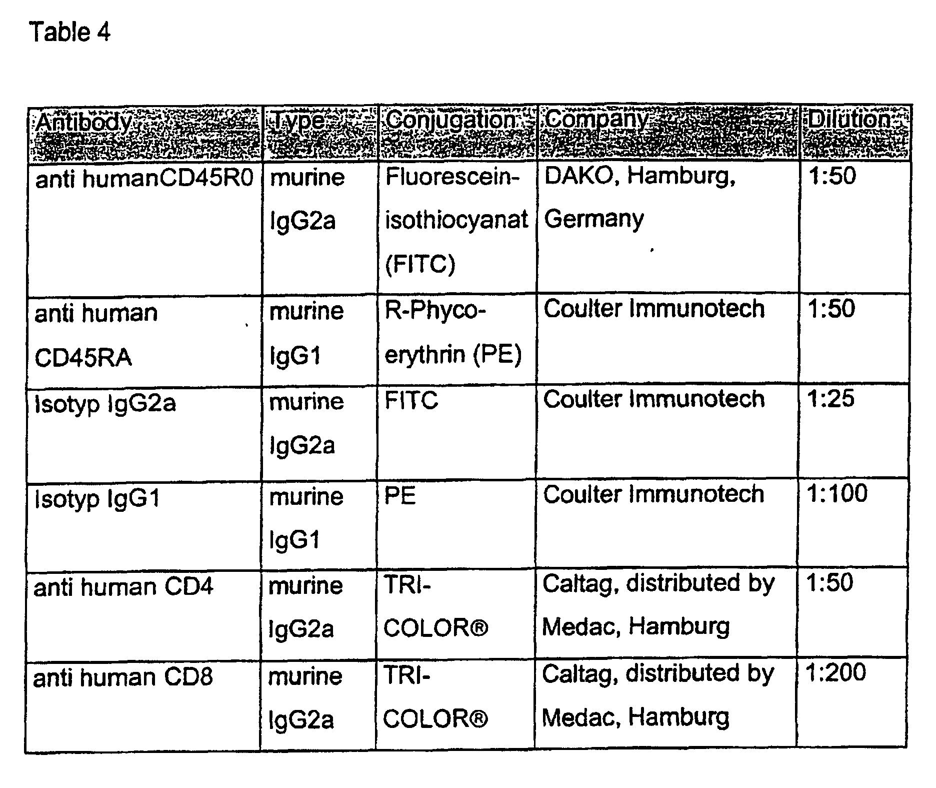

## Claims

1. A multifunctional compound, producible in a mammalian host cell as a secretable and fully functional heterodimer of two polypeptide chains, wherein one of said polypeptide chains comprises, as the only constant region domain of an immunoglobulin heavy chain the C_{H1}-domain and the other polypeptide chain comprises the constant C_{L}-domain of an immunoglobulin light chain, wherein said multifunctional compound further comprises, fused to said constant region domains at least two and up to four (poly)peptides having different receptor or ligand functions, wherein further at least two of said different (poly)peptides lack an intrinsic affinity for one another and wherein said polypeptide chains are linked via said constant domains.

2. The multifunctional compound of claim 1, wherein the functional domains, having receptor or ligand function, are C-and/or N-terminally linked to said constant immunoglobulin domains.

3. The multifunctional compound of claim 1 or 2, comprising at least three functional domains, having receptor or ligand function.

4. The multifunctional compound of anyone of claims 1 to 3, comprising four functional domains, having receptor or ligand function.

5. The multifunctional compound of anyone of claims 1 to 4, wherein at least two domains, having receptor or ligand function, are N-terminally linked to said constant immunoglobulin domains.

6. The multifunctional compound of any one of claims 1 to 5, wherein at least one of said domains, having receptor or ligand function, is in the format of an scFv-fragment or a functional part thereof.

7. The multifunctional compound of any one of claims 1 to 6, wherein at least one of said domains, having receptor or ligand function, is a T-cell co-stimulatory ligand, an antigen binding region specific for a tumor associated antigen, or a proteinaceous compound providing the primary activation signal for T-cells.

8. The multifunctional compound of any one of claims 6 or 7, wherein said scFv fragment or said functional part thereof comprise the V_{H} and the V_{L} regions of the murine anti-human 17-1A antibody M79, the V_{H} and the V_{L} regions of the anti-Lewis Y antibody, as shown in Fig. 6, the V_{H} and the V_{L} regions of the anti-CD3 antibody TR66, and/or the V_{H} and the V_{L} regions of the human anti-human EpCAM antibody as shown in Figure 55.

9. The multifunctional compound of claim 7, wherein the T-cell co-stimulatory ligand is a cell surface molecule or a fragment thereof expressed on antigen-presenting cells (APC).

10. The multifunctional compound of claim 9, wherein the antigen-presenting cell is a dendritic cell.

11. The multifunctional compound of claim 9, wherein the cell surface molecule is selected from the group consisting of B7-1, B7-2, ICAM-1, ICAM-2, ICAM-3, LFA-3 and CD137-ligand.

12. The multifunctional compound of any one of claims 1 to 5, wherein at least one of said domains, having receptor or ligand function, is an immunomodulating effector molecule or a fragment thereof.

13. The multifunctional compound of claim 12, wherein said immunomodulating effector molecule or said fragment thereof is selected from the group consisting of cytokines, chemokines, macrophage migration factor (MIF), T-cell receptors and soluble MHC molecules.

14. The multifunctional compound of claim 13, wherein said cytokine is selected from the group consisting of interleukins, interferons, GM-CSF, G-CSF, M-CSF, TNFs and VEGF.

15. The multifunctional compound of claim 13, wherein said chemokine is selected from the group consisting of IL-8, Eotaxin, GROα, GROβ, GROγ, IP10, MCP-1, MCP-2, MCP-3, MCP-4, MIG, MIP-1α, MIP-1β, NAP-2, RANTES, 1309, Lymphotactin and SDF-1.

16. The multifunctional compound of anyone of claims 1 to 5, wherein at least one of said domains, having receptor or ligand function, is FAS ligand (CD 95 L) or a fragment thereof.

17. The multifunctional compound of anyone of claims 1 to 5, wherein at least one of said domains, having receptor or ligand function, is a growth factor or a fragment thereof.

18. The multifunctional compound of anyone of claims 1 to 5, wherein at least one of said domains, having receptor or ligand function, is an angiogenesis inhibitor or a fragment thereof.

19. The multifunctional compound of any one of claims 1 to 18, wherein said constant domain of an immunoglobulin light chain is of the κ type.

20. The multifunctional compound of any one of claims 1 to 19, wherein said constant immunoglobulin domains and said functional receptor-ligand domains are connected by a polypeptide linker.

21. The multifunctional compound of claim 20, wherein said polypeptide linker comprises an Ig-hinge region or a plurality of glycine, alanine and/or serine.

22. The multifunctional compound of claim 21, wherein said Ig-hinge region is an IgG hinge region.

23. The multifunctional compound of claim 22, wherein the IgG hinge region is the upper hinge region of human IgG₃.

24. The multifunctional compound of any one of claims 1 to 23, wherein said functional domains, having receptor or ligand function, comprise GM-CSF, IL-2 and/or (an) scFv fragment(s) comprising the V_{H} and the V_{L} regions of the human anti-human EpCAM antibody, as shown in Figure 55.

25. The multifunctional compound of claim 24, wherein said GM-CSF and said IL-2 are C-terminally linked to said constant C_{H¹} or C_{L} domains and wherein said scFv fragment(s) comprising the V_{H} and the V_{L} regions of the human anti-human EpCAM antibody is (are) N-terminally linked to said constant C_{H¹} or C_{L} domains.

26. The multifunctional compound of any one of claims 1 to 25, wherein said C_{H}1 domain Is limited to a histidine tag, GST, Staphylococcus protein A, Lex A, a FLAG-tag or a MYC-tag.

27. The multifunctional compound of any one of claims 1 to 26, wherein said functional domains, having receptor or ligand function, is or is derived form a non-immunoglobulin domain.

28. A polynucleotide encoding the two polypeptide chains of the multifunctional compound as defined in any one of claims 1 to 27.

29. A vector comprising at least one polynucleotide of claim 28.

30. A mammalian host cell comprising at least one vector of claim 29.

31. The mammalian host cell of claim 30 which is a CHO cell or a myeloma cell.

32. A method of producing the multifunctional compound of any one of claims 1 to 27 comprising culturing the host cell of claim 30 or 31 under conditions that allow the synthesis and secretion of said multifunctional compound, and recovering said multifunctional compound from the culture.

33. A composition comprising the multifunctional compound of any one of claims 1 to 27, the polynucleotide of claim 28 or the vector of claim 29 and, optionally, a proteinaceous compound capable of providing the primary activation signal for T-cells.

34. The composition of claim 33 which is a pharmaceutical composition further comprising, optionally, a pharmaceutically acceptable carrier and/or the diluent and/or excipient.

35. The composition of claim 33 which is a diagnostic composition further comprising, optionally, suitable means for detection.

36. Use of the multifunctional compound of any one of claims 1 to 27, the polynucleotide of claim 28 and/or the vector of claim 29 for the preparation of a pharmaceutical composition for preventing and/or treating malignant cell growth.

37. The use of claim 36, wherein the malignant cell growth is related to malignancies of hemapoietic cells or to solid tumors.

38. The use of claim 37, wherein said malignancies of hemapoietic cells are lymphomas or leukemias.

39. The use of claim 37, wherein said solid tumors are carcinomas, melanomas or sarcomas.

40. A kit comprising the multifunctional compound of any one of claims 1 to 27 and, optionally, a proteinaceous compound capable of providing the primary activation signal for T-cells.

41. The composition of claim 33, the pharmaceutical composition of claim 34, the diagnostic composition of claim 35 or the kit of claim 40, wherein the proteinaceous compound capable of providing the primary activation signal for T-cells is a bispecific antibody interacting with the T-cell antigen CD3.

## Patentansprüche

1. Eine multifunktionale Verbindung, herstellbar in einer Säuger-Wirtszelle als ein sekretierbares und voll funktionales Heterodimer aus zwei Polypeptidketten, wobei die eine der Polypeptidketten als einzigste konstante Domänenregion einer schweren Immunglobulinkette die C_{H1}-Domäne umfasst, und die andere Polypeptidkette die konstante C_{L}-Domäne einer leichten Immunglobulinkette umfasst, wobei die multifunktionale Verbindung weiterhin, fusioniert an die konstanten Domänenregionen, mindestens zwei und bis zu vier (Poly)peptide mit unterschiedlichen Rezeptor- oder Ligandenfunktionen umfasst, wobei weiterhin mindestens zwei der unterschiedlichen (Poly)peptide keine intrinsische Affinität füreinander haben, und wobei die Polypeptidketten durch die konstanten Domänen verbunden sind.

2. Multifunktionale Verbindung nach Anspruch 1, wobei die funktionalen Domänen, mit Rezeptor- oder Ligandenfunktion, C- und/oder N-terminal an die konstante Immunglobulindomänen gekoppelt sind.

3. Multifunktionale Verbindung nach Anspruch 1 oder 2, umfassend mindestens drei funktionale Domänen mit Rezeptor- oder Ligandenfunktion.

4. Multifunktionale Verbindung nach einem der Ansprüche 1 bis 3, umfassend vier funktionale Domänen mit Rezeptor- oder Ligandenfunktion.

5. Multifunktionale Verbindung nach einem der Ansprüche 1 bis 4, wobei mindestens zwei Domänen mit Rezeptor- oder Ligandenfunktion N- terminal an die konstanten Immunoglobulindomänen gekoppelt sind.

6. Multifunktionale Verbindung nach einem der Ansprüche 1 bis 5, wobei mindestens eine der Domänen mit Rezeptor- oder Ligandenfunktion in der Form eines scFv-Fragments oder eines funktionalen Teils davon vorliegt.

7. Multifunktionale Verbindung nach einem der Ansprüche 1 bis 6, wobei mindestens eine der Domänen mit Rezeptor- oder Ligandenfunktion ein T-Zell co-stimulierender Ligand, eine Antigen-bindende Region spezifisch für ein Tumor-assoziiertes Antigen oder eine proteinartige Verbindung, welche das Primär-Aktivierungssignal für T-Zellen liefert, ist.

8. Multifunktionale Verbindung nach einem der Ansprüche 6 oder 7, wobei das scFv-Fragment oder der funktionale Teil davon die V_{H}- und die V_{L}-Regionen des murinen anti-Mensch 17-1a-Antikörpers M79, die V_{H}- und die V_{L}-Regionen des anti-Lewis-Y-Antikörpers, wie dargestellt in Figur 6, die V_{H}und die V_{L}-Regionen des anti-CD3-Antikörpers TR66, und/oder die V_{H}- und die V_{L}-Regionen des menschlichen anti-Mensch EpCAM-Antikörpers, wie in Figur 55 gezeigt, umfasst.

9. Multifunktionale Verbindung nach Anspruch 7, wobei der T-Zell-costimulierende Ligand ein Zelloberflächenmolekül oder ein Fragment davon ist, exprimiert auf antigenpräsentierenden Zellen (APC).

10. Multifunktionale Verbindung nach Anspruch 9, wobei die antigenpräsentierende Zelle eine dendritische Zelle ist.

11. Multifunktionale Verbindung nach Anspruch 9, wobei das Zelloberflächenmolekül aus der Gruppe bestehend aus B7-1, B7-2, ICAM-1, ICAM-2, ICAM-3, LFA-3 und CD137-Ligand ausgewählt ist.

12. Multifunktionale Verbindung nach einem der Ansprüche 1 bis 5, wobei mindestens eine der Domänen mit Rezeptor- oder Ligandenfunktion ein immunmodulierendes Effektormolekül oder ein Fragment davon ist.

13. Multifunktionale Verbindung nach Anspruch 12, wobei das immunmodulierende Effektormolekül oder das Fragment aus der Gruppe bestehend aus Zytokinen, Chemokinen, Macrophagenmigrationsfactor (MIF), T-Zell-Rezeptoren und löslichen MHC-Molekülen ausgewählt ist.

14. Multifunktionale Verbindung nach Anspruch 13, wobei das Zytokin aus der Gruppe bestehend aus Interleukinen, Interferonen, GM-CSF, G-CSF, M-CSF, TNFs und VEGF ausgewählt ist.

15. Multifunktionale Verbindung nach Anspruch 13, wobei das Chemokin aus der Gruppe bestehend aus IL-8, Eotaxin, GROα, GROβ, GROγ, IP10, MCP-1, MCP-2, MCP-3, MCP-4, MIG, MIP-1α. MIP-1β, NAP-2, RANTES, 1309, Lymphotactin und SDF-1 ausgewählt ist.

16. Multifunktionale Verbindung nach einem der Ansprüche 1 bis 5, wobei mindestens eine der Domänen mit Rezeptor- oder Ligandenfunktion ein FAS-Ligand (CD 95 L) oder ein Fragment davon ist.

17. Multifunktionale Verbindung nach einem der Ansprüche 1 bis 5, wobei mindestens eine der Domänen mit Rezeptor- oder Ligandenfunktion ein Wachstumsfaktor oder ein Fragment davon ist.

18. Multifunktionale Verbindung nach einem der Ansprüche 1 bis 5, wobei mindestens eine der Domänen mit Rezeptor- oder Ligandenfunktion ein Angiogenese-Inhibitor oder ein Fragment davon ist.

19. Multifunktionale Verbindung nach einem der Ansprüche 1 bis 18, wobei die konstante Domäne einer leichten Immunglobulinkette vom κ-Typ ist.

20. Multifunktionale Verbindung nach einem der Ansprüche 1 bis 19, wobei die konstanten Immunglobulindomänen und die funktionalen Rezeptor-Liganden-Domänen durch einen Polypeptidlinker verbunden sind.

21. Multifunktionale Verbindung nach Anspruch 20, wobei der Polypeptidlinker eine lg-Hinge-("Schamier"-)Region oder eine Vielzahl von Glycin, Alanin und/oder Serin umfasst.

22. Multifunktionale Verbindung nach Anspruch 21, wobei die Ig-Hinge-Region eine IgG-Hinge-Region ist.

23. Multifunktionale Verbindung nach Anspruch 22, wobei die IgG-Hinge-Region die obere Hinge-Region von menschlichem IgG₃ ist.

24. Multifunktionale Verbindung nach einem der Ansprüche 1 bis 23, wobei die funktionalen Domänen mit Rezeptor- oder Ligandenfunktion GM-CSF, IL-2 und/oder (ein) scFv-Fragment(e), umfassend die V_{H}- und die V_{L}-Region des menschlichen anti-Mensch EpCAM-Antikörpers, wie in Figur 55 gezeigt, umfassen.

25. Multifunktionale Verbindung nach Anspruch 24, wobei das GM-CSF und das IL-2 C-terminal an die konstanten C_{H1}- oder C_{L}-Domänen gekoppelt sind und wobei das/die scFv-Fragment(e), umfassend die V_{H}- und die V_{L}-Regionen des menschlichen anti-Mensch EpCAM-Antikörpers N-terminal an die konstante C_{H1}- oder C_{L}-Domänen gekoppelt ist/sind.

26. Multifunktionale Verbindung nach einem der Ansprüche 1 bis 25, wobei die C_{H1}-Domäne auf ein Hystidin-Tag, GST, Staphylococcusprotein A, Lex A, ein FLAG-tag oder ein MYC-tag begrenzt ist.

27. Multifunktionale Verbindung nach einem der Ansprüche 1 bis 26, wobei die funktionalen Domänen mit Rezeptor- oder Ligandenfunktion eine Nicht-Immunoglobulindomäne ist oder davon abgeleitet ist.

28. Polynucleotid, kodierend die zwei Polypeptidketten der multifunktionalen Verbindung wie in einem der Ansprüche 1 bis 27 definiert.

29. Vektor, umfassend mindestens ein Polynucleotid nach Anspruch 28.

30. Säugetier-Wirtszelle, umfassend mindestens einen Vektor nach Anspruch 29.

31. Säugetier-Wirtszelle nach Anspruch 30, welche eine CHO-Zelle oder eine Myelomazelle ist.

32. Verfahren zur Herstellung der multifunktionalen Verbindung nach einem der Ansprüche 1 bis 27, umfassend das Züchten der Wirtszelle nach Anspruch 30 oder 31 unter Bedingungen, die die Synthese und Sekretion der multifunktionalen Verbindung erlauben, und Gewinnen der multifunktionalen Verbindung aus der Kultur.

33. Verbindung, umfassend die multifunktionale Verbindung nach einem der Ansprüche 1 bis 27, das Polynucleotid aus Anspruch 28 oder den Vektor aus Anspruch 29 und, gegebenenfalls, eine proteinartige Verbindung, welche in der Lage ist das primäre Aktivierungssignal für T-Zellen zu liefern.

34. Verbindung nach Anspruch 33, welche ein Arnzneimittel ist, weiterhin gegebenenfalls umfassend einen pharmazeutisch verträglichen Träger und/oder das Verdünnungsmittel und/oder Exzipient.

35. Verbindung nach Anspruch 33, welche eine diagnostische Verbindung ist, weiterhin gegebenenfalls geeignete Nachweismittel umfassend.

36. Verwendung der multifunktionalen Verbindung nach einem der Ansprüche 1 bis 27, das Polynucleotid aus Anspruch 28 und/oder der Vektor aus Anspruch 29 zur Herstellung eines Arzneimittels zur Verhinderung und/oder Behandlung bösartigen Zellwachstums.

37. Verwendung nach Anspruch 36, wobei das bösartige Zellwachstum in Beziehung steht zu Bösartigkeiten von hämatopoetischen Zellen oder zu soliden Tumoren.

38. Verwendung nach Anspruch 37, wobei die Bösartigkeiten der hämatopoetischen Zellen Lymphome oder Leukämien sind.

39. Verwendung nach Anspruch 37, wobei die soliden Turmore Karzinome, Melanome oder Sarkome sind.

40. Kit, umfassend die multifunktionale Verbindung nach einem der Ansprüche 1 bis 27 und, gegebenenfalls, eine proteinartige Verbindung, welche in der Lage ist, das primäre Aktivierungssignal für T-Zellen zu liefern.

41. Verbindung nach Anspruch 33, Arzneimittel nach Anspruch 34, diagnostische Verbindung nach Anspruch 35 oder Kit nach Anspruch 40, wobei die proteinartige Verbindung, welche in der Lage ist, das primäre Aktivierungssignal für T-Zellen zu liefern, ein bi-spezifischer Antikörper ist, welcher mit dem T-Zell-Antigen CD3 in Wechselwirkung steht.

## Revendications

1. Composé multifonctionnel, apte à être produit dans une cellule hôte de mammifère sous forme d'un hélérodimère polypeptidique pouvant être sécrété et totalement fonctionnel, formé de deux chaînes polypeptidiques, dans lequel une desdites chaînes polypeptidiques comprend en tant que seul domaine de la région constante d'une chaîne lourde d'une immunoglobuline le domaine C_{H1} et l'autre chaîne polypeptidique comprend le domaine C_{L} constant de la chaîne légère d'une immunoglobuline, ledit composé multifonctionnel comprenant en outre au moins deux et jusqu'à quatre (poly)peptides ayant des fonctions de récepteur ou de ligand différentes, fusionnés avec lesdits domaines de région constante, dans lequel, en outre, au moins deux desdits (poly)peptides différents sont dépourvus d'une affinité intrinsèque l'un pour l'autre et dans lequel lesdites chaînes polypeptidiques sont liées par l'intermédiaire desdits domaines constants.

2. Composé multifonctionnel selon la revendication 1, dans lequel les domaines fonctionnels ayant une fonction de récepteur ou de ligand , sont liés par leur extrémité C-ou N-terminale auxdits domaines constants d'immunoglobuline.

3. Composé multifonctionnel selon la revendication 1 ou 2, comprenant au moins trois domaines fonctionnels ayant des fonctions de récepteur ou de ligand.

4. Composé multifonctionnel selon l'une quelconque des revendications 1 à 3, comprenant quatre domaines fonctionnels ayant des fonctions de récepteur ou de ligand.

5. Composé multifonctionnel selon l'une quelconque des revendications 1 à 4, dans lequel au moins deux domaines ayant des fonctions de récepteur ou de ligand sont liés par leur extrémité N-terminale auxdits domaines constants d'immunoglobuline.

6. Composé multifonctionnel selon l'une quelconque des revendications 1 à 5, dans lequel au moins un desdits domaines ayant des fonctions de récepteur ou de ligand est sous la forme d'un fragment scFv ou d'une de ses parties fonctionnelles.

7. Composé multifonctionnel selon l'une quelconque des revendications 1 à 6, dans lequel au moins un desdits domaines ayant des fonctions de récepteur ou de ligand est un ligand co-stimulateur d'une cellule T, une région de liaison à un antigène spécifique d'un antigène associé à une tumeur ou un composé protéinique fournissant le signal d'activation primaire pour des cellules T.

8. Composé multifonctionnel selon l'une quelconque des revendications 6 ou 7, dans lequel ledit fragment scFv ou sa partie fonctionnelle comprend les régions V_{H} et V_{L} de l'anticorps murin anti-17-1A humaine, M79, les régions V_{H} et V_{L} de l'anticorps anti-Lewis Y, tel que représenté à la Fig.6, les régions V_{H} et V_{L} de l'anticorps anti-CD3 TR66, et/ou les régions V_{H} et V_{L} de l'anticorps humain anti-EpCAM humaine tel que représenté à la Fig.55.

9. Composé multifonctionnel selon la revendication 7, dans lequel le ligand co-stimulateur des cellules T est une molécule de surface cellulaire ou un de ses fragments exprimée sur des cellules présentatrices d'antigène (APC).

10. Composé multifonctionnel selon la revendication 9, dans lequel la cellule présentatrice d'antigène est une cellule dendritique.

11. Composé multifonctionnel selon la revendication 9, dans lequel la molécule de surface cellulaire est choisie dans le groupe consistant en B7-1, B7-2, ICAM-1, ICAM-2, ICAM-3, LFA-3 et le ligand de CD-137.

12. Composé multifonctionnel selon l'une quelconque des revendications 1 à 5, dans lequel au moins un desdits domaines, ayant une fonction de récepteur ou de ligand est une molécule effcetrice immuno-modulatrice ou un de ses fragments.

13. Composé multifonctionnel selon la revendication 12, dans lequel ladite molécule effectrice immuno-modulatrice ou son fragment est choisie dans le groupe consistant en cytokines, chemokines, facteur de migration des macrophages (MIF), récepteurs de cellules T et molécules solubles du CMH.

14. Composé multifonctionnel selon la revendication 13, dans lequel ladite cytokine est choisie dans le groupe consistant en interleukines, interférons, GM-CSF, G-CSF, M-CSF, TNFs et VEGF.

15. Composé multifonctionnel selon la revendication 13, dans lequel ladite chemokine est choisie dans le groupe consistant en IL-8, eotaxine, GROα, GROβ, GROγ, IP10, MCP-1, MCP-2, MCP-3, MCP-4, MIG, MIP-1α, MIP-1β, NAP-2, RANTES, 1309, lymphotactine et SDF-1.

16. Composé multifonctionnel selon l'une quelconque des revendications 1 à 5, dans lequel au moins un desdits domaines ayant une fonction de récepteur ou de ligand est le ligand FAS (CD95L) ou un de ses fragments.

17. Composé multifonctionnel selon l'une quelconque des revendications 1 à 5, dans lequel au moins un desdits domaines ayant une fonction de récepteur ou de ligand est un facteur de croissance ou un de ses fragments.

18. Composé multifonctionnel selon l'une quelconque des revendications 1 à 5, dans lequel au moins un desdits domaines ayant une fonction de récepteur ou de ligand est un inhibiteur de l'angiogénèse ou un de ses fragments.

19. Composé multifonctionnel selon l'une quelconque des revendications 1 à 18, dans lequel ledit domaine constant d'une chaîne légère d'immunoglobuline est de type κ.

20. Composé multifonctionnel selon l'une quelconque des revendications 1 à 19, dans lequel lesdits domaines constants d'immunoglobuline et lesdits domaines fonctionnels récepteur-ligand sont reliés au moyen d'un fragment, de liaison polypeptidique.

21. Composé multifonctionnel selon la revendication 20, dans lequel ledit fragment de liaison polypeptidique comprend une région charniére d'Ig ou plusieurs glycines, alanines, et/ou sérines.

22. Composé multifonctionnel selon la revendication 21, dans lequel ladite région charnière d'Ig est une région charnière d'IgG.

23. Composé multifonctionnel selon la revendication 22, dans lequel ladite région charnière d'IgG est la région charnière supérieure d'IgG₃ humaine.

24. Composé multifonctionnel selon l'une quelconque des revendications 1 à 23, dans lequel lesdits domaines fonctionnels ayant une fonction de récepteur ou de ligand comprennent GM-CSF, IL-2 et/ou un(des) fragment(s) scFv comprenant les régions V_{H} et V_{L} de l'anticorps humain anti-EpCAM humaine, tel que représenté à la Fig.55.

25. Composé multifonctionnel selon la revendication 24, dans lequel lesdits GM-CSF et IL-2 sont liés par l'intermédiaire de leur extrémité C-terminale auxdits domaines constants C_{H1} ou C_{L} et dans lequel le(s) dit(s) fragment(s) scFv comprenant les régions V_{H} et V_{L} de l'anticorps humain anti-EpCAM humaine est(sont) lié(s) par l'intermédiaire de leur extrémité N-terminale auxdits domaines constants C_{H1} ou C_{L}.

26. Composé multifonctionnel selon l'une quelconque des revendications 1 à 25, dans lequel ledit domaine C_{H1} est limité à une étiquette histidine, GST, la protéine A de Staphylococcus, Lex A, une étiquette FLAG ou une étiquette MYC.

27. Composé multifonctionnel selon l'une quelconque des revendications 1 à 26, dans lequel lesdits domaines fonctionnels, ayant une fonction de récepteur ou de ligand est ou sont dérivés d'un domaine non-immunoglobuline.

28. Polynucléotide codant pour les deux chaînes polypeptidiques du composé multifonctionnel tel que défini dans l'une quelconque des revendications 1 à 27.

29. Vecteur comprenant au moins un polynucléotide selon la revendication 28.

30. Cellule hôte de mammifère comprenant au moins un vecteur selon la revendication 29.

31. Cellule hôte de mammifère selon la revendication 30 qui est une cellule CHO ou une cellule de myélome.

32. Méthode de production d' un composé multifonctionnel selon l'une quelconque des revendications 1 à 27, comprenant la culture de la cellule hôte selon la revendication 30 ou 31 dans des conditions qui permettent la synthèse et la sécrétion dudit composé multifonctionnel, et la récupération dudit composé multifonctionnel à partir de la culture.

33. Composition comprenant le composé multifonctionnel selon l'une quelconque des revendications 1 à 27, le polynucléotide selon la revendication 28 ou le vecteur selon la revendication 29, et, éventuellement, un composé protéinique capable de fournir le signal d'activation primaire pour des cellules T.

34. Composition selon la revendication 33, qui est une composition pharmaceutique comprenant en outre, éventuellement, un support pharmaceutiquement acceptable et/ou un diluant et/ou excipient.

35. Composition selon la revendication 33, qui est une composition de diagnostic comprenant en outre, éventuellement, des moyens appropriés à la détection.

36. Utilisation d'un composé multifonctionnel selon l'une quelconque des revendications 1 à 27, du polynucléotide selon la revendication 28 et/ou du vecteur selon la revendication 29, pour la préparation d'une composition pharmaceutique pour la prévention et/ou le traitement de la croissance de cellules malignes.

37. Utilisation selon la revendication 36, dans laquelle la croissance de cellules malignes est lice à des états malins des cellules hématopoïétiques ou à des tumeurs solides.

38. Utilisation selon la revendication 37, dans laquelle lesdits états malins de cellules hématopoïétiques sont des lymphomes ou des leucémies.

39. Utilisation selon la revendication 37, dans laquelle lesdites tumeurs solides sont des carcinomes, des mélanomes ou des sarcomes.

40. Kit comprenant le composé multifonctionnel selon l'une quelconque des revendications 1 à 27, et, éventuellement, un composé protéinique capable de fournir le signal d'activation primaire pour des cellules T.

41. Composition selon la revendication 33, composition pharmaceutique selon la revendication 34, composition de diagnostic selon la revendication 35 ou kit selon la revendication 40, dans lesquels le composé protéinique capable de fournir le signal d'activation primaire pour des cellules T est un anticorps bispécifique interagissant avec l'antigène CD3 des cellules T.
